(19) Europäisches Patentamt
European Patent Office
Office européen des brevets

(11) EP 2 740 356 A1

(12) EUROPÄISCHE PATENTANMELDUNG

(43) Veröffentlichungstag:
**11.06.2014 Patentblatt 2014/24**

(51) Int Cl.:
***A01N 37/36*** *(2006.01)* ***A01P 21/00*** *(2006.01)*

(21) Anmeldenummer: **12195685.8**

(22) Anmeldetag: **05.12.2012**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(71) Anmelder: **Bayer CropScience AG**
**40789 Monheim (DE)**

(72) Erfinder:
- **Müller, Thomas, Dr.**
  **60323 Frankfurt/Main (DE)**
- **Frackenpohl, Jens, Dr.**
  **60322 Frankfurt/Main (DE)**
- **Schmutzler, Dirk**
  **65795 Hattersheim (DE)**
- **Dittgen, Jan, Dr.**
  **60316 Frankfurt (DE)**
- **Heinemann, Ines, Dr.**
  **65719 Hofheim (DE)**
- **Ruiz-Santaella Moreno, Juan Pedro, Dr.**
  **40789 Monheim (DE)**
- **Hills, Martin Jeffrey**
  **65510 Idstein (DE)**
- **von Koskull-Döring, Pascal, Dr.**
  **51373 Leverkusen (DE)**

(74) Vertreter: **Gerhards, Frank**
**Bayer Intellectual Property GmbH**
**Alfred-Nobel-Str. 10**
**40789 Monheim (DE)**

# (54) Substituierte (2Z)-5(1-Hydroxycyclohexyl)pent-2-en-4-insäure-Derivate

(57) substituierter (2Z)-5(1-Hydroxycyclohexyl) pent-2-en-4-insäure-Derivate der allgemeinen Formel (I) oder deren Salze

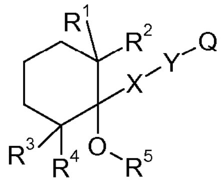

**(I)**

wobei die Reste in der allgemeinen Formel (I) den in der Beschreibung gegebenen Definitionen entsprechen, zur Steigerung der Stresstoleranz in Pflanzen gegenüber abiotischem Stress, sowie zur Stärkung des Pflanzenwachstums und/oder zur Erhöhung des Pflanzenertrags.

EP 2 740 356 A1

## Beschreibung

Die Erfindung betrifft substituierte (2Z)-5(1-Hydroxycyclohexyl)pent-2-en-4-insäure-Derivate, Verfahren zu deren Herstellung und ihre Verwendung zur Steigerung der Stresstoleranz in Pflanzen gegenüber abiotischem Stress, sowie zur Stärkung des Pflanzenwachstums und/oder zur Erhöhung des Pflanzenertrags.

Es ist bekannt, dass bestimmte 5-(1,2-Epoxy-2,6,6-trimethylcyclohexyl)-3-methylpenta-2,4-diensäuren und ihre Derivate Eigenschaften besitzen, die den Pflanzenwuchs beeinflussen (vgl. NL6811769, DE1953152). Die Verwendung von substituierten 5-Cyclohex-2-en-1-yl-penta-2,4-dienyl- und 5-Cyclohex-2-en-1-yl-pent-2-en-4-inyl-olen, -thioethern und aminen als Inhibitoren der Epoxycarotenoiddioxygenase und als Keimungsinhibitoren wird in US2010/0160166 beschrieben. Es ist ebenfalls bekannt, daß (2Z,4E)-5-[(1S,6S)-1-Hydroxy-2,2,6-trimethylcyclohexyl]penta-2,4-diensäure und (2Z,4E)-5-[(1R,6R)-1-Hydroxy-2,2,6-trimethylcyclohexyl]penta-2,4-diensäure mit Cytochrom P707A interagieren (vgl. Current Med. Chem. 2010, 17, 3230).

Es ist ebenfalls bekannt, daß substituierte 1-(Phenylethinyl)-cyclohexanole mit bestimmten Substituentengruppen in der meta-Position des Phenylrestes als Wirkstoffe in der Augenheilkunde verwendet werden können (vgl. WO2009005794, W02009058216). Weiterhin ist die Herstellung von bestimmten substituierten 1-(Phenylethinyl)-cyclohexanolen beschrieben, z. B. 1-[(4-Methoxyphenyl)ethinyl]-2,2,6-trimethylcyclohexanol und 1-[(3-Isopropyl-4-methoxyphenyl)ethinyl]-2,2,6-trimethylcyclohexanol (vgl. Bioorg. Med. Chem. 2007, 15, 2736), 2-Methyl-1-(phenylethinyl)cyclohexanol (Org. Lett. 2005, 7, 1363), 1-[(3-Methoxyphenyl)ethinyl]-2,6-dimethylcyclohexanol und 1-(Phenylethinyl)-2,6-dimethylcyclo-hexanol (Can. J. Chem. 1973, 51, 3620), Ethyl-2-hydroxy-1,3-dimethyl-2-(phenylethinyl)cyclohexancarboxylat und Ethyl-2-hydroxy-2-[(3-isopropylphenyl)ethinyl]-1,3-dimethylcyclohexancarboxylat (J. Am. Chem. Soc. 1954, 76, 5380; Synlett 2005, 2919).

Von den erfindungsgemäßen substituierten (2Z)-5(1-Hydroxycyclohexyl)pent-2-en-4-insäure-Derivaten ist dagegen die Verwendung zur Steigerung der Stresstoleranz in Pflanzen gegenüber abiotischem Stress, zur Stärkung des Pflanzenwachstums und/oder zur Erhöhung des Pflanzenertrags nicht beschrieben.

Es ist bekannt, dass Pflanzen auf natürliche Stressbedingungen, wie beispielsweise Kälte, Hitze, Trockenheit, Verwundung, Pathogenbefall (Viren, Bakterien, Pilze, Insekten) etc. aber auch auf Herbizide mit spezifischen oder unspezifischen Abwehrmechanismen reagieren [Pflanzenbiochemie, S. 393-462 , Spektrum Akademischer Verlag, Heidelberg, Berlin, Oxford, Hans W. Heldt, 1996.; Biochemistry and Molecular Biology of Plants, S. 1102-1203, American Society of Plant Physiologists, Rockville, Maryland, eds. Buchanan, Gruissem, Jones, 2000]. In Pflanzen sind zahlreiche Proteine und die sie codierenden Gene bekannt, die an Abwehrreaktionen gegen abiotischen Stress (z.B. Kälte, Hitze, Trockenheit, Salz, Überflutung) beteiligt sind. Diese gehören teilweise zu Signaltransduktionsketten (z.B. Transkriptionsfaktoren, Kinasen, Phosphatasen) oder bewirken eine physiologische Antwort der Pflanzenzelle (z.B. Ionentransport, Entgiftung reaktiver Sauerstoff-Spezies). Zu den Signalkettengenen der abiotischen Stressreaktion gehören u.a. Transkriptionsfaktoren der Klassen DREB und CBF (Jaglo-Ottosen et al., 1998, Science 280: 104-106). An der Reaktion auf Salzstress sind Phosphatasen vom Typ ATPK und MP2C beteiligt. Ferner wird bei Salzstress häufig die Biosynthese von Osmolyten wie Prolin oder Sucrose aktiviert. Beteiligt sind hier z.B. die Sucrose-Synthase und Prolin-Transporter (Hasegawa et al., 2000, Annu Rev Plant Physiol Plant Mol Biol 51: 463-499). Die Stressabwehr der Pflanzen gegen Kälte und Trockenheit benutzt z.T. die gleichen molekularen Mechanismen. Bekannt ist die Akkumulation von sogenannten Late Embryogenesis Abundant Proteins (LEA-Proteine), zu denen als wichtige Klasse die Dehydrine gehören (Ingram and Bartels, 1996, Annu Rev Plant Physiol Plant Mol Biol 47: 277-403, Close, 1997, Physiol Plant 100: 291-296). Es handelt sich dabei um Chaperone, die Vesikel, Proteine und Membranstrukturen in gestressten Pflanzen stabilisieren (Bray, 1993, Plant Physiol 103: 1035-1040). Außerdem erfolgt häufig eine Induktion von Aldehyd-Deydrogenasen, welche die bei oxidativem Stress entstehenden reaktiven Sauerstoff-Spezies (ROS) entgiften (Kirch et al., 2005, Plant Mol Biol 57: 315-332).

Heat Shock Faktoren (HSF) und Heat Shock Proteine (HSP) werden bei Hitzestress aktiviert und spielen hier als Chaperone eine ähnliche Rolle wie die Dehydrine bei Kälte- und Trockenstress (Yu et al., 2005, Mol Cells 19: 328-333).

Eine Reihe von pflanzenendogenen Signalstoffen, die in die Stresstoleranz bzw. die Pathogenabwehr involviert sind, bereits bekannt. Zu nennen sind hier beispielsweise Salicylsäure, Benzoesäure, Jasmonsäure oder Ethylen [Biochemistry and Molecular Biology of Plants, S. 850-929, American Society of Plant Physiologists, Rockville, Maryland, eds. Buchanan, Gruissem, Jones, 2000]. Einige dieser Substanzen oder deren stabile synthetische Derivate und abgeleitete Strukturen sind auch bei externer Applikation auf Pflanzen oder Saatgutbeizung wirksam und aktivieren Abwehrreaktionen, die eine erhöhte Stress- bzw. Pathogentoleranz der Pflanze zur Folge haben [Sembdner, and Parthier, 1993, Ann. Rev. Plant Physiol. Plant Mol. Biol. 44: 569-589].

Es ist weiter bekannt, dass chemische Substanzen die Toleranz von Pflanzen gegen abiotischen Stress erhöhen können. Derartige Substanzen werden dabei entweder durch Saatgut-Beizung, durch Blattspritzung oder durch Bodenbehandung appliziert. So wird eine Erhöhung der abiotischen Stresstoleranz von Kulturpflanzen durch Behandlung mit Elicitoren der Systemic Acquired Resistance (SAR) oder Abscisinsäure-Derivaten beschrieben (Schading and Wei, WO200028055; Abrams et al., US5518995; Abrams et al., W02005/108345; Abrams et al., US2010/0160166; Abrams

and Gusta, US5201931; Abrams et al, WO9723441; Kim et al., US5481034; Sasaki et al., US5254694; Bliesener et al., EP78509; Melcher et al. 2010, Nature Structural & Molecular Biology advanced online publication; Asami and Yoshida 1999, RIKEN Review 21: 17-19; Ueno et al. 2005, Bioorganic & Medicinal Chemistry 13: 3359-3370; Todoroki et al. 2010, Current Medicinal Chemistry 17: 3230-3244; Cutler et al. 2010 Annu. Rev. Plant Biol. 61:651-679; Todoroki et al. 2004, Bioorganic & Medicinal Chemistry 12: 363-370; 52: 8081-8098 Todoroki et al. 2000, Tetrahedron 56: 8095-8100; Todoroki et al. 1996, Tetrahedron 52: 8081-8098; Churchill et al., 1998, Plant Growth Regul 25: 35-45; Schubert et al. 1991, Plant Growth Regul 10: 27-32; Chen and MacTaggert 1986, Agricultural and Biological Chemistry 50: 1097-1100). Desweiteren wurden Effekte von Wachstumsregulatoren auf die Stresstoleranz von Kulturpflanzen beschrieben (Morrison and Andrews, 1992, J Plant Growth Regul 11: 113-117, RD-259027). In diesem Zusammenhang ist ebenfalls bekannt, dass ein wachstumsregulierendes Naphthylsulfonamid (4-Brom-N-(pyridin-2-ylmethyl)naphthalin-1-sulfonamid) die Keimung von Pflanzensamen in der gleichen Weise wie Abscisinsäure beeinflusst (Park et al. Science 2009, 324, 1068-1071). Außerdem ist bekannt, dass ein weiteres Naphthylsulfonamid, N-(6-aminohexyl)-5-chlornaphthalin-1-sulfonamid, den Calcium-Spiegel in Pflanzen beeinflusst, die einem Kälteschock ausgesetzt wurden (Cholewa et al. Can. J. Botany 1997, 75, 375-382).

Auch bei Anwendung von Fungiziden, insbesondere aus der Gruppe der Strobilurine oder der Succinat Dehydrogenase Inhibitoren werden ähnliche Effekte beobachtet, die häufig auch mit einer Ertragssteigerung einhergehen (Draber et al., DE-3534948, Bartlett et al., 2002, Pest Manag Sci 60: 309). Es ist ebenfalls bekannt, dass das Herbizid Glyphosat in niedriger Dosierung das Wachstum einiger Pflanzenarten stimuliert (Cedergreen, Env. Pollution 2008, 156, 1099).

Bei osmotischem Stress ist eine Schutzwirkung durch Applikation von Osmolyten wie z.B. Glycinbetain oder deren biochemischen Vorstufen, z.B. Cholin-Derivate beobachtet worden (Chen et al., 2000, Plant Cell Environ 23: 609-618, Bergmann et al., DE-4103253). Auch die Wirkung von Antioxidantien wie z.B Naphtole und Xanthine zur Erhöhung der abiotischen Stresstoleranz in Pflanzen wurde bereits beschrieben (Bergmann et al., DD-277832, Bergmann et al.,DD-277835). Die molekularen Ursachen der Anti-Stress-Wirkung dieser Substanzen sind jedoch weitgehend unbekannt.

Es ist weiter bekannt, dass die Toleranz von Pflanzen gegenüber abiotischem Stress durch eine Modifikation der Aktivität von endogenen Poly-ADP-ribose Polymerasen (PARP) oder Poly-(ADP-ribose) glycohydrolasen (PARG) erhöht werden kann (de Block et al., The Plant Journal, 2004, 41, 95; Levine et al., FEBS Lett. 1998, 440, 1; WO0004173; W004090140).

Somit ist bekannt, dass Pflanzen über mehrere endogene Reaktionsmechanismen verfügen, die eine wirksame Abwehr gegenüber verschiedensten Schadorganismen und/oder natürlichem abiotischem Stress bewirken können.

Da sich aber die ökologischen und ökonomischen Anforderungen an moderne Pflanzenbehandlungsmittel laufend erhöhen, beispielsweise was Toxizität, Selektivität, Aufwandmenge, Rückstandsbildung und günstige Herstellbarkeit angeht, und außerdem z. B. Probleme mit Resistenzen auftreten können, besteht die ständige Aufgabe neue Pflanzenbehandlungsmittel zu entwickeln, die zumindest in Teilbereichen Vorteile gegenüber den bekannten aufweisen.

Daher bestand die Aufgabe der vorliegenden Erfindung darin, weitere Verbindungen bereitzustellen, die die Toleranz gegenüber abiotischem Stress in Pflanzen erhöhen.

Gegenstand der vorliegenden Erfindung ist Verwendung substituierter (2Z)-5(1-Hydroxycyclohexyl)pent-2-en-4-insäure-Derivate der allgemeinen Formel (I) oder deren Salze

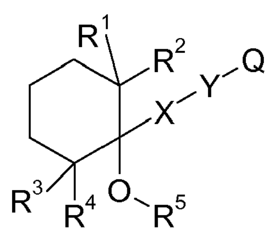

**(I)**

zur Toleranzerhöhung gegenüber abiotischem Stress in Pflanzen, wobei

[X-Y] für die Gruppierungen

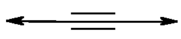 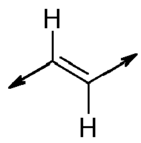 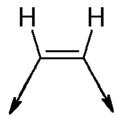 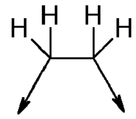

$[X\text{-}Y]^1$ , $[X\text{-}Y]^2$ , $[X\text{-}Y]^3$ und $[X\text{-}Y]^4$ ,

steht,
und Q für die Gruppierungen Q-1, Q-2, Q-3, Q-4 und Q-5 steht

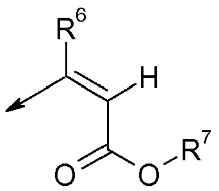
**Q-1**

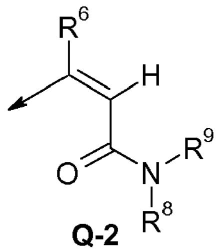
**Q-2**

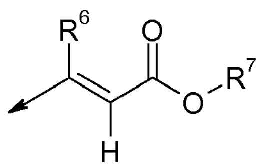
**Q-3**

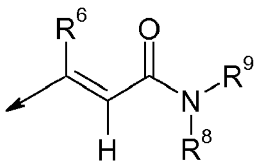
**Q-4**

und

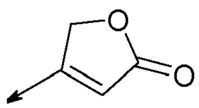

**Q-5**

und

$R^1$ $(C_1-C_{20})$-Alkyl, $(C_2-C_{20})$-Alkenyl, $(C_2-C_{20})$-Alkinyl, $(C_3-C_{20})$-Cycloalkyl, $(C_1-C_{20})$-Alkoxy, $(C_1-C_{20})$-Alkylthio, $(C_1-C_{20})$-Haloalkyl, $(C_2-C_{20})$-Haloalkenyl, $(C_2-C_{20})$-Haloalkinyl, $(C_1-C_{20})$-Haloalkoxy, $(C_1-C_{20})$-Haloalkylthio, $(C_3-C_{20})$-Cycloalkyl, $(C_3-C_{20})$-Cycloalkanyloxy, $(C_3-C_{20})$-Cycloalkanylthio, $(C_3-C_{20})$-Cycloalkenyl, $(C_3-C_{20})$-Cycloalkenyloxy, $(C_3-C_{20})$-Cycloalkenylthio, Phenyl, Heteroaryl, Heterocyclyl, wobei in den vorgenannten Gruppen das Heteroaryl ein aromatischer fünf-, sechs- oder siebengliedriger Heterocyclus und das das Heterocyclyl ein gesättigter oder teilweise ungesättigter fünf-, sechs- oder siebengliedriger Heterocyclus ist, die jeweils ein, zwei, drei oder vier Heteroatome aus der Gruppe O, N und S enthalten, oder $NR^aR^b$, wobei zwei Reste $R^1$ die an dasselbe Kohlenstoffatom gebunden sind, gemeinsam mit dem Kohlenstoffatom, an das sie gebunden sind, auch einen $(C_3-C_6)$-Cycloalkylring bilden können; wobei $NR^aR^b$ wie unten definiert sind; wobei jeder der letztgenannten drei Reste unabhängig voneinander unsubstituiert oder durch einen oder mehrere Reste aus der Gruppe Halogen, Hydroxy, $(C_1-C_4)$-Alkoxy und $(C_1-C_4)$-Alkylthio substituiert ist, bedeutet,

$R^2$ Wasserstoff, $(C_1-C_{20})$-Alkyl, $(C_2-C_{20})$-Alkenyl, $(C_2-C_{20})$-Alkinyl, $(C_3-C_{20})$-Cycloalkyl, $(C_2-C_{20})$-Alkoxy, $(C_2-C_{20})$-Alkylthio, $(C_2-C_{20})$-Haloalkyl, $(C_2-C_{20})$-Haloalkenyl, $(C_2-C_{20})$-Haloalkinyl, $(C_1-C_{20})$-Haloalkoxy, $(C_1-C_{20})$-Haloalkylthio, $(C_3-C_{20})$-Cycoalkanyl, $(C_3-C_{20})$-Cycloalkanyloxy, $(C_3-C_{20})$-Cycloalkanylthio, $(C_3-C_{20})$-Cycloalkenyl, $(C_3-C_{20})$-Cycloalkenyloxy, $(C_3-C_{20})$-Cycloalkenylthio, Phenyl, Heteroaryl, Heterocyclyl, wobei in den vorgenannten Gruppen das Heteroaryl ein aromatischer fünf-, sechs- oder siebengliedriger Heterocyclus und das das Heterocyclyl ein gesättigter oder teilweise ungesättigter fünf-, sechs- oder siebengliedriger Heterocyclus ist, die jeweils ein, zwei, drei oder vier Heteroatome aus der Gruppe O, N und S enthalten, oder $NR^aR^b$, wobei zwei Reste $R^1$, die an dasselbe Kohlenstoffatom gebunden sind, gemeinsam mit dem Kohlenstoffatom, an das sie gebun-

den sind, auch einen ($C_3$-$C_6$)-Cycloalkylring bilden können; wobei $NR^aR^b$ wie unten definiert sind; wobei jeder der letztgenannten drei Reste unabhängig voneinander unsubstituiert oder durch einen oder mehrere Reste aus der Gruppe Halogen, Hydroxy, ($C_1$-$C_4$)-Alkoxy und ($C_1$-$C_4$)-Alkylthio substituiert ist, bedeutet,

$R^3$ Wasserstoff, ($C_1$-$C_{20}$)-Alkyl, ($C_2$-$C_{20}$)-Alkenyl, ($C_2$-$C_{20}$)-Alkinyl, ($C_3$-$C_{20}$)-Cycloalkyl, ($C_1$-$C_{20}$)-Alkoxy, ($C_1$-$C_{20}$)-Alkylthio, ($C_1$-$C_{20}$)-Haloalkyl, ($C_2$-$C_{20}$)-Haloalkenyl, ($C_2$-$C_{20}$)-Haloalkinyl, ($C_1$-$C_{20}$)-Haloalkoxy, ($C_1$-$C_{20}$)-Haloalkylthio, ($C_3$-$C_{20}$)-Cycloalkanyl, ($C_3$-$C_{20}$)-Cycloalkanyloxy, ($C_3$-$C_{20}$)-Cycloalkanylthio, ($C_3$-$C_{20}$)-Cycloalkenyl, ($C_3$-$C_{20}$)-Cycloalkenyloxy, ($C_3$-$C_{20}$)-Cycloalkenylthio, Phenyl, Heteroaryl, Heterocyclyl, wobei in den vorgenannten Gruppen das Heteroaryl ein aromatischer fünf-, sechs- oder siebengliedriger Heterocyclus und das das Heterocyclyl ein gesättigter oder teilweise ungesättigter fünf-, sechs- oder siebengliedriger Heterocyclus ist, die jeweils ein, zwei, drei oder vier Heteroatome aus der Gruppe O, N und S enthalten, oder $NR^aR^b$, wobei zwei Reste $R^1$, die an dasselbe Kohlenstoffatom gebunden sind, gemeinsam mit dem Kohlenstoffatom, an das sie gebunden sind, auch einen ($C_3$-$C_6$)-Cycloalkylring bilden können; wobei $NR^aR^b$ wie unten definiert sind; wobei jeder der letztgenannten drei Reste unabhängig voneinander unsubstituiert oder durch einen oder mehrere Reste aus der Gruppe Halogen, Hydroxy, ($C_1$-$C_4$)-Alkoxy und ($C_1$-$C_4$)-Alkylthio substituiert ist, bedeutet,

$R^4$ Wasserstoff, ($C_1$-$C_{20}$)-Alkyl, ($C_2$-$C_{20}$)-Alkenyl, ($C_2$-$C_{20}$)-Alkinyl, ($C_3$-$C_{20}$)-Cycloalkyl, ($C_1$-$C_{20}$)-Alkoxy, ($C_1$-$C_{20}$)-Alkylthio, ($C_1$-$C_{20}$)-Haloalkyl, ($C_1$-$C_{20}$)-Haloalkenyl, ($C_1$-$C_{20}$)-Haloalkinyl, ($C_1$-$C_{20}$)-Haloalkoxy, ($C_1$-$C_{20}$)-Haloalkylthio, ($C_3$-$C_{20}$)-Cycloalkanyl, ($C_3$-$C_{20}$)-Cycloalkanyloxy, ($C_3$-$C_{20}$)-Cycloalkanylthio, ($C_3$-$C_{20}$)-Cycloalkenyl, ($C_3$-$C_{20}$)-Cycloalkenyloxy, ($C_3$-$C_{20}$)-Cycloalkenylthio, Phenyl, Heteroaryl, Heterocyclyl, wobei in den vorgenannten Gruppen das Heteroaryl ein aromatischer fünf-, sechs- oder siebengliedriger Heterocyclus und das das Heterocyclyl ein gesättigter oder teilweise ungesättigter fünf-, sechs- oder siebengliedriger Heterocyclus ist, die jeweils ein, zwei, drei oder vier Heteroatome aus der Gruppe O, N und S enthalten, oder $NR^aR^b$, wobei zwei Reste $R^1$, die an dasselbe Kohlenstoffatom gebunden sind, gemeinsam mit dem Kohlenstoffatom, an das sie gebunden sind, auch einen ($C_3$-$C_6$)-Cycloalkylring bilden können; wobei jeder der Reste $R^a$, $R^b$, $R^c$, $R^d$, $R^e$, $R^f$ und $R^e$ inklusive Substituenten 1 bis 30 C-Atome, vorzugsweise 1 bis 24 C-Atome, insbesondere 1 bis 20 C-Atome aufweist, bedeutet, jeder der letztgenannten drei Reste unabhängig voneinander unsubstituiert oder durch einen oder mehrere Reste aus der Gruppe Halogen, Hydroxy, ($C_1$-$C_4$)-Alkoxy und ($C_1$-$C_4$)-Alkylthio substituiert ist, bedeutet,

$R^5$ Wasserstoff, ($C_1$-$C_{20}$)-Alkyl, ($C_2$-$C_{20}$)-Alkenyl, ($C_2$-$C_{20}$)-Alkinyl, ($C_3$-$C_{20}$)-Cycloalkyl, ($C_1$-$C_{20}$)-Haloalkyl, ($C_2$-$C_{20}$)-Haloalkenyl, ($C_2$-$C_{20}$)-Haloalkinyl, ($C_3$-$C_{20}$)-Cycloalkanyl, ($C_3$-$C_{20}$)-Cycloalkenyl, wobei jeder der letztgenannten drei Reste unabhängig voneinander unsubstituiert oder durch einen oder mehrere Reste aus der Gruppe Halogen, CN, Hydroxy, ($C_1$-$C_4$)-Alkoxy und ($C_1$-$C_4$)-Alkylthio substituiert ist, bedeutet, oder einen Rest der Formel $SiR^aR^bR^c$, wobei in der letztgenannten Formel jeder der Reste $R^a$, $R^b$ und $R^c$ unabhängig voneinander Wasserstoff oder einen gegebenenfalls substituierten Kohlenwasserstoffrest bedeutet, und inklusive Substituenten 1 bis 30 C-Atome, vorzugsweise 1 bis 24 C-Atome, insbesondere 1 bis 20 C-Atome aufweist, bedeutet,

$R^6$ Wasserstoff, ($C_2$-$C_{20}$)-Alkyl, ($C_2$-$C_{20}$)-Alkenyl, ($C_2$-$C_{20}$)-Alkinyl, ($C_3$-$C_{20}$)-Cycloalkyl, ($C_1$-$C_{20}$)-Haloalkyl, ($C_2$-$C_{20}$)-Haloalkenyl, ($C_2$-$C_{20}$)-Haloalkinyl, ($C_3$-$C_{20}$)-Cycloalkanyl, ($C_3$-$C_{20}$)-Cycloalkanyloxy, ($C_3$-$C_{20}$)-Cycloalkanylthio, ($C_3$-$C_{20}$)-Cycloalkenyl, wobei jeder der letztgenannten drei Reste unabhängig voneinander unsubstituiert oder durch einen oder mehrere Reste aus der Gruppe Halogen, Hydroxy, ($C_1$-$C_4$)-Alkoxy und ($C_1$-$C_4$)-Alkylthio substituiert ist, bedeutet,

$R^7$ Wasserstoff, ($C_1$-$C_{20}$)-Alkyl, ($C_2$-$C_{20}$)-Alkenyl, ($C_2$-$C_{20}$)-Alkinyl, ($C_3$-$C_{20}$)-Cycloalkyl, ($C_1$-$C_{20}$)-Haloalkyl, ($C_2$-$C_{20}$)-Haloalkenyl, ($C_2$-$C_{20}$)-Haloalkinyl, ($C_3$-$C_{20}$)-Cycloalkanyl, ($C_3$-$C_{20}$)-Cycloalkenyl, ($C_3$-$C_{20}$)-Cycloalkenyloxy, wobei jeder der letztgenannten drei Reste unabhängig voneinander unsubstituiert oder durch einen oder mehrere Reste aus der Gruppe Halogen, Hydroxy, ($C_1$-$C_4$)-Alkoxy und ($C_1$-$C_4$)-Alkylthio substituiert ist, bedeutet, oder einen Rest der Formel $SiR^aR^bR^c$, wobei in der letztgenannten Formel jeder der Reste $R^a$, $R^b$ und $R^c$ unabhängig voneinander Wasserstoff oder einen gegebenenfalls substituierten Kohlenwasserstoffrest bedeutet, und inklusive Substituenten 1 bis 30 C-Atome, vorzugsweise 1 bis 24 C-Atome, insbesondere 1 bis 20 C-Atome aufweist, bedeutet,

$R^8$ für Wasserstoff, $(C_1-C_{20})$-Alkyl, $(C_3-C_{20})$-Cycloalkyl, Halogen, $(C_2-C_{20})$-Alkenyl, $(C_2-C_{20})$-Alkinyl, $(C_1-C_{20})$-Haloalkyl, $(C_1-C_{20})$-Cyanoalkyl, $(C_3-C_{20})$Cycloalkylalkyl, Aryl-$(C_1-C_{20})$-alkyl, Heteroaryl-$(C_1-C_{20})$-alkyl, $(C_1-C_{20})$-Alkylcarbonyl, $(C_1-C_{20})$-Alkoxycarbonyl, $(C_2-C_{20})$-Alkenyloxycarbonyl, $(C_2-C_{20})$-Alkenylalkyloxycarbonyl, Aryl-$(C_1-C_{20})$-alkyloxycarbonyl, $(C_3-C_{20})$-Cycloalkoxycarbonyl, $(C_3-C_{20})$-Cycloalkyloxycarbonyl, $(C_1-C_{20})$-Alkylsulfonyl, Arylsulfonyl, $(C_3-C_{20})$-Cycloalkylsulfonyl, $(C_1-C_{20})$-Alkylsulfinyl, Arylsulfinyl, $(C_3-C_{20})$-Cycloalkylsulfinyl, $(C_1-C_{20})$-Alkoxycarbonyl-$(C_1-C_{20})$-alkyl, Hydroxycarbonyl-$(C_1-C_{20})$-alkyl, Arylalkoxycarbonyl-$(C_1-C_{20})$-alkyl, $(C_3-C_{20})$Cycloalkylalkoxycarbonyl-$(C_1-C_{20})$-alkyl, Alkoxycarbonyl-$(C_3-C_{20})$-cycloalkyl, Hydroxycarbonyl-$(C_3-C_{20})$-Cycloalkyl, $(C_1-C_{20})$-Alkoxycarbonyl-$(C_3-C_{20})$-cycloalkenyl, Hydroxycarbonyl-$(C_3-C_{20})$-cycloalkenyl, Bis-$(C_1-C_{20})$-Alkylamino-$(C_1-C_{20})$-alkyl steht, bedeutet,

$R^9$ für Wasserstoff, $(C_1-C_{20})$-Alkyl, $(C_3-C_{20})$-Cycloalkyl, Halogen, $(C_1-C_{20})$-Halogenalkyl, $(C_2-C_{20})$-Alkinyl, $(C_2-C_{20})$-Alkenyl, $(C_1-C_{20})$-Cyanoalkyl, Aryl-$(C_1-C_{20})$-alkyl, Heteroaryl-$(C_1-C_{20})$-alkyl, $(C_1-C_{20})$-Alkylcarbonyl, $(C_1-C_{20})$-Alkoxycarbonyl, $(C_1-C_{20})$-Alkylsulfonyl, Arylsulfonyl, $(C_3-C_{20})$-Cycloalkylsulfonyl, $(C_1-C_{20})$-Alkylsulfinyl, Arylsulfinyl, $(C_3-C_{20})$Cycloalkylsulfinyl, $(C_1-C_{20})$-Alkoxycarbonyl-$(C_1-C_{20})$-alkyl steht,

oder $R^8$ und $R^9$ zusammen Bestandteil einer gegebenenfalls substituierten Sulfilimin- oder Amidingruppe sind oder ein Iminophosphoran bilden.

Die oben aufgeführten allgemeinen oder in Vorzugsbereichen aufgeführten Restedefinitionen gelten sowohl für die Endprodukte der Formel (I) als auch entsprechend für die jeweils zur Herstellung benötigten Ausgans- oder Zwischenprodukte. Diese Restedefinitionen können untereinander, also auch zwischen den angegebenen bevorzugten Bereichen beliebig kombiniert werden.

Im Hinblick auf die erfindungsgemäßen Verbindungen werden die vorstehend und weiter unten verwendeten Bezeichnungen erläutert. Diese sind dem Fachmann geläufig und haben insbesondere die im Folgenden erläuterten Bedeutungen:

"Alkoxy" bedeutet ein über ein Sauerstoffatom gebundenen Alkylrest, Alkenyloxy bedeutet ein über ein Sauerstoffatom gebundenen Alkenylrest, Alkinyloxy bedeutet ein über ein Sauerstoffatom gebundenen Alkinylrest, Cycloalkyloxy bedeutet ein über ein Sauerstoffatom gebundenen Cycloalkylrest und Cycloalkenyloxy bedeutet ein über ein Sauerstoffatom gebundenen Cycloalkenylrest.

Der Begriff "Aryl" bedeutet ein gegebenenfalls substituiertes mono-, bi- oder polycyclisches aromatisches System mit vorzugsweise 6 bis 14, insbesondere 6 bis 10 Ring-C-Atomen, beispielsweise Phenyl, Naphthyl, Anthryl, Phenanthrenyl, und ähnliches, vorzugsweise Phenyl.

Vom Begriff "gegebenenfalls substituiertes Aryl" sind auch mehrcyclische Systeme, wie Tetrahydronaphtyl, Indenyl, Indanyl, Fluorenyl, Biphenylyl, umfasst, wobei die Bindungsstelle am aromatischen System ist. Von der Systematik her ist "Aryl" in der Regel auch von dem Begriff "gegebenenfalls substituiertes Phenyl" umfasst.

Erfindungsgemäß steht der Ausdruck "Heteroaryl" für heteroaromatische Verbindungen, d. h. vollständig ungesättigte aromatische heterocyclische Verbindungen, vorzugsweise für 5- bis 7-gliedrige Ringe mit 1 bis 3, vorzugsweise 1 oder 2 gleichen oder verschiedenen Heteroatomen, vorzugsweise O, S oder N. Erfindungsgemäße Heteroaryle sind beispielsweise 1H-Pyrrol-1-yl; 1H-Pyrrol-2-yl; 1H-Pyrrol-3-yl; Furan-2-yl; Furan-3-yl; Thien-2-yl; Thien-3-yl, 1H-Imidazol-1-yl; 1H-Imidazol-2-yl; 1H-Imidazol-4-yl; 1H-Imidazol-5-yl; 1H-Pyrazol-1-yl; 1H-Pyrazol-3-yl; 1H-Pyrazol-4-yl; 1H-Pyrazol-5-yl, 1H-1,2,3-Triazol-1-yl, 1H-1,2,3-Triazol-4-yl, 1H-1,2,3-Triazol-5-yl, 2H-1,2,3-Triazol-2-yl, 2H-1,2,3-Triazol-4-yl, 1H-1,2,4-Triazol-1-yl, 1H-1,2,4-Triazol-3-yl, 4H-1,2,4-Triazol-4-yl, 1,2,4-Oxadiazol-3-yl, 1,2,4-Oxadiazol-5-yl, 1,3,4-Oxadiazol-2-yl, 1,2,3-Oxadiazol-4-yl, 1,2,3-Oxadiazol-5-yl, 1,2,5-Oxadiazol-3-yl, Azepinyl, Pyridin-2-yl, Pyridin-3-yl, Pyridin-4-yl, Pyrazin-2-yl, Pyrazin-3-yl, Pyrimidin-2-yl, Pyrimidin-4-yl, Pyrimidin-5-yl, Pyridazin-3-yl, Pyridazin-4-yl, 1,3,5-Triazin-2-yl, 1,2,4-Triazin-3-yl, 1,2,4-Triazin-5-yl, 1,2,4-Triazin-6-yl, 1,2,3-Triazin-4-yl, 1,2,3-Triazin-5-yl, 1,2,4-, 1,3,2-, 1,3,6- und 1,2,6-Oxazinyl, Isoxazol-3-yl, Isoxazol-4-yl, Isoxazol-5-yl, 1,3-Oxazol-2-yl, 1,3-Oxazol-4-yl, 1,3-Oxazol-5-yl, Isothiazol-3-yl, Isothiazol-4-yl, Isothiazol-5-yl, 1,3-Thiazol-2-yl, 1,3-Thiazol-4-yl, 1,3-Thiazol-5-yl, Oxepinyl, Thiepinyl, 1,2,4-Triazolonyl und 1,2,4-Diazepinyl. Die erfindungsgemäßen Heteroarylgruppen können ferner mit einem oder mehreren, gleichen oder verschiedenen Resten substituiert sein. Sind zwei benachbarte Kohlenstoffatome Bestandteil eines weiteren aromatischen Rings, so handelt es sich um annellierte heteroaromatische Systeme, wie benzokondensierte oder mehrfach annellierte Heteroaromaten. Bevorzugt sind beispielsweise Chinolin; Isochinolin; Chinoxalin; Chinazolin; Cinnolin; 1,5-Naphthyridin; 1,6-Naphthyridin; 1,7-Naphthyridin; 1,8-Naphthyridin; 2,6-Naphthyridin; 2,7-Naphthyridin; Phthalazin; Pyridopyrazine; Pyridopyrimidine; Pyridopyridazine; Pteridine; Pyrimidopyrimidine.

Die Bezeichnung "Halogen" bedeutet beispielsweise Fluor, Chlor, Brom oder Iod. Wird die Bezeichnung für

einen Rest verwendet, dann bedeutet "Halogen" beispielsweise ein Fluor-, Chlor-, Brom- oder Iodatom.

Erfindungsgemäß bedeutet "Alkyl" einen geradkettigen oder verzweigten offenkettigen, gesättigten Kohlenwasserstoffrest, der gegebenenfalls ein- oder mehrfach substituiert ist. Bevorzugte Substituenten sind Halogenatome, Alkoxy-, Haloalkoxy-, Cyano-, Alkylthio, Haloalkylthio-, Amino- oder Nitrogruppen, besonders bevorzugt sind Methoxy, Methyl, Fluoralkyl, Cyano, Nitro, Fluor, Chlor, Brom oder Iod.

"Haloalkyl", "-alkenyl" und "-alkinyl" bedeuten durch gleiche oder verschiedene Halogenatome, teilweise oder vollständig substituiertes Alkyl, Alkenyl bzw. Alkinyl, z.B. Monohaloalkyl (= Monohalogenalkyl) wie z. B. $CH_2CH_2Cl$, $CH_2CH_2Br$, $CHClCH_3$, $CH_2Cl$, $CH_2F$; Perhaloalkyl wie z. B. $CCl_3$, $CClF_2$, $CFCl_2$,$CF_2CClF_2$, $CF_2CClFCF_3$; Polyhaloalkyl wie z. B. $CH_2CHFCl$, $CF_2CClFH$, $CF_2CBrFH$, $CH_2CF_3$; Der Begriff Perhaloalkyl umfasst dabei auch den Begriff Perfluoralkyl.

"Fluoralkyl" bedeutet einen geradkettigen oder verzweigten offenkettigen, gesättigten und durch Fluor substituierten Kohlenwasserstoffrest, wobei sich mindestens ein Fluoratom an einer der möglichen Positionen befindet.

"Perfluoralkyl" bedeutet einen geradkettigen oder verzweigten offenkettigen, gesättigten und vollständig durch Fluor substituierten Kohlenwasserstoffrest wie z.B. $CF_3$, $CF_2CF_3$, $CF_2CF_2CF_3$

"Teilfluoriertes Alkyl" bedeutet einen geradkettigen oder verzweigten, gesättigten Kohlenwasserstoff, der einfach oder mehrfach durch Fluor substituiert ist, wobei sich die entsprechenden Fluoratome als Substituenten an einem oder mehreren verschiedenen Kohlenstoffatomen der geradkettigen oder verzweigten Kohlenwasserstoffkette befinden können, wie z. B. $CHFCH_3$, $CH_2CH_2F$, $CH_2CH_2CF_3$, $CHF_2$, $CH_2F$, $CHFCF_2CF_3$.

"Teilfluoriertes Haloalkyl" bedeutet einen geradkettigen oder verzweigten, gesättigten Kohlenwasserstoff, der durch verschiedenene Halogenatomen mit mindestens einem Fluoratom substituiert ist, wobei alle anderen gegebenenfalls vorhandenen Halogenatome ausgewählt sind aus der Gruppe Fluor, Chlor oder Brom, Iod. Die entsprechenden Halogenatome können sich dabei als Substituenten an einem oder mehreren verschiedenen Kohlenstoffatomen der geradkettigen oder verzweigten Kohlenwasserstoffkette befinden. Teilfluoriertes Haloalkyl schließt auch die vollständige Substitution der geradkettigen oder verzweigten Kette durch Halogen unter Beteiligung von mindestens einem Fluoratom ein.

Haloalkoxy ist z.B. $OCF_3$, $OCHF_2$, $OCH_2F$, $OCF_2CF_3$, $OCH_2CF_3$ und $OCH_2CH_2Cl$; Entsprechendes gilt für Haloalkenyl und andere durch Halogen substituierten Reste.

Der Ausdruck "($C_1$-$C_4$)-Alkyl" bedeutet eine Kurzschreibweise für Alkyl mit einem bis 4 Kohlenstoffatomen entsprechend der Bereichsangabe für C-Atome, d. h. umfasst die Reste Methyl, Ethyl, 1-Propyl, 2-Propyl, 1-Butyl, 2-Butyl, 2-Methylpropyl oder tert-Butyl. Allgemeine Alkylreste mit einem größeren angegebenen Bereich von C-Atomen, z. B. "($C_1$-$C_6$)-Alkyl", umfassen entsprechend auch gradkettige oder verzweigte Alkylreste mit einer größeren Zahl von C-Atomen, d. h. gemäß Beispiel auch die Alkylreste mit 5 und 6 C-Atomen.

Wenn nicht speziell angegeben, sind bei den Kohlenwasserstoffresten wie Alkyl-, Alkenyl- und Alkinylresten, auch in zusammengesetzten Resten, die niederen Kohlenstoffgerüste, z.B. mit 1 bis 6 C-Atomen bzw. bei ungesättigten Gruppen mit 2 bis 6 C-Atomen, bevorzugt. Alkylreste, auch in den zusammengesetzten Resten wie Alkoxy, Haloalkyl usw., bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl, Heptyle, wie n-Heptyl, 1-Methylhexyl und 1,4-Dimethylpentyl; Alkenyl- und Alkinylreste haben die Bedeutung der den Alkylresten entsprechenden möglichen ungesättigten Reste, wobei mindestens eine Doppelbindung bzw. Dreifachbindung enthalten ist. Bevorzugt sind Reste mit einer Doppelbindung bzw. Dreifachbindung.

Alkenyl schließt insbesondere auch geradkettige oder verzweigte offenkettige Kohlenwasserstoffreste mit mehr als einer Doppelbindung ein, wie 1,3-Butadienyl und 1,4-Pentadienyl, aber auch Allenyl- oder Kumulenyl-reste mit einer bzw. mehreren kumulierten Doppelbindungen, wie beispielsweise Allenyl (1,2-Propadienyl), 1,2-Butadienyl und 1,2,3-Pentatrienyl. Alkenyl bedeutet z.B. Vinyl, welches ggf. durch weitere Alkylreste substituiert sein kann, z.B. Prop-1-en-1-yl, But-1-en-1-yl, Allyl, 1-Methyl-prop-2-en-1-yl, 2-Methyl-prop-2-en-1-yl, But-2-en-1-yl, 1-Methyl-but-3-en-1-yl und 1-Methyl-but-2-en-1-yl, 2-Methylprop-1-en-1-yl, 1-Methylprop-1-en-1-yl, 1-Methylprop-2-en-1-yl, 2-Methyl-prop-2-en-1-yl, But-2-en-1-yl, But-3-en-1-yl, 1-Methyl-but-3-en-1-yl oder 1-Methyl-but-2-en-1-yl, Pentenyl, 2-Methylpentenyl oder Hexenyl.

Alkinyl schließt insbesondere auch geradkettige oder verzweigte offenkettige Kohlenwasserstoffreste mit mehr als einer Dreifachbindung oder auch mit einer oder mehreren Dreifachbindungen und einer oder mehreren Doppelbindungen ein, wie beispielsweise 1,3-Butatrienyl bzw. 3-Penten-1-in-1-yl. ($C_2$-$C_6$)-Alkinyl bedeutet beispielsweise Ethinyl, Propargyl, 1-Methyl-prop-2-in-1-yl, 2-Butinyl, 2-Pentinyl oder 2-Hexinyl, vorzugsweise Propargyl, But-2-in-1-yl, But-3-in-1-yl oder 1-Methyl-but-3-in-1-yl.

Der Begriff "Cycloalkyl" bedeutet ein carbocyclisches, gesättigtes Ringsystem mit vorzugsweise 3-8 Ring-C-Atomen, z.B. Cyclopropyl, Cyclobutyl, Cyclopentyl oder Cyclohexyl. Im Falle von gegebenenfalls substituiertem Cycloalkyl werden cyclische Systeme mit Substituenten umfasst, wobei auch Substituenten mit einer Doppelbindung am Cycloalkylrest, z. B. eine Alkylidengruppe wie Methyliden, umfasst sind. Im Falle von gegebenenfalls substituiertem Cycloalkyl werden auch mehrcyclische aliphatische Systeme umfaßt, wie beispielsweise Bicyclo[1.1.0]butan-1-yl, Bicyclo[1.1.0]butan-2-yl, Bicyclo[2.1.0]pentan-1-yl, Bicyclo[2.1.0]pentan-2-yl, Bicyclo[2.1.0]pentan-5-yl, Bicyclo[2.2.1]hept-2-yl (Norbornyl), Bicyclo[2.2.2]octan-2-yl, Adamantan-1-yl und Adamantan-2-yl. Der Ausdruck "($C_3$-$C_7$)-Cycloalkyl" bedeutet eine Kurzschreibweise für Cycloalkyl mit drei bis 7 Kohlenstoffatomen entsprechend der Bereichsangabe für C-Atome

Im Falle von substituiertem Cycloalkyl werden auch spirocyclische aliphatische Systeme umfaßt, wie beispielsweise Spiro[2.2]pent-1-yl, Spiro[2.3]hex-1-yl, Spiro[2.3]hex-4-yl, 3-Spiro[2.3]hex-5-yl.

"Cycloalkenyl" bedeutet ein carbocyclisches, nicht aromatisches, partiell ungesättigtes Ringsystem mit vorzugsweise 4-8 C-Atomen, z.B. 1-Cyclobutenyl, 2-Cyclobutenyl, 1-Cyclopentenyl, 2-Cyclopentenyl, 3-Cyclopentenyl, oder 1-Cyclohexenyl, 2-Cyclohexenyl, 3-Cyclohexenyl, 1,3-Cyclohexadienyl oder 1,4-Cyclohexadienyl, wobei auch Substituenten mit einer Doppelbindung am Cycloalkenylrest, z. B. eine Alkylidengruppe wie Methyliden, umfasst sind. Im Falle von gegebenenfalls substituiertem Cycloalkenyl gelten die Erläuterungen für substituiertes Cycloalkyl entsprechend.

Der Begriff "Alkyliden", z. B. auch in der Form ($C_1$-$C_{10}$)-Alkyliden, bedeutet den Rest eines geradkettigen oder verzweigten offenkettigen Kohlenwasserstoffrests, der über eine Zweifachbindung gebunden ist. Als Bindungsstelle für Alkyliden kommen naturgemäß nur Positionen am Grundkörper in Frage, an denen zwei H-Atome durch die Doppelbindung ersetzt werden können; Reste sind z. B. $=CH_2$, $=CH-CH_3$, $=C(CH_3)-CH_3$, $=C(CH_3)-C_2H_5$ oder $=C(C_2H_5)-C_2H_5$. Cycloalkyliden bedeutet ein carbocyclischer Rest, der über eine Zweifachbindung gebunden ist.

Ein heterocyclischer Rest (Heterocyclyl) enthält mindestens einen heterocyclischen Ring (=carbocyclischer Ring, in dem mindestens ein C-Atom durch ein Heteroatom ersetzt ist, vorzugsweise durch ein Heteroatom aus der Gruppe N, O, S, P, B, Si, Se) der gesättigt, ungesättigt oder heteroaromatisch ist und dabei unsubstituiert oder substituiert sein kann, wobei die Bindungsstelle an einem Ringatom lokalisiert ist. Ist der Heterocyclylrest oder der heterocyclische Ring gegebenenfalls substituiert, kann er mit anderen carbocyclischen oder heterocyclischen Ringen annelliert sein. Im Falle von gegebenenfalls substituiertem Heterocyclyl werden auch mehrcyclische Systeme umfaßt, wie beispielsweise 8-Aza-bicyclo[3.2.1]octanyl oder 1-Aza-bicyclo[2.2.1]heptyl. Im Falle von gegebenenfalls substituiertem Heterocyclyl werden auch spirocyclische Systeme umfaßt, wie beispielsweise 1-Oxa-5-aza-spiro[2.3]hexyl. Wenn nicht anders definiert, enthält der heterocyclische Ring vorzugsweise 3 bis 9 Ringatome, insbesondere 3 bis 6 Ringatome, und ein oder mehrere, vorzugsweise 1 bis 4, insbesondere 1, 2 oder 3 Heteroatome im heterocyclischen Ring, vorzugsweise aus der Gruppe N, O, und S, wobei jedoch nicht zwei Sauerstoffatome direkt benachbart sein sollen, wie beispielsweise mit einem Heteroatom aus der Gruppe N, O und S 1-oder 2- oder 3-Pyrrolidinyl, 3,4-Dihydro-2H-pyrrol-2- oder 3-yl, 2,3-Dihydro-1H-pyrrol-1-oder 2- oder 3- oder 4- oder 5-yl; 2,5-Dihydro-1H-pyrrol-1- oder 2- oder 3-yl, 1-oder 2-oder 3- oder 4-Piperidinyl; 2,3,4,5-Tetrahydropyridin-2- oder 3- oder 4- oder 5-yl oder 6-yl; 1,2,3,6-Tetrahydropyridin-1- oder 2- oder 3- oder 4- oder 5- oder 6-yl; 1,2,3,4-Tetrahydropyridin-1- oder 2- oder 3- oder 4- oder 5- oder 6-yl; 1,4-Dihydropyridin-1-oder 2- oder 3- oder 4-yl; 2,3-Dihydropyridin-2- oder 3- oder 4- oder 5- oder 6-yl; 2,5-Dihydropyridin-2- oder 3- oder 4- oder 5- oder 6-yl, 1- oder 2- oder 3- oder 4-Azepanyl; 2,3,4,5-Tetrahydro-1H-azepin-1- oder 2- oder 3- oder 4- oder 5- oder 6- oder 7-yl; 2,3,4,7-Tetrahydro-1H-azepin-1- oder 2- oder 3- oder 4- oder 5- oder 6- oder 7-yl; 2,3,6,7-Tetrahydro-1H-azepin-1- oder 2- oder 3- oder 4-yl; 3,4,5,6-Tetrahydro-2H-azepin-2- oder 3- oder 4- oder 5- oder 6- oder 7-yl; 4,5-Dihydro-1H-azepin-1- oder 2-oder 3- oder 4-yl; 2,5-Dihydro-1H-azepin-1- oder -2- oder 3- oder 4- oder 5- oder 6-oder 7-yl; 2,7-Dihydro-1H-azepin-1- oder -2- oder 3- oder 4-yl; 2,3-Dihydro-1H-azepin-1- oder -2- oder 3- oder 4- oder 5- oder 6- oder 7-yl; 3,4-Dihydro-2H-azepin-2- oder 3-oder 4- oder 5- oder 6- oder 7-yl; 3,6-Dihydro-2H-azepin-2- oder 3- oder 4- oder 5-oder 6- oder 7-yl; 5,6-Dihydro-2H-azepin-2- oder 3- oder 4- oder 5- oder 6- oder 7-yl; 4,5-Dihydro-3H-azepin-2- oder 3- oder 4- oder 5- oder 6- oder 7-yl; 1H-Azepin-1- oder -2- oder 3- oder 4- oder 5- oder 6- oder 7-yl; 2H-Azepin-2- oder 3- oder 4- oder 5- oder 6- oder 7-yl; 3H-Azepin-2- oder 3- oder 4- oder 5- oder 6- oder 7-yl; 4H-Azepin-2- oder 3- oder 4- oder 5- oder 6- oder 7-yl. Bevorzugte 3-Ring und 4-Ring-Heterocyclen sind beispielsweise 1- oder 2-Aziridinyl, Oxiranyl, Thiiranyl, 1- oder 2- oder 3-Azetidinyl, 2-oder 3-Oxetanyl, 2- oder 3-Thietanyl, 1,3-Dioxetan-2-yl.

Handelt es sich es sich um einen teilweise oder vollständig gesättigten StickstoffHeterocyclus, so kann dieser sowohl über Kohlenstoff als auch über den Stickstoff mit dem Rest des Moleküls verknüpft sein.

Erfindungsgemäß steht "Arylsulfonyl" für gegebenenfalls substituiertes Phenylsulfonyl oder gegebenenfalls substituiertes polycyclisches Arylsulfonyl, hier insbesondere gegebenenfalls substituiertes Naphthyl-sulfonyl, beispielsweise substituiert durch Halogen, Cyano, Nitro, Alkyl-, Haloalkyl-, Haloalkoxy-, Amino-, Alkylamino-, Alkylcarbonylamino-, Dialkylamino- oder Alkoxy-gruppen.

Erfindungsgemäß steht "Cycloalkylsulfonyl" - in Alleinstellung oder als Bestandteil einer chemischen Gruppe - für gegebenenfalls substituiertes Cycloalkylsulfonyl, vorzugsweise mit 3 bis 6 Kohlenstoffatomen wie beispielsweise Cyclopropylsulfonyl, Cyclobutylsulfonyl, Cyclopentylsulfonyl oder Cyclohexylsulfonyl.

Erfindungsgemäß steht "Alkylsulfonyl" - in Alleinstellung oder als Bestandteil einer chemischen Gruppe - für geradkettiges oder verzweigtes Alkylsulfonyl, vorzugsweise mit 1 bis 8, besonders bevorzugt mit 1 bis 6 Kohlenstoffatomen wie beispielsweise Methylsulfonyl, Ethylsulfonyl, n-Propylsulfonyl, Isopropylsulfonyl, n-Butylsulfonyl, Isobutylsulfonyl, sec-Butylsulfonyl und tert-Butylsulfonyl.

Erfindungsgemäß steht "Alkylthio" - in Alleinstellung oder als Bestandteil einer chemischen Gruppe - für geradkettiges oder verzweigtes S-Alkyl, vorzugsweise mit 1 bis 8, besonders bevorzugt mit 1 bis 6 Kohlenstoffatomen,

wie beispielsweise Methylthio, Ethylthio, n-Propylthio, Isopropylthio, n-Butylthio, Isobutylthio, sec-Butylthio und tert-Butylthio. Alkenylthio bedeutet ein über ein Schwefelatom gebundenen Alkenylrest, Alkinylthio bedeutet ein über ein Schwefelatom gebundenen Alkinylrest, Cycloalkylthio bedeutet ein über ein Schwefelatom gebundenen Cycloalkylrest und Cycloalkenylthio bedeutet ein über ein Schwefelatom gebundenen Cycloalkenylrest.

Die Verbindungen der allgemeinen Formel (I) können je nach Art und Verknüpfung der Substituenten als Stereoisomere vorliegen. Die durch ihre spezifische Raumform definierten möglichen Stereoisomere, wie Enantiomere, Diastereomere, Z- und E-Isomere sind alle von der Formel (I) umfasst. Sind beispielsweise eine oder mehrere Alkenylgruppen vorhanden, so können Diastereomere (Z- und E-Isomere) auftreten. Sind beispielsweise ein oder mehrere asymmetrische Kohlenstoffatome vorhanden, so können Enantiomere und Diastereomere auftreten. Stereoisomere lassen sich aus den bei der Herstellung anfallenden Gemischen nach üblichen Trennmethoden erhalten. Die chromatographische Trennung kann sowohl im analytischen Maßstab zur Feststellung des Enantiomerenüberschusses bzw. des Diastereomerenüberschusses, wie auch im präparativen Maßstab zur Herstellung von Prüfmustern für die biologische Ausprüfung erfolgen. Ebenso können Stereoisomere durch Einsatz stereoselektiver Reaktionen unter Verwendung optisch aktiver Ausgangs- und/oder Hilfsstoffe selektiv hergestellt werden. Die Erfindung betrifft somit auch alle Stereoisomeren, die von der allgemeinen Formel (I) umfasst, jedoch nicht mit ihrer spezifischen Stereoform angegeben sind, sowie deren Gemische.

Bevorzugt ist die erfindungsgemäße Verwendung von Verbindungen der Formel (I) oder deren Salze, worin

[X-Y] für die Gruppierungen

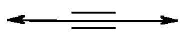

**[X-Y]$^1$** ,

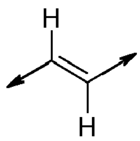

**[X-Y]$^2$** und

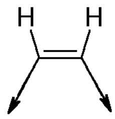

**[X-Y]$^3$**

steht,
und Q für die Gruppierungen Q-1, Q-2, Q-3 und Q-5 steht

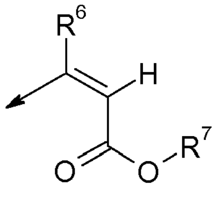

**Q-1**

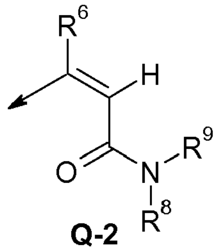

**Q-2**

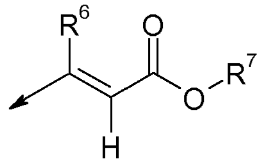

**Q-3** und

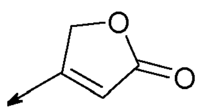

**Q-5**

und

$R^1$ ($C_1$-$C_{10}$)-Alkyl, ($C_2$-$C_{10}$)-Alkenyl, ($C_2$-$C_{10}$)-Alkinyl, ($C_3$-$C_{10}$)-Cycloalkyl, ($C_1$-$C_{10}$)-Alkoxy, ($C_1$-$C_{10}$)-Alkylthio, ($C_1$-$C_{10}$)-Haloalkyl, ($C_1$-$C_{10}$)-Haloalkenyl, ($C_2$-$C_{10}$)-Haloalkinyl, ($C_1$-$C_{10}$)-Haloalkoxy, ($C_1$-$C_{10}$)-Haloalkylthio, ($C_3$-$C_{10}$)-Cycloalkyl, ($C_3$-$C_{10}$)-Cycloalkanyloxy, ($C_3$-$C_{10}$)-Cycloalkanylthio, ($C_3$-$C_{10}$)-Cycloalkenyl, ($C_3$-$C_{10}$)-Cycloalkenyloxy, ($C_3$-$C_{10}$)-Cycloalkenylthio, Phenyl, Heteroaryl, Heterocyclyl, wobei in den vorgenannten Gruppen das Heteroaryl ein aromatischer fünf-, sechs- oder siebengliedriger Heterocyclus und das das Heterocyclyl ein gesättigter oder teilweise ungesättigter fünf-, sechs- oder siebengliedriger Heterocyclus ist, die jeweils ein, zwei, drei oder vier Heteroatome aus der Gruppe O, N und S enthalten, oder $NR^aR^b$, wobei zwei Reste $R^1$, die an dasselbe Kohlenstoffatom gebunden sind, gemeinsam mit dem Kohlenstoffatom, an das sie gebunden sind, auch einen ($C_3$-$C_6$)-Cycloalkylring bilden können; wobei $NR^aR^b$ wie unten definiert sind; wobei jeder der letztgenannten drei Reste unabhängig voneinander unsubstituiert oder durch einen oder mehrere Reste aus der Gruppe Halogen, Hydroxy, ($C_1$-$C_4$)-Alkoxy und ($C_1$-$C_4$)-Alkylthio substituiert ist, bedeutet,

$R^2$ Wasserstoff, ($C_1$-$C_{10}$)-Alkyl, ($C_2$-$C_{10}$)-Alkenyl, ($C_2$-$C_{10}$)-Alkinyl, ($C_3$-$C_{10}$)-Cycloalkyl, ($C_1$-$C_{10}$)-Alkoxy, ($C_1$-$C_{10}$)-Al-

kylthio, $(C_1-C_{10})$-Haloalkyl, $(C_1-C_{10})$-Haloalkenyl, $(C_2-C_{10})$-Haloalkinyl, $(C_1-C_{10})$-Haloalkoxy, $(C_1-C_{10})$-Haloalkylthio, $(C_3-C_{10})$-Cycloalkanyloxy, $(C_3-C_{10})$-Cycloalkanylthio, $(C_3-C_{10})$-Cycloalkenyl, $(C_3-C_{10})$-Cycloalkenyloxy, $(C_3-C_{10})$-Cycloalkenylthio, Phenyl, Heteroaryl, Heterocyclyl, wobei in den vorgenannten Gruppen das Heteroaryl ein aromatischer fünf-, sechs- oder siebengliedriger Heterocyclus und das das Heterocyclyl ein gesättigter oder teilweise ungesättigter fünf-, sechs- oder siebengliedriger Heterocyclus ist, die jeweils ein, zwei, drei oder vier Heteroatome aus der Gruppe O, N und S enthalten, oder $NR^aR^b$, wobei zwei Reste $R^1$, die an dasselbe Kohlenstoffatom gebunden sind, gemeinsam mit dem Kohlenstoffatom, an das sie gebunden sind, auch einen $(C_3-C_6)$-Cycloalkylring bilden können; wobei $NR^aR^b$ wie unten definiert sind; wobei jeder der letztgenannten drei Reste unabhängig voneinander unsubstituiert oder durch einen oder mehrere Reste aus der Gruppe Halogen, Hydroxy, $(C_1-C_4)$-Alkoxy und $(C_1-C_4)$-Alkylthio substituiert ist, bedeutet,

$R^3$ Wasserstoff, $(C_1-C_{10})$-Alkyl, $(C_2-C_{10})$-Alkenyl, $(C_2-C_{10})$-Alkinyl, $(C_3-C_{10})$-Cycloalkyl, $(C_1-C_{10})$-Alkoxy, $(C_1-C_{10})$-Alkylthio, $(C_1-C_{10})$-Haloalkyl, $(C_2-C_{10})$-Haloalkenyl, $(C_2-C_{10})$-Haloalkinyl, $(C_1-C_{10})$-Haloalkoxy, $(C_1-C_{10})$-Haloalkylthio, $(C_3-C_{10})$-Cycloalkanyloxy, $(C_3-C_{10})$-Cycloalkanylthio, $(C_3-C_{10})$-Cycloalkenyl, $(C_3-C_{10})$-Cycloalkenyloxy, $(C_3-C_{10})$-Cycloalkenylthio, Phenyl, Heteroaryl, Heterocyclyl, wobei in den vorgenannten Gruppen das Heteroaryl ein aromatischer fünf-, sechs- oder siebengliedriger Heterocyclus und das das Heterocyclyl ein gesättigter oder teilweise ungesättigter fünf-, sechs- oder siebengliedriger Heterocyclus ist, die jeweils ein, zwei, drei oder vier Heteroatome aus der Gruppe O, N und S enthalten, oder $NR^aR^b$, wobei zwei Reste $R^1$, die an dasselbe Kohlenstoffatom gebunden sind, gemeinsam mit dem Kohlenstoffatom, an das sie gebunden sind, auch einen $(C_3-C_6)$-Cycloalkylring bilden können; wobei $NR^aR^b$ wie unten definiert sind; wobei jeder der letztgenannten drei Reste unabhängig voneinander unsubstituiert oder durch einen oder mehrere Reste aus der Gruppe Halogen, Hydroxy, $(C_1-C_4)$-Alkoxy und $(C_1-C_4)$-Alkylthio substituiert ist, bedeutet,

$R^4$ Wasserstoff, $(C_1-C_{10})$-Alkyl, $(C_2-C_{10})$-Alkenyl, $(C_2-C_{10})$-Alkinyl, $(C_3-C_{10})$-Cycloalkyl, $(C_1-C_{10})$-Alkoxy, $(C_1-C_{10})$-Alkylthio, $(C_1-C_{10})$-Haloalkyl, $(C_2-C_{10})$-Haloalkenyl, $(C_2-C_{10})$-Haloalkinyl, $(C_1-C_{10})$-Haloalkoxy, $(C_1-C_{10})$-Haloalkylthio, $(C_3-C_{10})$-Cycloalkanyloxy, $(C_3-C_{10})$-Cycloalkanylthio, $(C_3-C_{10})$-Cycloalkenyl, $(C_3-C_{10})$-Cycloalkenyloxy, $(C_3-C_{10})$-Cycloalkenylthio, Phenyl, Heteroaryl, Heterocyclyl, wobei in den vorgenannten Gruppen das Heteroaryl ein aromatischer fünf-, sechs- oder siebengliedriger Heterocyclus und das das Heterocyclyl ein gesättigter oder teilweise ungesättigter fünf-, sechs- oder siebengliedriger Heterocyclus ist, die jeweils ein, zwei, drei oder vier Heteroatome aus der Gruppe O, N und S enthalten, oder $NR^aR^b$, wobei zwei Reste $R^1$, die an dasselbe Kohlenstoffatom gebunden sind, gemeinsam mit dem Kohlenstoffatom, an das sie gebunden sind, auch einen $(C_3-C_6)$-Cycloalkylring bilden können; wobei jeder der Reste $R^a$, $R^b$, $R^c$, $R^d$, $R^e$, $R^f$ und $R^e$ inklusive Substituenten 1 bis 30 C-Atome, vorzugsweise 1 bis 24 C-Atome, insbesondere 1 bis 20 C-Atome aufweist, bedeutet,
jeder der letztgenannten drei Reste unabhängig voneinander unsubstituiert oder durch einen oder mehrere Reste aus der Gruppe Halogen, Hydroxy, $(C_1-C_4)$-Alkoxy und $(C_1-C_4)$-Alkylthio substituiert ist, bedeutet,

$R^5$ Wasserstoff, $(C_1-C_{10})$-Alkyl, $(C_2-C_{10})$-Alkenyl, $(C_2-C_{10})$-Alkinyl, $(C_3-C_{10})$-Cycloalkyl, $(C_1-C_{10})$-Haloalkyl, $(C_2-C_{10})$-Haloalkenyl, $(C_2-C_{10})$-Haloalkinyl, $(C_3-C_{10})$-Cycloalkenyl, wobei jeder der letztgenannten drei Reste unabhängig voneinander unsubstituiert oder durch einen oder mehrere Reste aus der Gruppe Halogen, CN, Hydroxy, $(C_1-C_4)$-Alkoxy und $(C_1-C_4)$-Alkylthio substituiert ist, bedeutet, oder einen Rest der Formel $SiR^aR^bR^c$, wobei in der letztgenannten Formel jeder der Reste $R^a$, $R^b$ und $R^c$ unabhängig voneinander Wasserstoff oder einen gegebenenfalls substituierten Kohlenwasserstoffrest bedeutet, inklusive Substituenten 1 bis 30 C-Atome, vorzugsweise 1 bis 24 C-Atome, insbesondere 1 bis 20 C-Atome aufweist, bedeutet,

$R^6$ Wasserstoff, $(C_2-C_{10})$-Alkyl, $(C_2-C_{10})$-Alkenyl, $(C_2-C_{10})$-Alkinyl, $(C_3-C_{10})$-Cycloalkyl, $(C_1-C_{10})$-Haloalkyl, $(C_2-C_{10})$-Haloalkenyl, $(C_2-C_{10})$-Haloalkinyl, $(C_3-C_{10})$-Cycloalkanyloxy, $(C_3-C_{10})$-Cycloalkanylthio, $(C_3-C_{10})$-Cycloalkenyl, wobei jeder der letztgenannten drei Reste unabhängig voneinander unsubstituiert oder durch einen oder mehrere Reste aus der Gruppe Halogen, Hydroxy, $(C_1-C_4)$-Alkoxy und $(C_1-C_4)$-Alkylthio substituiert ist, bedeutet,

$R^7$ Wasserstoff, $(C_1-C_{10})$-Alkyl, $(C_2-C_{10})$-Alkenyl, $(C_2-C_{10})$-Alkinyl, $(C_3-C_{10})$-Cycloalkyl, $(C_1-C_{10})$-Haloalkyl, $(C_2-C_{10})$-Haloalkenyl, $(C_2-C_{10})$-Haloalkinyl, $(C_3-C_{10})$-Cycloalkenyl, $(C_3-C_{10})$-Cycloalkenyloxy, wobei jeder der letztgenannten drei Reste unabhängig voneinander unsubstituiert oder durch einen oder mehrere Reste aus der Gruppe Halogen, Hydroxy, $(C_1-C_4)$-Alkoxy und $(C_1-C_4)$-Alkylthio substituiert ist, bedeutet, oder einen Rest der Formel $SiR^aR^bR^c$, wobei in der letztgenannten Formel jeder der Reste $R^a$, $R^b$ und $R^c$ unabhängig voneinander Wasserstoff oder einen gegebenenfalls substituierten Kohlenwasserstoffrest bedeutet, inklusive Substituenten 1 bis 30 C-Atome, vorzugsweise 1 bis 24 C-Atome, insbesondere 1 bis 20 C-Atome aufweist, bedeutet,

$R^8$ für Wasserstoff, $(C_1-C_{10})$-Alkyl, $(C_1-C_{10})$-Cycloalkyl, Halogen, $(C_2-C_{10})$-Alkenyl, $(C_2-C_{10})$-Alkinyl, $(C_1-C_{10})$-Haloalkyl, $(C_1-C_{10})$-Cyanoalkyl, $(C_3-C_{10})$Cycloalkylalkyl, Aryl-$(C_1-C_{10})$-alkyl, Heteroaryl-$(C_1-C_{10})$-alkyl, $(C_1-C_{10})$-Alkylcarbonyl, $(C_1-C_{10})$-Alkoxycarbonyl, $(C_2-C_{10})$-Alkenyloxycarbonyl, $(C_2-C_{10})$-Alkenylalkyloxycarbonyl, Aryl-$(C_1-C_{10})$-alkyloxycarbonyl, $(C_3-C_{10})$-Cycloalkoxycarbonyl, $(C_3-C_{10})$-Cycloalkyloxycarbonyl, $(C_1-C_{10})$-Alkylsulfonyl, Arylsulfonyl, $(C_3-C_{10})$-Cycloalkylsulfonyl, $(C_1-C_{10})$-Alkylsulfinyl, Arylsulfinyl, $(C_3-C_{10})$-Cycloalkylsulfinyl, $(C_1-C_{10})$-Alkoxycarbonyl-$(C_1-C_{10})$-alkyl, Hydroxycarbonyl-$(C_1-C_{10})$-alkyl, Arylalkoxycarbonyl-$(C_1-C_{10})$-alkyl, $(C_3-C_{10})$Cycloalkylalkoxycarbonyl-$(C_1-C_{10})$-alkyl, Alkoxycarbonyl-$(C_3-C_{10})$-cycloalkyl, Hydroxycarbonyl-$(C_3-C_{10})$-cycloalkyl, $(C_1-C_{10})$-Alkoxycarbonyl-$(C_3-C_{10})$-cycloalkenyl, Hydroxycarbonyl-$(C_3-C_{10})$-cycloalkenyl, Bis-$(C_1-C_{10})$-Alkylamino-$(C_1-C_{10})$-alkyl steht, bedeutet,

$R^9$ für Wasserstoff, $(C_1-C_{10})$-Alkyl, $(C_3-C_{10})$-Cycloalkyl, Halogen, $(C_1-C_{10})$-Halogenalkyl, $(C_2-C_{10})$-Alkinyl, $(C_2-C_{10})$-Alkenyl, $(C_1-C_{10})$-Cyanoalkyl, Aryl-$(C_1-C_{10})$-alkyl, Heteroaryl-$(C_1-C_{10})$-alkyl, $(C_1-C_{10})$-Alkylcarbonyl, $(C_1-C_1)$-Alkoxycarbonyl, $(C_1-C_{10})$-Alkylsulfonyl, Arylsulfonyl, $(C_3-C_{10})$-Cycloalkylsulfonyl, $(C_1-C_{10})$-Alkylsulfinyl, Arylsulfinyl, $(C_3-C_{10})$Cycloalkylsulfinyl, $(C_1-C_{10})$-Alkoxycarbonyl-$(C_1-C_{10})$-alkyl steht,

oder

$R^8$ und $R^9$ zusammen Bestandteil einer gegebenenfalls substituierten Sulfilimin- oder Amidingruppe sind oder ein Iminophosphoran bilden.

Besonders bevorzugt ist die erfindungsgemäße Verwendung von Verbindungen (I) oder deren Salze, worin [X-Y] für die Gruppierung

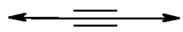

**[X-Y]¹**

steht,

und Q für die Gruppierungen Q-1 steht

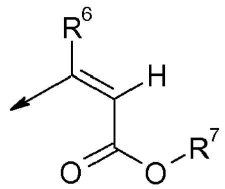

**Q-1**

und

$R^1$ $(C_1-C_6)$-Alkyl, $(C_2-C_6)$-Alkenyl, $(C_2-C_6)$-Alkinyl, $(C_3-C_6)$-Cycloalkyl, $(C_1-C_6)$-Alkoxy, $(C_1-C_6)$-Alkylthio, $(C_1-C_6)$-Haloalkyl, $(C_2-C_6)$-Haloalkenyl, $(C_2-C_6)$-Haloalkinyl, $(C_1-C_6)$-Haloalkoxy, $(C_1-C_6)$-Haloalkylthio, $(C_3-C_6)$-Cycloalkyl, $(C_3-C_6)$-Cycloalkanyloxy, $(C_3-C_6)$-Cycloalkanylthio, $(C_3-C_6)$-Cycloalkenyl, $(C_3-C_6)$-Cycloalkenyloxy, $(C_3-C_6)$-Cycloalkenylthio, Phenyl, Heteroaryl, Heterocyclyl, wobei in den vorgenannten Gruppen das Heteroaryl ein aromatischer fünf-, sechs- oder siebengliedriger Heterocyclus und das das Heterocyclyl ein gesättigter oder teilweise ungesättigter fünf-, sechs- oder siebengliedriger Heterocyclus ist, die jeweils ein, zwei, drei oder vier Heteroatome aus der Gruppe O, N und S enthalten, oder $NR^aR^b$, wobei zwei Reste $R^1$, die an dasselbe Kohlenstoffatom gebunden sind, gemeinsam mit dem Kohlenstoffatom, an das sie gebunden sind, auch einen $(C_3-C_6)$-Cycloalkylring bilden können; wobei $NR^aR^b$ wie unten definiert sind; wobei jeder der letztgenannten drei Reste unabhängig voneinander unsubstituiert oder durch einen oder mehrere Reste aus der Gruppe Halogen, Hydroxy, $(C_1-C_4)$-Alkoxy und $(C_1-C_4)$-Alkylthio substituiert ist, bedeutet,

$R^2$ Wasserstoff, ($C_1$-$C_6$)-Alkyl, ($C_2$-$C_6$)-Alkenyl, ($C_2$-$C_6$)-Alkinyl, ($C_3$-$C_6$)-Cycloalkyl, ($C_1$-$C_6$)-Alkoxy, ($C_1$-$C_6$)-Alkylthio, ($C_1$-$C_6$)-Haloalkyl, ($C_2$-$C_6$)-Haloalkenyl, ($C_2$-$C_6$)-Haloalkinyl, ($C_1$-$C_6$)-Haloalkoxy, ($C_1$-$C_6$)-Haloalkylthio, ($C_3$-$C_6$)-Cycloalkanyloxy, ($C_3$-$C_6$)-Cycloalkanylthio, ($C_3$-$C_6$)-Cycloalkenyl, ($C_3$-$C_6$)-Cycloalkenyloxy, ($C_3$-$C_6$)-Cycloalkenylthio, Phenyl, Heteroaryl, Heterocyclyl, wobei in den vorgenannten Gruppen das Heteroaryl ein aromatischer fünf-, sechs- oder siebengliedriger Heterocyclus und das das Heterocyclyl ein gesättigter oder teilweise ungesättigter fünf-, sechs- oder siebengliedriger Heterocyclus ist, die jeweils ein, zwei, drei oder vier Heteroatome aus der Gruppe O, N und S enthalten, oder $NR^aR^b$, wobei zwei Reste $R^1$, die an dasselbe Kohlenstoffatom gebunden sind, gemeinsam mit dem Kohlenstoffatom, an das sie gebunden sind, auch einen ($C_3$-$C_6$)-Cycloalkylring bilden können; wobei $NR^aR^b$ wie unten definiert sind; wobei jeder der letztgenannten drei Reste unabhängig voneinander unsubstituiert oder durch einen oder mehrere Reste aus der Gruppe Halogen, Hydroxy, ($C_1$-$C_4$)-Alkoxy und ($C_1$-$C_4$)-Alkylthio substituiert ist, bedeutet,

$R^3$ Wasserstoff, ($C_1$-$C_6$)-Alkyl, ($C_2$-$C_6$)-Alkenyl, ($C_2$-$C_6$)-Alkinyl, ($C_3$-$C_6$)-Cycloalkyl, ($C_1$-$C_6$)-Alkoxy, ($C_1$-$C_6$)-Alkylthio, ($C_1$-$C_6$)-Haloalkyl, ($C_2$-$C_6$)-Haloalkenyl, ($C_2$-$C_{10}$)-Haloalkinyl, ($C_1$-$C_6$)-Haloalkoxy, ($C_1$-$C_6$)-Haloalkylthio, ($C_3$-$C_6$)-Cycloalkanyloxy, ($C_3$-$C_6$)-Cycloalkanylthio, ($C_3$-$C_6$)-Cycloalkenyl, ($C_3$-$C_6$)-Cycloalkenyloxy, ($C_3$-$C_6$)-Cycloalkenylthio, Phenyl, Heteroaryl, Heterocyclyl, wobei in den vorgenannten Gruppen das Heteroaryl ein aromatischer fünf-, sechs- oder siebengliedriger Heterocyclus und das das Heterocyclyl ein gesättigter oder teilweise ungesättigter fünf-, sechs- oder siebengliedriger Heterocyclus ist, die jeweils ein, zwei, drei oder vier Heteroatome aus der Gruppe O, N und S enthalten, oder $NR^aR^b$, wobei zwei Reste $R^1$, die an dasselbe Kohlenstoffatom gebunden sind, gemeinsam mit dem Kohlenstoffatom, an das sie gebunden sind, auch einen ($C_3$-$C_6$)-Cycloalkylring bilden können; wobei $NR^aR^b$ wie unten definiert sind; wobei jeder der letztgenannten drei Reste unabhängig voneinander unsubstituiert oder durch einen oder mehrere Reste aus der Gruppe Halogen, Hydroxy, ($C_1$-$C_4$)-Alkoxy und ($C_1$-$C_4$)-Alkylthio substituiert ist, bedeutet,

$R^4$ Wasserstoff, ($C_1$-$C_6$)-Alkyl, ($C_2$-$C_6$)-Alkenyl, ($C_2$-$C_6$)-Alkinyl, ($C_3$-$C_6$)-Cycloalkyl, ($C_1$-$C_6$)-Alkoxy, ($C_1$-$C_6$)-Alkylthio, ($C_1$-$C_6$)-Haloalkyl, ($C_1$-$C_6$)-Haloalkenyl, ($C_1$-$C_6$)-Haloalkinyl, ($C_1$-$C_6$)-Haloalkoxy, ($C_1$-$C_6$)-Haloalkylthio, ($C_3$-$C_6$)-Cycloalkanyloxy, ($C_3$-$C_6$)-Cycloalkanylthio, ($C_3$-$C_6$)-Cycloalkenyl, ($C_3$-$C_6$)-Cycloalkenyloxy, ($C_3$-$C_6$)-Cycloalkenylthio, Phenyl, Heteroaryl, Heterocyclyl, wobei in den vorgenannten Gruppen das Heteroaryl ein aromatischer fünf-, sechs- oder siebengliedriger Heterocyclus und das das Heterocyclyl ein gesättigter oder teilweise ungesättigter fünf-, sechs- oder siebengliedriger Heterocyclus ist, die jeweils ein, zwei, drei oder vier Heteroatome aus der Gruppe O, N und S enthalten, oder $NR^aR^b$, wobei zwei Reste $R^1$, die an dasselbe Kohlenstoffatom gebunden sind, gemeinsam mit dem Kohlenstoffatom, an das sie gebunden sind, auch einen ($C_3$-$C_6$)-Cycloalkylring bilden können; wobei jeder der Reste $R^a$, $R^b$, $R^c$, $R^d$, $R^e$, $R^f$ und $R^e$ inklusive Substituenten 1 bis 30 C-Atome, vorzugsweise 1 bis 24 C-Atome, insbesondere 1 bis 20 C-Atome aufweist, bedeutet, jeder der letztgenannten drei Reste unabhängig voneinander unsubstituiert oder durch einen oder mehrere Reste aus der Gruppe Halogen, Hydroxy, ($C_1$-$C_4$)-Alkoxy und ($C_1$-$C_4$)-Alkylthio substituiert ist, bedeutet,

$R^5$ Wasserstoff, ($C_1$-$C_6$)-Alkyl, ($C_2$-$C_6$)-Alkenyl, ($C_2$-$C_6$)-Alkinyl, ($C_3$-$C_6$)-Cycloalkyl, ($C_1$-$C_6$)-Haloalkyl, ($C_2$-$C_6$)-Haloalkenyl, ($C_2$-$C_6$)-Haloalkinyl, ($C_3$-$C_6$)-Cycloalkenyl, wobei jeder der letztgenannten drei Reste unabhängig voneinander unsubstituiert oder durch einen oder mehrere Reste aus der Gruppe Halogen, CN, Hydroxy, ($C_1$-$C_4$)-Alkoxy und ($C_1$-$C_4$)-Alkylthio substituiert ist, bedeutet, oder einen Rest der Formel $SiR^aR^bR^c$, wobei in der letztgenannten Formel jeder der Reste $R^a$, $R^b$ und $R^c$ unabhängig voneinander Wasserstoff oder einen gegebenenfalls substituierten Kohlenwasserstoffrest bedeutet, inklusive Substituenten 1 bis 30 C-Atome, vorzugsweise 1 bis 24 C-Atome, insbesondere 1 bis 20 C-Atome aufweist, bedeutet,

$R^6$ Wasserstoff, ($C_2$-$C_8$)-Alkyl, ($C_2$-$C_8$)-Alkenyl, ($C_2$-$C_8$)-Alkinyl, ($C_3$-$C_8$)-Cycloalkyl, ($C_1$-$C_8$)-Haloalkyl, ($C_2$-$C_8$)-Haloalkenyl, ($C_2$-$C_8$)-Haloalkinyl, ($C_3$-$C_8$)-Cycloalkanyloxy, ($C_3$-$C_8$)-Cycloalkanylthio, ($C_3$-$C_8$)-Cycloalkenyl, wobei jeder der letztgenannten drei Reste unabhängig voneinander unsubstituiert oder durch einen oder mehrere Reste aus der Gruppe Halogen, Hydroxy, ($C_1$-$C_4$)-Alkoxy und ($C_1$-$C_4$)-Alkylthio substituiert ist, bedeutet,

$R^7$ Wasserstoff, ($C_1$-$C_7$)-Alkyl, ($C_2$-$C_7$)-Alkenyl, ($C_2$-$C_7$)-Alkinyl, ($C_3$-$C_7$)-Cycloalkyl, ($C_1$-$C_7$)-Haloalkyl, ($C_2$-$C_7$)-Haloalkenyl, ($C_2$-$C_7$)-Haloalkinyl, ($C_3$-$C_7$)-Cycloalkenyl, ($C_3$-$C_7$)-Cycloalkenyloxy, wobei jeder der letztgenannten drei Reste unabhängig voneinander unsubstituiert oder durch einen oder mehrere Reste aus der Gruppe Halogen, Hydroxy, ($C_1$-$C_4$)-Alkoxy und ($C_1$-$C_4$)-Alkylthio substituiert ist, bedeutet, oder einen Rest der Formel $SiR^aR^bR^c$, wobei in der letztgenannten Formel jeder der Reste $R^a$, $R^b$ und $R^c$ unabhängig voneinander Wasserstoff oder einen gegebenenfalls substituierten Kohlenwasserstoffrest bedeutet, inklusive Substituenten 1 bis 30 C-Atome, vorzugsweise 1 bis 24 C-Atome, insbesondere 1 bis 20 C-Atome aufweist, bedeutet, Weiterhin bevorzugt ist die erfindungsgemäße Verwendung von Verbindungen der Formeln (Ia), (Ib), (Ic), (Id) und (Ie), worin $R^6$ und $R^7$ die

für Formel (I) genannten bzw. bevorzugt genannten Bedeutungen haben.

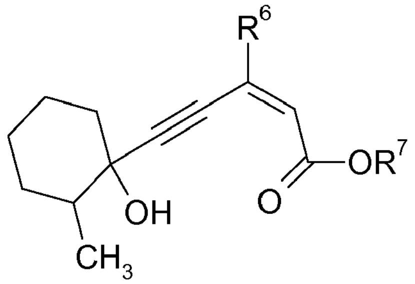

(Ia)

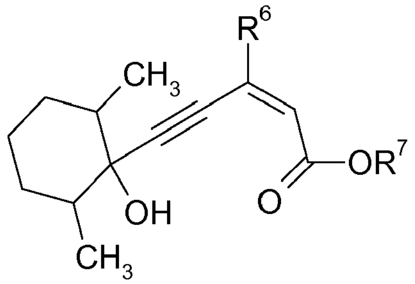

(Ib)

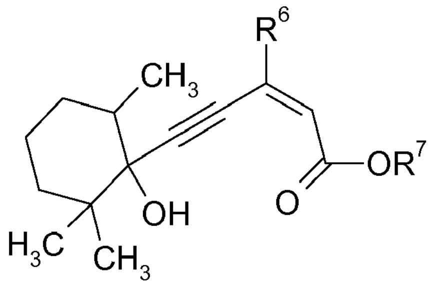

(Ic)

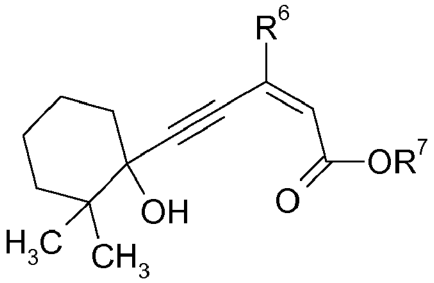

(Id)

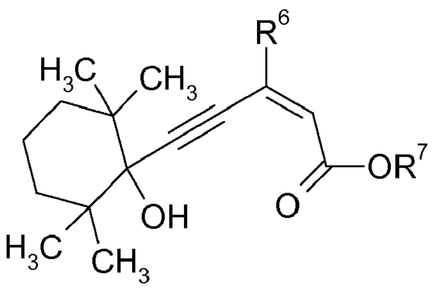

(Ie)

Dabei sind solche Verbindungen besonders bevorzugt, in welchen die Bedeutungen für [X-Y], $R^1$, $R^2$, $R^3$, $R^4$,

$R^5$, $R^6$, $R^7$, $R^8$ und $R^9$ mit denen aus den weiter unten genannten Tabellen 1 bis 5 übereinstimmen oder deren allgemeineren oben genannten Bedeutungen entsprechen.

Im Wesentlichen sind die zuvor genannten substituierte (2Z)-5(1-Hydroxycyclohexyl)pent-2-en-4-insäure-Derivate der allgemeinen Formel (I) ebenfalls noch nicht im Stand der Technik bekannt. Somit gelten als weiterer Teil der Erfindung substituierte (2Z)-5(1-Hydroxycyclohexyl)pent-2-en-4-insäure-Derivate der allgemeinen Formel (I),

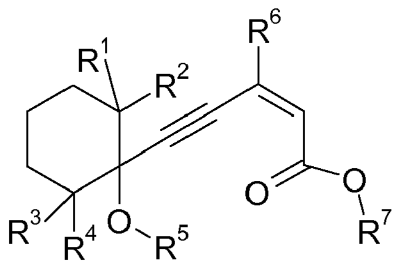

**(I)**

worin

$R^1$ $(C_1-C_6)$-Alkyl, $(C_2-C_6)$-Alkenyl, $(C_2-C_6)$-Alkinyl, $(C_3-C_6)$-Cycloalkyl, $(C_1-C_6)$-Alkoxy, $(C_1-C_6)$-Alkylthio, $(C_1-C_6)$-Haloalkyl, $(C_2-C_6)$-Haloalkenyl, $(C_1-C_6)$-Haloalkinyl, $(C_1-C_6)$-Haloalkoxy, $(C_1-C_6)$-Haloalkylthio, $(C_3-C_6)$-Cycloalkyl, $(C_3-C_6)$-Cycloalkanyloxy, $(C_3-C_6)$-Cycloalkanylthio, $(C_3-C_6)$-Cycloalkenyl, $(C_3-C_6)$-Cycloalkenyloxy, $(C_3-C_6)$-Cycloalkenylthio, Phenyl, Heteroaryl, Heterocyclyl, wobei in den vorgenannten Gruppen das Heteroaryl ein aromatischer fünf-, sechs- oder siebengliedriger Heterocyclus und das das Heterocyclyl ein gesättigter oder teilweise ungesättigter fünf-, sechs- oder siebengliedriger Heterocyclus ist, die jeweils ein, zwei, drei oder vier Heteroatome aus der Gruppe O, N und S enthalten, oder $NR^aR^b$, wobei zwei Reste $R^1$, die an dasselbe Kohlenstoffatom gebunden sind, gemeinsam mit dem Kohlenstoffatom, an das sie gebunden sind, auch einen $(C_3-C_6)$-Cycloalkylring bilden können; wobei $NR^aR^b$ wie unten definiert sind; wobei jeder der letztgenannten drei Reste unabhängig voneinander unsubstituiert oder durch einen oder mehrere Reste aus der Gruppe Halogen, Hydroxy, $(C_1-C_4)$-Alkoxy und $(C_1-C_4)$-Alkylthio substituiert ist, bedeutet,

$R^2$ Wasserstoff, $(C_1-C_6)$-Alkyl, $(C_2-C_6)$-Alkenyl, $(C_2-C_6)$-Alkinyl, $(C_3-C_6)$-Cycloalkyl, $(C_1-C_6)$-Alkoxy, $(C_1-C_6)$-Alkylthio, $(C_1-C_6)$-Haloalkyl, $(C_2-C_6)$-Haloalkenyl, $(C_2-C_6)$-Haloalkinyl, $(C_1-C_6)$-Haloalkoxy, $(C_1-C_6)$-Haloalkylthio, $(C_3-C_6)$-Cycloalkanyloxy, $(C_3-C_6)$-Cycloalkanylthio, $(C_3-C_6)$-Cycloalkenyl, $(C_3-C_6)$-Cycloalkenyloxy, $(C_3-C_6)$-Cycloalkenylthio, Phenyl, Heteroaryl, Heterocyclyl, wobei in den vorgenannten Gruppen das Heteroaryl ein aromatischer fünf-, sechs- oder siebengliedriger Heterocyclus und das das Heterocyclyl ein gesättigter oder teilweise ungesättigter fünf-, sechs- oder siebengliedriger Heterocyclus ist, die jeweils ein, zwei, drei oder vier Heteroatome aus der Gruppe O, N und S enthalten, oder $NR^aR^b$, wobei zwei Reste $R^1$, die an dasselbe Kohlenstoffatom gebunden sind, gemeinsam mit dem Kohlenstoffatom, an das sie gebunden sind, auch einen $(C_3-C_6)$-Cycloalkylring bilden können; wobei $NR^aR^b$ wie unten definiert sind; wobei jeder der letztgenannten drei Reste unabhängig voneinander unsubstituiert oder durch einen oder mehrere Reste aus der Gruppe Halogen, Hydroxy, $(C_1-C_4)$-Alkoxy und $(C_1-C_4)$-Alkylthio substituiert ist, bedeutet,

$R^3$ Wasserstoff, $(C_1-C_6)$-Alkyl, $(C_2-C_6)$-Alkenyl, $(C_2-C_6)$-Alkinyl, $(C_3-C_6)$-Cycloalkyl, $(C_1-C_6)$-Alkoxy, $(C_1-C_6)$-Alkylthio, $(C_1-C_6)$-Haloalkyl, $(C_1-C_6)$-Haloalkenyl, $(C_2-C_{10})$-Haloalkinyl, $(C_1-C_6)$-Haloalkoxy, $(C_1-C_6)$-Haloalkylthio, $(C_3-C_6)$-Cycloalkanyloxy, $(C_3-C_6)$-Cycloalkanylthio, $(C_3-C_6)$-Cycloalkenyl, $(C_3-C_6)$-Cycloalkenyloxy, $(C_3-C_6)$-Cycloalkenylthio, Phenyl, Heteroaryl, Heterocyclyl, wobei in den vorgenannten Gruppen das Heteroaryl ein aromatischer fünf-, sechs- oder siebengliedriger Heterocyclus und das das Heterocyclyl ein gesättigter oder teilweise ungesättigter fünf-, sechs- oder siebengliedriger Heterocyclus ist, die jeweils ein, zwei, drei oder vier Heteroatome aus der Gruppe O, N und S enthalten, oder $NR^aR^b$, wobei zwei Reste $R^1$, die an dasselbe Kohlenstoffatom gebunden sind, gemeinsam mit dem Kohlenstoffatom, an das sie gebunden sind, auch einen $(C_3-C_6)$-Cycloalkylring bilden können; wobei $NR^aR^b$ wie unten definiert sind; wobei jeder der letztgenannten drei Reste unabhängig voneinander unsubstituiert oder durch einen oder mehrere Reste aus der Gruppe Halogen, Hydroxy, $(C_1-C_4)$-Alkoxy und $(C_1-C_4)$-Alkylthio substituiert ist, bedeutet,

$R^4$ Wasserstoff, $(C_1-C_6)$-Alkyl, $(C_2-C_6)$-Alkenyl, $(C_2-C_6)$-Alkinyl, $(C_3-C_6)$-Cycloalkyl, $(C_1-C_6)$-Alkoxy, $(C_1-C_6)$-Alkylthio, $(C_1-C_6)$-Haloalkyl, $(C_1-C_6)$-Haloalkenyl, $(C_2-C_6)$-Haloalkinyl, $(C_1-C_6)$-Haloalkoxy, $(C_1-C_6)$-Haloalkylthio, $(C_3-C_6)$-Cycloalkanyloxy, $(C_3-C_6)$-Cycloalkanylthio, $(C_3-C_6)$-Cycloalkenyl, $(C_3-C_6)$-Cycloalkenyloxy, $(C_3-C_6)$-Cycloalkenylthio, Phenyl, Heteroaryl, Heterocyclyl, wobei in den vorgenannten Gruppen das Heteroaryl ein aromatischer fünf-, sechs- oder siebengliedriger Heterocyclus und das das Heterocyclyl ein gesättigter oder teilweise ungesättigter fünf-, sechs- oder siebengliedriger Heterocyclus ist, die jeweils ein, zwei, drei oder vier Heteroatome aus der Gruppe O, N und S enthalten, oder $NR^aR^b$, wobei zwei Reste $R^1$, die an dasselbe Kohlenstoffatom gebunden sind, gemeinsam mit dem Kohlenstoffatom, an das sie gebunden sind, auch einen $(C_3-C_6)$-Cycloalkylring bilden können; wobei jeder der Reste $R^a$, $R^b$, $R^c$, $R^d$, $R^e$, $R^f$ und $R^e$ inklusive Substituenten 1 bis 30 C-Atome, vorzugsweise 1 bis 24 C-Atome, insbesondere 1 bis 20 C-Atome aufweist, bedeutet, jeder der letztgenannten drei Reste unabhängig voneinander unsubstituiert oder durch einen oder mehrere Reste aus der Gruppe Halogen, Hydroxy, $(C_1-C_4)$-Alkoxy und $(C_1-C_4)$-Alkylthio substituiert ist, bedeutet,

$R^5$ Wasserstoff, $(C_1-C_6)$-Alkyl, $(C_2-C_6)$-Alkenyl, $(C_2-C_6)$-Alkinyl, $(C_3-C_6)$-Cycloalkyl, $(C_1-C_6)$-Haloalkyl, $(C_2-C_6)$-Haloalkenyl, $(C_2-C_6)$-Haloalkinyl, $(C_3-C_6)$-Cycloalkenyl, wobei jeder der letztgenannten drei Reste unabhängig voneinander unsubstituiert oder durch einen oder mehrere Reste aus der Gruppe Halogen, CN, Hydroxy, $(C_1-C_4)$-Alkoxy und $(C_1-C_4)$-Alkylthio substituiert ist, bedeutet, oder einen Rest der Formel $SiR^aR^bR^c$, wobei in der letztgenannten Formel jeder der Reste $R^a$, $R^b$ und $R^c$ unabhängig voneinander Wasserstoff oder einen gegebenenfalls substituierten Kohlenwasserstoffrest bedeutet, inklusive Substituenten 1 bis 30 C-Atome, vorzugsweise 1 bis 24 C-Atome, insbesondere 1 bis 20 C-Atome aufweist, bedeutet,

$R^6$ Wasserstoff, $(C_2-C_8)$-Alkyl, $(C_2-C_8)$-Alkenyl, $(C_2-C_8)$-Alkinyl, $(C_3-C_8)$-Cycloalkyl, $(C_1-C_8)$-Haloalkyl, $(C_2-C_8)$-Haloalkenyl, $(C_2-C_8)$-Haloalkinyl, $(C_3-C_8)$-Cycloalkanyloxy, $(C_3-C_8)$-Cycloalkanylthio, $(C_3-C_8)$-Cycloalkenyl, wobei jeder der letztgenannten drei Reste unabhängig voneinander unsubstituiert oder durch einen oder mehrere Reste aus der Gruppe Halogen, Hydroxy, $(C_1-C_4)$-Alkoxy und $(C_1-C_4)$-Alkylthio substituiert ist, bedeutet,

$R^7$ Wasserstoff, $(C_1-C_7)$-Alkyl, $(C_2-C_7)$-Alkenyl, $(C_1-C_7)$-Alkinyl, $(C_3-C_7)$-Cycloalkyl, $(C_1-C_7)$-Haloalkyl, $(C_2-C_7)$-Haloalkenyl, $(C_2-C_7)$-Haloalkinyl, $(C_3-C_7)$-Cycloalkenyl, $(C_3-C_7)$-Cycloalkenyloxy, wobei jeder der letztgenannten drei Reste unabhängig voneinander unsubstituiert oder durch einen oder mehrere Reste aus der Gruppe Halogen, Hydroxy, $(C_1-C_4)$-Alkoxy und $(C_1-C_4)$-Alkylthio substituiert ist, bedeutet, oder einen Rest der Formel $SiR^aR^bR^c$, wobei in der letztgenannten Formel jeder der Reste $R^a$, $R^b$ und $R^c$ unabhängig voneinander Wasserstoff oder einen gegebenenfalls substituierten Kohlenwasserstoffrest bedeutet, inklusive Substituenten 1 bis 30 C-Atome, vorzugsweise 1 bis 24 C-Atome, insbesondere 1 bis 20 C-Atome aufweist, bedeutet,

Weiterhin bevorzugt sind Verbindungen der Formeln (Ia), (Ib), (Ic), (Id) und (Ie), worin $R^6$ und $R^7$ die für Formel (I) genannten bzw. bevorzugt genannten Bedeutungen haben.

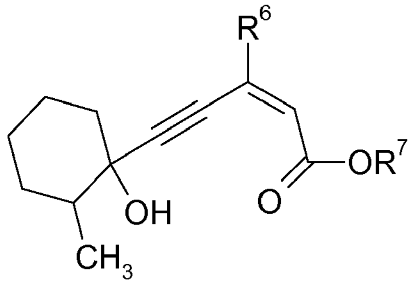

(Ia)

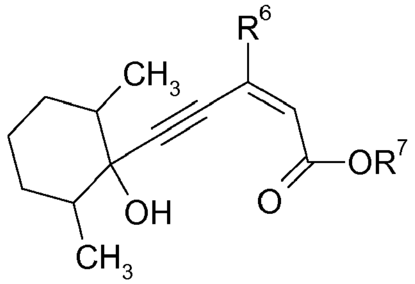

(Ib)

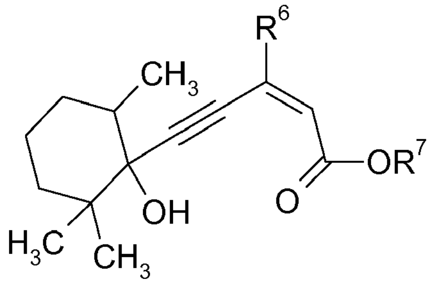

(Ic)

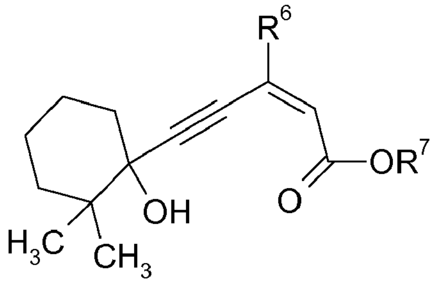

(Id)

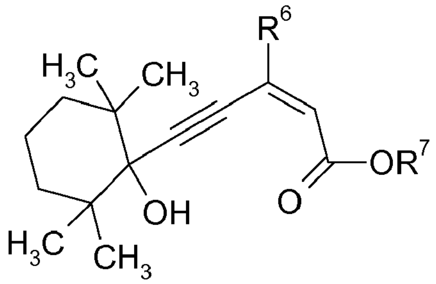

(Ie)

Dabei sind solche Verbindungen besonders bevorzugt, in welchen die Bedeutungen für [X-Y], $R^1$, $R^2$, $R^3$, $R^4$, $R^5$, $R^6$, $R^7$, $R^8$ und $R^9$ mit denen aus den weiter unten genannten Tabellen 1 bis 5 übereinstimmen oder deren allgemeineren oben genannten Bedeutungen entsprechen.

Gegenstand der Erfindung sind auch Verfahren zur Herstellung der Verbindungen der allgemeinen Formel (I) oder deren Salze.

Die Verbindungen der allgemeinen Formel (I) können beispielsweise nach folgendem Syntheseschema hergestellt werden.

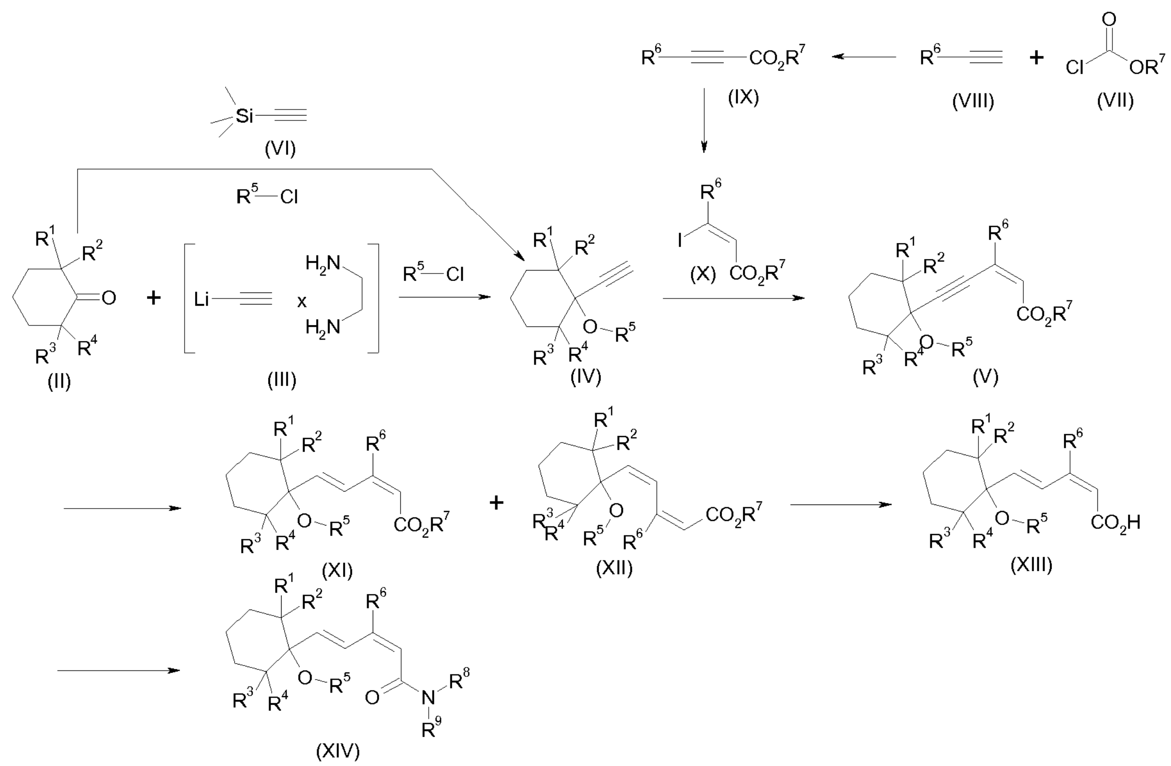


In dem Reaktionsschema sind die Reste für [X-Y], $R^1$, $R^2$, $R^3$, $R^4$, $R^5$, $R^6$, $R^7$, $R^8$ und $R^9$ wie oben beschrieben definiert, und $R^7$ ist vorzugsweise eine Methyl- oder EthylGruppe.

Die Verbindungen der allgemeinen Formeln (II) sind entweder kommerziell erhältlich oder können nach oder analog dem Fachmann bekannten Methoden hergestellt werden (z. B. Houben-Weyl, Bd. 7/2a, 1973, 677-788; Curr. Org. Chem. 2003,7, 967-993). Eine Verbindung der Formel (IV) ist entweder kommerziell erhältlich oder lässt sich durch Reaktion von einer Verbindung der Formel (III) in einem adäquaten Lösungsmittel wie zum Beispiel Benzol herstellen (z. B. Journal of Organic Chemistry 1964, 29, 1872-1876 und Journal of American Chemical Society 1992, 114, 2544-2559). Die Reaktion findet bevorzugt in dem Temperaturbereich zwischen -20°C und 100°C statt. Alternativ lässt sich eine Verbindung der Formel (IV) durch Reaktion mit der Verbindung der Formel (VI) in Gegenwart einer Base wie Lithiumdiisopropylamid (LDA) in einem adäquaten Lösungsmittel wie zum Beispiel Tetrahydrofuran (THF) gefolgt von einer Abspaltung der Trimethylsilylgruppe in Gegenwart einer Base wie Kaliumcarbonat in einem Alkohol (wie zum Beispiel Methanol) herstellen (z. B. Journal of American Chemical Society 1987, 109, 4717; Chemical Communications 1985, 958; Org. Biomol. Chem. 2006, 4, 4186-4192). Die Reaktion findet bevorzugt in dem Temperaturbereich zwischen -20°C und 100°C statt. Eine Verbindung der Formel (V) lässt sich beispielsweise durch Reaktion von einer Verbindung der Formel (IV) in einem geeigneten Lösungsmittel mit einem Vinyliodid der Formel (X) unter Zusatz einer adäquaten Menge eines Übergangsmetallkatalysators, insbesondere Palladium-katalysatoren wie Bis(triphenylphosphin)palladium(II)dichlorid oder Tetrakis(triphenylphosphin)palladium(0) vorzugsweise bei Raumtemperatur in Gegenwart von Kupfer(I)iodid und einer organischen Base wie zum Beispiel Diethylamin in einem organischen Lösungsmittel wie Toluol darstellen. Die geometrischen Isomere des Vinyliodides der Formel (X) lassen sich ebenfalls in der oben beschriebenen übergangsmetallkatalysierten Reaktion einsetzen. Eine Synthese der allgemeinen Formel (V) ist in Journal of Fluorine Chemistry 101 (2000) 31-33, Tetrahedron Letters 1997 38 (38) 6729-6732, Journal of the American Chemical Society 1997 119 (4) 698-708, Tetrahedron Letters 1996 37 (23) 3971-3974 und Tetrahedron Letters 1985 26 (24) 2881-2884 beschrieben. Allgemein eignen sich Methoden von Kreuzkupplungen, die in R. D. Larsen, Organometallics in Process Chemistry 2004 Springer Verlag, die in I. Tsuji, Palladium Reagents and Catalysts 2004 Wiley, die in M. Beller, C. Bolm, Transition Metals for Organic Synthesis 2004 VCH-Wiley und die in E.-i Negishi, C. Xu, Handbook of Organopalladium Chemistry for Organic Synthesis 2002, 1, 531-549 beschrieben werden. Weitere geeignete Synthesemethoden sind in Chem. Rev. 2003, 103, 1979-2017 , Pure Appl. Chem. 2004, 76, 565-576; Tetrahedron 2009, 65, 8418-8427; Journal of Combinatorial Chemistry 2009, 11, 900-906 und Org. Biomol. Chem. 2006, 4, 4186-4192 beschrieben. Die Z-Vinyliodide der allgemeinen Formel (X) sind entweder kommerziell erhältlich oder können nach oder analog dem Fachmann bekannten Methoden hergestellt werden (z. B. Journal of Fluorine Chemistry 1981, 17, 249; Organic Letters 2000, 2, 3407-3410; US 6153786 A1, 2000 und Tetrahedron letters 2008, 49 (5) 794-798). Die Reduktion zu Verbindungen der allgemeinen Formel (XII) lässt sich in Gegenwart eines Übergangsmetallkatalysators wie zum Beispiel Lindlars Katalysator mit Wasserstoff in einem Alkohol (wie z. B. n-Butanol) durchführen (Tetrahedron 1987, 43, 4107; Tetrahedron 1983, 39, 2315; Journal of Organic Synthesis 1983, 48, 4436 und Journal of the American Chemical Society 1984, 106, 2735). Eine Verbindung der allgemeinen Formelen (XI) und (XII) lassen sich durch Reduktion einer korrespondierenden Verbindung (V) mit Natrium-bis-(2-methoxyethoxy)-aluminiumhydrid (Red-Al®, Vitrid®) in einem geeigneten Lösungsmittel wie Tetrahydrofuran herstellen (z. B. Helvetica Chimica Acta 1978, 61, 2616-2627; Org. Biomol. Chem. 2006, 4, 4186-4192; Bioorg. Med. Chem. 2004, 12, 363-370; Tetrahedron 2003, 59, 9091-9100 und Org. Biomol. Chem. 2006, 4, 1400-1412). Alternativ lassen sich als Reduktionsmittel auch Lithiumaluminiumhydrid (z. B. Synthesis 1977, 561; Tetrahedron Letters 1992, 33, 3477 und Tetrahedron Letters 1974, 1593), Natriumborhydrid in einem Alkohol (z. B. Methanol oder Ethanol) (z. B. Organic Letters 2004, 6, 1785), Lithium gelöst in einem Gemisch aus Ethylamin und tert.-Butanol (z. B. Helvetica Chimica Acta 1986, 69, 368) oder unter Nutzung eines geeigneten Trialkoxysilans in Gegenwart einer adäquaten Menge eines Übergangsmetallkatalysators (z. B. Tris-(cyano)ruthenium-1,2,3,4,5-pentacyclopentadienylhexafluorophosphat oder Tris-(cyano)ruthenium-cyclopentadienylhexafluorophosphat; Journal of the American Chemical Society 2002, 124, 7622 und Journal of the American Chemical Society 2005, 127, 17645) verwenden. In Abhängigkeit von den Reaktionsbedingungen können bei den Hydrierungen der Dreifachbindung als weitere Reaktionsprodukte auch die entsprechenden erfindungsgemäßen (E,E)-konfigurierten Verbindungen der allgemeinen Formel (XII) enstehen. Die Verbindungen der allgemeinen Formeln (V, XI und XII) lassen nach oder analog dem Fachmann bekannten Methoden zu den entsprechenden Carbonsäuren (wie z. B. XIII) verseifen. Die Verseifung lässt sich in Gegenwart einer Base oder einer Lewis-Säure durchführen. Die Base kann ein Hydroxid-Salz von einem Alkali-Metall (wie zum Beispiel Lithium, Natrium oder Kalium) sein, und die Verseifungsreaktion findet bevorzugt in dem Temperaturbereich zwischen Raumtemperatur und 100°C statt. Die Lewis-Säure kann Bortribromid sein, und die Reaktion in einem Temperaturbereich zwischen -20°C und 100°C, vorzugsweise -5°C und 50 °C durchgeführt werden. Die Verbindungen der Formel (XIII) lassen sich gegebenenfalls mit einem Amin in Gegenwart eines eines wasserentziehenden Mittels, beispielsweise die Dicyclohexylcarbodiimid (DCC), zur Verbindung der Formel (XIV) umsetzen. Als organische Lösungsmittel eignen sich beispielsweise polare protische oder aprotische Lösungsmittel wie z. B. Dichlormethan, Trichlormethan, Pyridin, Tetrahydrofuran und Dioxan, oder Nitrile wie Acetonitril, oder Amide wie Dimethylformamid bzw. deren Mischungen und die Reaktion in einem Temperaturbereich zwischen -20°C und 50 °C, vorzugsweise -10 °C und 30°C durchgeführt wird. Allgemein eignen sich Methoden zur Synthese von Peptiden, die in N. Leo Benoiton, Chemistry of Peptide Synthesis 2006 CRC Press, beschrieben werden.

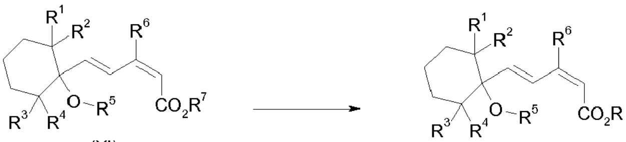

(XI) (XVI)

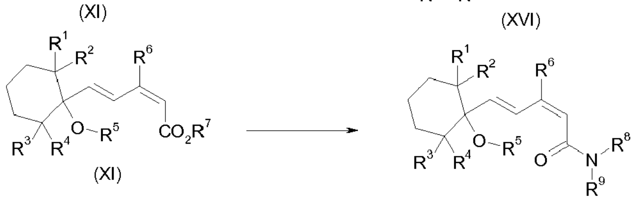

(XI) (XIV)

Alternativ lassen sich Verbindungen der Formel (XV) durch Umesterungen von Estern der Formel (XI) in Gegenwart einer Broensted-Säure oder einer Lewis-Säure herstellen. Die Broensted-Säure kann Schwefelsäure oder ein Wasserstoffhalogenid gelöst in einem Alkohol (wie zum Beispiel Methanol, Ethanol, n-Propanol, 2-Propanol oder n-Butanol) sein. Die Umesterung findet bevorzugt in dem Temperaturbereich zwischen Raumtemperatur und 100 °C statt. Die Lewis-Säure kann Titantetraisopropoxylat sein und die Reaktion in einem Temperaturbereich zwischen 0 °C und 150 °C, vorzugsweise 0 °C und 100 °C durchgeführt wird. Weiterhin lassen sich Verbindungen der Formel (XIV) durch Aminolyse von Estern der Formel (XI) in Gegenwart des entsprechenden Amines (XV) in einem Alkohol (wie zum Beispiel Methanol, Ethanol, n-Propanol, 2-Propanol oder n-Butanol) herstellen. Die Aminolyse findet bevorzugt in dem Temperaturbereich zwischen Raumtemperatur und 100 °C statt.

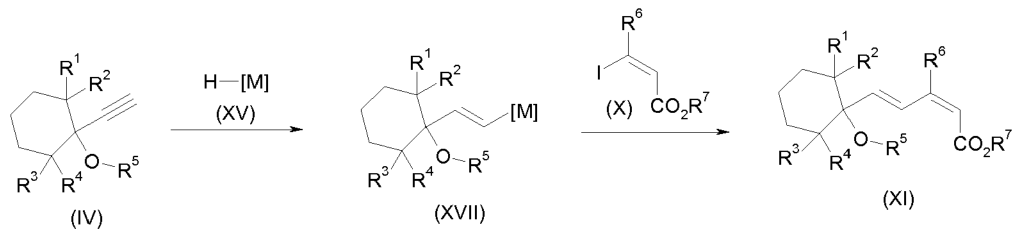


Alternativ lassen sich Verbindungen der Formel (XI) durch eine Metall- oder Halbmetallhydrid-vermittelte Überführung der Verbindungen der allgemeinen Formel (IV) in einem geeigneten Lösungsmittel (z. B. Tetrahydrofuran oder Dichlormethan) in die entsprechenden (E)-[M]-1-Vinyl-Verbindungen der allgemeinen Formel (XVII) (z. B. Organic Letters 2002, 4, 703 und Angewandte Chemie Int. Ed. 2006, 45, 2916), wobei [M] für eine weiter substituierte Metall- oder Halbmetallkomponente aus der Reihe Zinn, Germanium, Blei, Bor, Aluminium oder Zirconium steht (z. B. [M] = Tri-n-butylstannyl oder Bis-Cyclopentadienylchlorzirconyl; Organic Letters 2010, 12, 1056 und Organic Letters 2005, 7, 5191). Die so erhaltenden Verbindungen der allgemeinen Formel (XVII) können durch eine Kupplung mit einem entsprechend substituierten Vinyliodid der allgemeinen Formel (X) oder deren geometrischen Isomeren in einem geeigneten Lösungsmittel (wie zum Beispiel Tetrahydrofuran oder N,N-Dimethylformamid) unter Zusatz einer adäquaten Menge eines Übergangsmetallkatalysators, insbesondere Palladium-katalysatoren wie Bis(triphenylphosphin)palladium(II)dichlorid oder Tetrakis(triphenylphosphin)palladium(0) zu den Verbindungen der Formel (XI) umsetzen.

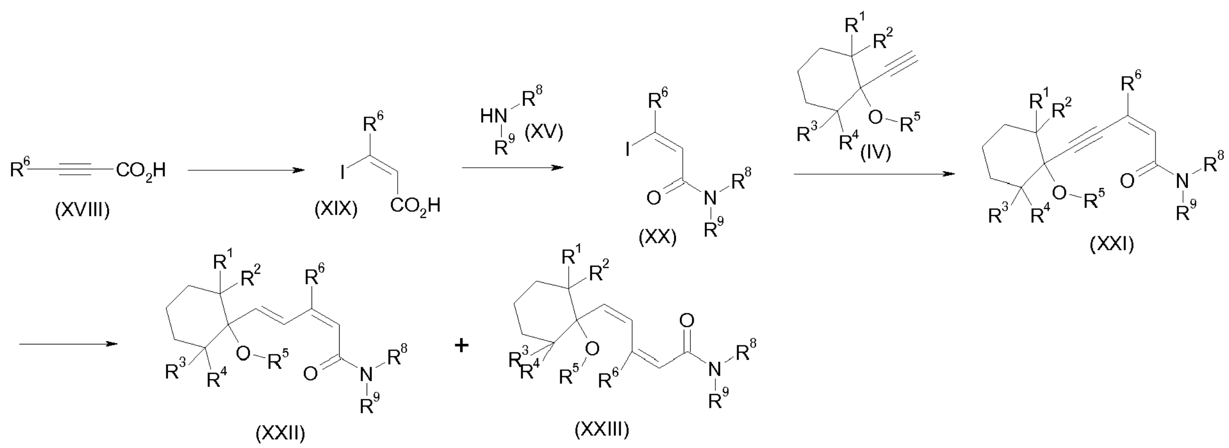


Alternativ lassen sich die Vinyliodid der allgemeinen Formel (XIX) aus den Säuren der allgemeinen Formel (XVIII) herstellen. Die Verbindungen der Formel (XIX) lassen sich gegebenenfalls mit einem Amin in Gegenwart eines eines wasserentziehenden Mittels, beispielsweise die Dicyclohexylcarbodiimid (DCC), zur Verbindung der Formel (XX) umsetzen. Als organische Lösungsmittel eignen sich beispielsweise polare protische oder aprotische Lösungsmittel wie z. B. Dichlormethan, Trichlormethan, Pyridin, Tetrahydrofuran und Dioxan, oder Nitrile wie Acetonitril, oder Amide wie Dimethylformamid bzw. deren Mischungen und die Reaktion in einem Temperaturbereich zwischen -20 °C und 50 °C, vorzugsweise -10 °C und 30 °C durchgeführt wird. Allgemein eignen sich Methoden zur Synthese von Peptiden, die in N. Leo Benoiton, Chemistry of Peptide Synthesis 2006 CRC Press, beschrieben werden. Diese lassen sich dann wie bereits in einer oben beschriebenen übergangsmetallkatalysierten Reaktion mit Verbindungen der allgemeinen Formel (IV) zu einer Verbindung der allgemeinen Formel (XXI) umsetzen. Die Reduktion der Verbindung der allgemeinen Formel (XXI) zu den Verbindungen der allgemeinen Formeln (XXII) und (XXIII) erfolgt ebenfalls gemäß den oben beschriebenen Verfahren.

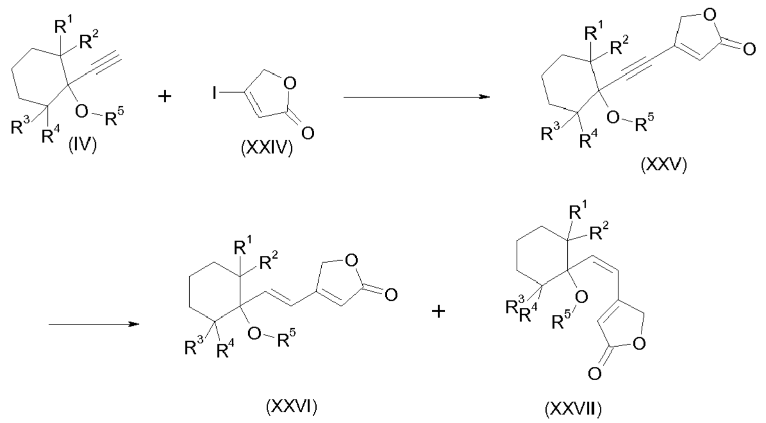

(IV) (XXIV) (XXV)

(XXVI) (XXVII)

Weiterhin lassen sich auch cyclische Vinyliodverbindungen der allgemeinen Formel (XXIV) mit Verbindungen der allgemeinen Formel (IV) nach den oben beschriebenen Verfahren zu den Verbindungen der allgemeinen Formel (XXV) kuppeln. Die Reduktion zu den (E)- bzw. (Z)-Olefinen (XXVI) und (XXVII) erfolgt ebenfalls nach den oben beschriebenen Verfahren.

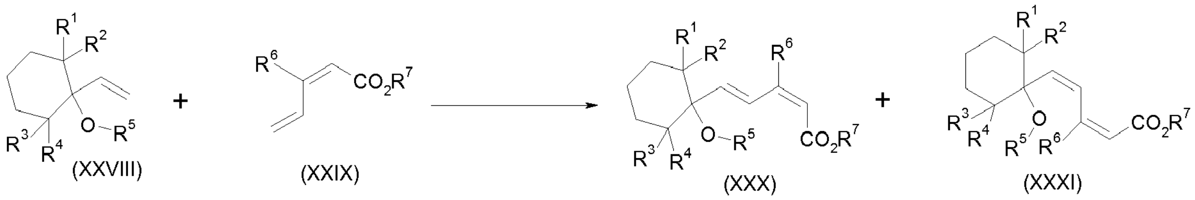

(XXVIII) (XXIX) (XXX) (XXXI)

Die Verbindungen der allgemeinen Formeln (XXVIII) und (XXIX) oder dessen geometrisches Isomer lassen sich in einem geeigneten Lösungsmittel (wie zum Beispiel Dichlormethan) unter Zusatz einer adäquaten Menge eines Übergangsmetallkatalysators, insbesondere Ruthenium-Katalysatoren wie Benzyliden-bis-(tricyclohexylphosphin)-dichlororuthenium und (1,3-Bis-(2,4,6-trimethylphenyl)-2-imidazolidinyliden)-dichlor-(phenylmethylen)-(tricyclohexylphosphin)-ruthenium zu den Verbindungen der Formel (XXX) und (XXXI) umsetzen.

Weiterhin kann die Verbindung der allgemeinen Formel (XXX) ausgehend von S-Abscisinsäure herstellen, Biooganic Medicinal Chemistry 2007 15 (18) 6311-6322.

Die Verfahrensmaßnahmen und bevorzugten Varianten für die Herstellungen sind weiter oben näher beschrieben. Die angegebenen Beispielnummern entsprechen denen in den nachstehenden Tabellen 1 bis 5.

Herstellbeispiele

Beispiel A1 (Tabelle 3, Beispiel Nr. 3-4)

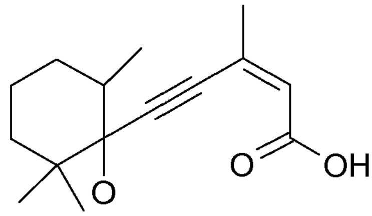

(2Z)-5-(1-Hydroxy-2,2,6-trimethylcyclohexyl)-3-methylpent-2-en-4-insäure

A1 1) Ethyl-(2Z)-3-iodbut-2-enoat

10,9 g (97,22 mmol) Ethylbut-2-inoat werden in 35 ml Essigsäure unter Stickstoff-Inertgasatmosphäre gelöst und mit 23,3 g Natriumiodid (155,54 mmol) versetzt. Die gelbe Lösung erhitzt man 1 h zum Sieden. Nach dem Abkühlen

verdünnt man die Lösung mit Wasser und extrahiert die wässrige Phase mit Diethylether. Man trennt die Phasen, wäscht die organische Phase mit gesättigter NatriumhydrogencarbonatLösung, trocknet die organische Phase über Magnesiumsulfat und entfernt das Lösungsmittel im Vakuum. Man erhält 17,6 g (75 %) des gewünschten Produktes. $^1$H-NMR (400 MHz, $CDCl_3$ δ, ppm) 6.29 (s, 1H); 4.22 (q, 2H); 2.73 (s, 3H); 1.30 (t, 3H).

A1 2) Ethyl-(2Z)-5-(1-hydroxy-2,2,6-trimethylcyclohexyl)-3-methylpent-2-en-4-inoat

428,5 mg (2,50 mmol) 1-Ethinyl-2,2,6-trimethylcyclohexanol, 720,1 mg (3,00 mmol) Ethyl-(2Z)-3-iodbut-2-enoat, 71,42 mg (0,375 mmol) Kupfer-(I)-iodid und 175,48 mg (0,250 mmol) Bis(triphenylphosphin)palladiumdichlorid werden unter Stickstoff-Inertgasatmosphäre in 10 ml Toluol suspendiert. Die Suspension versetzt man mit 0,701 ml (5,00 mmol) Diisopropylamin und rührt die Suspension 5 Stunden bei Raumtemperatur. Man verdünnt die Reaktionslösung mit Diethylether, extrahiert mit einer gesättigten Ammoniumchlorid-Lösung, trocknet die organische Phase über Magnesiumsulfat und entfernt das Lösungsmittel im Vakuum. Das Rohprodukt reinigt man durch Chromatographie (Ethylacetat: n-Heptan 1:3).

Man erhält 433 mg (61 %) des gewünschten Produktes. $^1$H-NMR (400 MHz, $CDCl_3$ δ ppm) 5.98 (s, 1H), 4.19 (q, 2H), 2.30 (s, 1H), 2.04 (s, 3H), 1.91 (m, 1H), 1.65 (m, 2H), 1.51-1.35 (m, 4H), 1.28 (t, 3H), 1.12 (s, 3H), 1.07 (d, 3H), 1.03 (s, 3H).

A1 3) (2Z)-5-(1-Hydroxy-2,2,6-trimethylcyclohexyl)-3-methylpent-2-en-4-insäure

189 mg (0,679 mmol) Ethyl-(2Z)-5-(1-hydroxy-2,2,6-trimethylcyclohexyl)-3-methylpent-2-en-4-inoat werden in 4 ml Tetrahydrofuran gelöst und mit 1 ml 6 M Natronlauge versetzt. Die Lösung erhitzt man 11 h auf 80 °C, kühlt auf Raumtemperatur ab und säuert mit 6 M Salzsäure an. Die wäßrige Phase extrahiert man mit Ethylacetat. Man erhält 141 mg (81 %) des gewünschten Produktes. $^1$H-NMR (400 MHz, $CDCl_3$ δ, ppm) 6.01 (s, 1H); 2.09 (s, 3H); 1.92 (m, 1H); 1.69-1.31 (m, 6H); 1,12 (s, 3H); 1.06 (d, 3H); 1.02 (s, 3H).

Beispiel A2 (Tabelle 3, Beispiel Nr. 3-10)

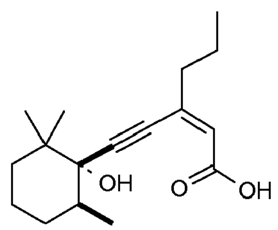

(2Z)-3-[(1-Hydroxy-2,2,6-trimethylcyclohexyl)ethinyl]hex-2-ensäure

A21) (2Z)-3-[(1-Hydroxy-2,2,6-trimethylcyclohexyl)ethinyl]hex-2-ensäure

2,2,6-Trimethylcyclohexanon (200 mg, 1.43 mmol) wurde in einem Rundkolben unter Argon in abs. Tetrahydrofuran (3 ml) gelöst und tropfenweise zu einer Lösung eines Lithiumacetylid-Ethylendiaminkomplexes (171 mg, 1.85 mmol, 85% Gehalt) in abs. Tetrahydrofuran (20 ml) gegeben. Die Reaktionslösung wurde nach erfolgter Zugabe 2 h lang bei Raumtemperatur gerührt, anschließend mit Wasser versetzt und unter vermindertem Druck eingeengt. Der verbleibende Rückstand wurde mit Wasser und Dichlormethan versetzt und die wässrige Phase mehrfach mit Dichlormethan extrahiert. Die vereinigten organischen Phasen wurden über Magnesiumsulfat getrocknet, filtriert und unter vermindertem Druck eingeengt. Durch säulenchromatographische Reinigung des erhaltenen Rohproduktes (Gradient Essigester/Heptan) wurde 1-Ethinyl-2,2,6-trimethylcyclohexanol (198 mg, 84 % der Theorie) als farbloses Öl isoliert. Anschließend wurden Kupfer(I)iodid (35 mg, 0.18 mmol) und Bis(triphenylphosphin)palladium(II)chlorid (95 mg, 0.14 mmol) unter Argon in einem ausgeheizten Rundkolben vorgelegt und mit abs. Toluol (3 ml) sowie Ethyl-(2Z)-3-iodhex-2-enoat (242 mg, 0.90 mmol) versetzt. Nach 10 Min Rühren bei Raumtemperatur erfolgte die tropfenweise Zugabe einer Lösung von 1-Ethinyl-2,2,6-trimethylcyclohexanol (150 mg, 0.90 mmol) in abs. Toluol (1 ml) und von Diisopropylamin (0.25 ml, 1.80 mmol). Das resultierende Reaktionsgemisch wurde 3 h lang bei Raumtemperatur gerührt und danach mit Wasser versetzt. Die wässrige Phase wurde mehrfach mit Dichlormethan extrahiert. Die vereinigten organischen Phasen wurden über Magnesiumsulfat getrocknet, filtriert und unter vermindertem Druck eingeengt. Durch abschließende säulenchromato-graphische Reinigung des erhaltenen Rohproduktes (unter Verwendung eines Essigester/Heptan-Gradienten) wurde Ethyl-(2Z)-3-{[1-hydroxy-2,2,6-trimethylcyclohexyl]ethinyl}hex-2-enoat (290 mg, 99 % der Theorie) in Form eines farblosen Öles isoliert. $^1$H-NMR (400 MHz, $CDCl_3$ $\delta$, ppm) 5.96 (s, 1H), 4.19 (q, 2H), 2.27 (t, 2H), 1.93 (m, 1H), 1.62 (m, 4H), 1.48 (m, 2H), 1.35 (m, 2H), 1.28 (t, 3H), 1.14 (s, 3H), 1.11 (m, 1H), 1.08 (d, 3H), 1.03 (s, 3H), 0.92 (t, 3H).

A2 2) (2Z)-3-[(1-Hydroxy-2,2,6-trimethylcyclohexyl)ethinyl]hex-2-ensäure

Ethyl-(2Z)-3-{[1-hydroxy-2,2,6-trimethylcyclohexyl]ethinyl}hex-2-enoat (150 mg, 0.49 mmol) wurde in Methanol (3 ml) gelöst und mit fein gepulvertem Kaliumhydroxid (86 mg, 1.54 mmol) versetzt. Das resultierende Reaktionsgemisch wurde 2 h lang bei einer Temperatur von 50 °C gerührt und nach dem Abkühlen auf Raumtemperatur mit Wasser versetzt und mit verd. Salzsäure auf pH 7 eingestellt. Die wässrige Phase wurde mehrfach mit Dichlormethan extrahiert. Die vereinigten organischen Phasen wurden über Magnesiumsulfat getrocknet, filtriert und unter vermindertem Druck eingeengt. Durch abschließende säulenchromatographische Reinigung des erhaltenen Rohproduktes (unter Verwendung eines Essigester/Heptan-Gradienten) wurde (2Z)-3-[(1-Hydroxy-2,2,6-trimethylcyclohexyl)ethin-yl]hex-2-en-säure (290 mg, 99 % der Theorie) in Form eines schwach gelblichen Feststoffes isoliert. $^1$H-NMR (400 MHz, $CDCl_3$ $\delta$, ppm) 6.00 (s, 1H), 2.30 (q, 2H), 1.92 (m, 1H), 1.63 (m, 2H), 1.48-1.38 (m, 3H), 1.37-1.30 (m, 3H), 1.12 (s, 3H), 1.08 (d, 3H), 1.03 (s, 3H), 0.94 (t, 3H).

Beispiel A3 (Tabelle 4, Beispiel Nr. 4-9)

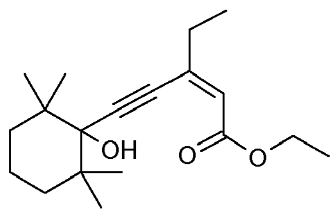

Ethyl-(2Z)-3-ethyl-5-(1-hydroxy-2,2,6,6-tetramethylcyclohexyl)pent-2-en-4-inoat

A3 1) Ethyl-(2Z)-3-ethyl-5-(1-hydroxy-2,2,6,6-tetramethylcyclohexyl)pent-2-en-4-inoat

2,2,6,6-Tetramethylcyclohexanon (1.20 g, 7.78 mmol) wurde in einem Rundkolben unter Argon in abs. Tetrahydrofuran (8 ml) gelöst und tropfenweise zu einer Lösung eines Lithiumacetylid-Ethylendiaminkomplexes (1.16 g, 10.11 mmol, 80% Gehalt) in abs. Tetrahydrofuran (20 ml) gegeben. Die Reaktionslösung wurde nach erfolgter Zugabe 3 h lang bei Raumtemperatur gerührt, anschließend mit Wasser versetzt und unter vermindertem Druck eingeengt. Der verbleibende Rückstand wurde mit Wasser und Dichlormethan versetzt und die wässrige Phase mehrfach mit Dichlormethan extrahiert. Die vereinigten organischen Phasen wurden über Magnesiumsulfat getrocknet, filtriert und unter vermindertem Druck eingeengt. Durch säulenchromatographische Reinigung des erhaltenen Rohproduktes (Gradient Essigester/Heptan) wurde 1-Ethinyl-2,2,6,6-tetramethylcyclohexanol (1.40 g, 95 % der Theorie) als farbloses Öl isoliert. Anschließend wurden Kupfer(I)iodid (32 mg, 0.17 mmol) und Bis(triphenylphosphin)palladium(II)chlorid (88 mg, 0.13 mmol) unter Argon in einem ausgeheizten Rundkolben vorgelegt und mit abs. Toluol (3 ml) sowie Ethyl-(2Z)-3-iodpent-2-enoat (211 mg, 0.83 mmol) versetzt. Nach 10 Min Rühren bei Raumtemperatur erfolgte die tropfenweise Zugabe einer Lösung von 1-Ethinyl-2,2,6,6-tetramethylcyclo-hexanol (150 mg, 0.83 mmol) in abs. Toluol (1 ml) und von Diisopropylamin (0.23 ml, 1.66 mmol). Das resultierende Reaktionsgemisch wurde 3 h lang bei Raumtemperatur gerührt und danach mit Wasser versetzt. Die wässrige Phase wurde mehrfach mit Dichlormethan extrahiert. Die vereinigten organischen Phasen wurden über Magnesiumsulfat getrocknet, filtriert und unter vermindertem Druck eingeengt. Durch abschließende säulenchromat-ographische Reinigung des erhaltenen Rohproduktes (unter Verwendung eines Essigester/Heptan-Gradienten) wurde Ethyl-(2Z)-3-ethyl-5-(1-hydroxy-2,2,6,6-tetramethylcyclo-hexyl)pent-2-en-4-inoat (180 mg, 67 % der Theorie) in Form eines farblosen Öles isoliert. $^1$H-NMR (400 MHz, $CDCl_3$ $\delta$, ppm) 5.98 (s, 1H), 4.19 (q, 2H), 2.31 (q, 2H), 1.55 (m, 3H), 1.35 (m, 3H), 1.28 (t, 3H), 1.20 (s, 6H), 1.16 (m, 3H), 1.12 (s, 6H).

In der Tabelle bedeuten:

| | | | |
|---|---|---|---|
| Bu = | Butyl | Et = | Ethyl |
| Me = | Methyl | Ph = | Phenyl |

(fortgesetzt)

| | | | |
|---|---|---|---|
| Pr = | Propyl | | |
| i = | iso | s = | sekundär |
| t = | tertiär | c = | cyclo |

Entsprechendes gilt für die zusammengesetzten Ausdrücke wie

| | |
|---|---|
| iPr = | Isopropyl |
| iBu = | Isobutyl |
| sBu = | sec.-Butyl |
| tBu = | tert.-Butyl |
| cPr = | Cyclopropyl |
| cPentyl = | Cyclopentyl |
| cHexyl = | Cyclohexyl |

Ist in den Tabellen ein Alkylrest ohne weitere Kennzeichnung aufgeführt, so handelt es sich um den geradkettigen Alkylrest, d. h. beispielsweise Bu = n-Bu = n-Butyl.

Die Zahlenindizes in den Formelausdrücken sind in der Tabelle nicht tiefgestellt, sondern in derselben Zeilenhöhe und Schriftgröße wie die Atomsymbole angeordnet.

Beispielsweise entspricht die Formel CF3 in der Tabelle der Formel $CF_3$ gemäß üblicher Schreibweise mit tiefgestelltem Index oder die Formel CH2CH(CH2CH3)2 der Formel $CH_2CH(CH_2CH_3)_2$ mit tiefgestellten Indices.

In Analogie zu oben angeführten und in den nachstehenden Tabellen rezitierten Herstellungsbeispielen und unter Berücksichtigung der allgemeinen Angaben zur Herstellung von substituierten (2Z)-5(1-Hydroxycyclohexyl)pent-2-en-4-insäure-Derivaten sowie deren Analoga der allgemeinen Formel (I) erhält man folgende in den Tabellen 1 bis 5 spezifisch genannten Verbindungen:

Tabelle 1: Verbindungen der Formel (I)

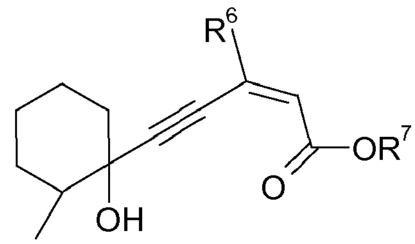

(I)

| Beispiel Nr. | $R^6$ | $R^7$ |
|---|---|---|
| 1-1 | H | H |
| 1-2 | H | Me |
| 1-3 | H | Et |
| 1-4 | Me | H |
| 1-5 | Me | Me |
| 1-6 | Me | Et |
| 1-7 | Et | H |
| 1-8 | Et | Me |
| 1-9 | Et | Et |
| 1-10 | n-Pr | H |
| 1-11 | n-Pr | Me |
| 1-12 | n-Pr | Et |
| 1-13 | i-Pr | H |

(fortgesetzt)

| Beispiel Nr. | $R^6$ | $R^7$ |
|---|---|---|
| 1-14 | i-Pr | Me |
| 1-15 | i-Pr | Et |
| 1-16 | c-Pr | H |
| 1-17 | c-Pr | Me |
| 1-18 | c-Pr | Et |
| 1-19 | n-Bu | H |
| 1-20 | n-Bu | Me |
| 1-21 | n-Bu | Et |
| 1-22 | i-Bu | H |
| 1-23 | i-Bu | Me |
| 1-24 | i-Bu | Et |
| 1-25 | CF3 | H |
| 1-26 | CF3 | Me |
| 1-27 | CF3 | Et |

Tabelle 2: Verbindungen der Formel (I)

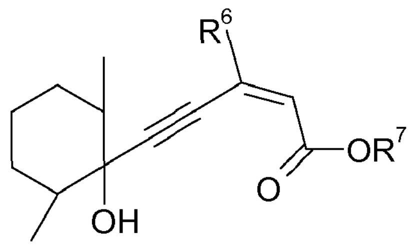

(I)

| Beispiel Nr. | $R^6$ | $R^7$ |
|---|---|---|
| 2-1 | H | H |
| 2-2 | H | Me |
| 2-3 | H | Et |
| 2-4 | Me | H |
| 2-5 | Me | Me |
| 2-6 | Me | Et |
| 2-7 | Et | H |
| 2-8 | Et | Me |
| 2-9 | Et | Et |
| 2-10 | n-Pr | H |
| 2-11 | n-Pr | Me |
| 2-12 | n-Pr | Et |
| 2-13 | i-Pr | H |
| 2-14 | i-Pr | Me |
| 2-15 | i-Pr | Et |

(fortgesetzt)

| Beispiel Nr. | $R^6$ | $R^7$ |
|---|---|---|
| 2-16 | c-Pr | H |
| 2-17 | c-Pr | Me |
| 2-18 | c-Pr | Et |
| 2-19 | n-Bu | H |
| 2-20 | n-Bu | Me |
| 2-21 | n-Bu | Et |
| 2-22 | i-Bu | H |
| 2-23 | i-Bu | Me |
| 2-24 | i-Bu | Et |
| 2-25 | CF3 | H |
| 2-26 | CF3 | Me |
| 2-27 | CF3 | Et |

Tabelle 3: Verbindungen der Formel (I)

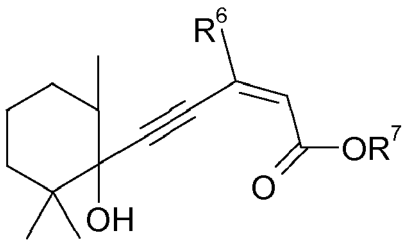

(I)

| Beispiel Nr. | $R^6$ | $R^7$ |
|---|---|---|
| 3-1 | H | H |
| 3-2 | H | Me |
| 3-3 | H | Et |
| 3-4 | Me | H |
| 3-5 | Me | Me |
| 3-6 | Me | Et |
| 3-7 | Et | H |
| 3-8 | Et | Me |
| 3-9 | Et | Et |
| 3-10 | n-Pr | H |
| 3-11 | n-Pr | Me |
| 3-12 | n-Pr | Et |
| 3-13 | i-Pr | H |
| 3-14 | i-Pr | Me |
| 3-15 | i-Pr | Et |
| 3-16 | c-Pr | H |
| 3-17 | c-Pr | Me |

(fortgesetzt)

| Beispiel Nr. | $R^6$ | $R^7$ |
|---|---|---|
| 3-18 | c-Pr | Et |
| 3-19 | n-Bu | H |
| 3-20 | n-Bu | Me |
| 3-21 | n-Bu | Et |
| 3-22 | i-Bu | H |
| 3-23 | i-Bu | Me |
| 3-24 | i-Bu | Et |
| 3-25 | CF3 | H |
| 3-26 | CF3 | Me |
| 3-27 | CF3 | Et |

Tabelle 4: Verbindungen der Formel (I)

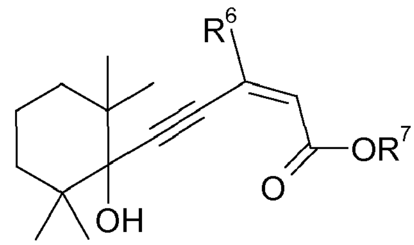

(I)

| Beispiel Nr. | $R^6$ | $R^7$ |
|---|---|---|
| 4-1 | H | H |
| 4-2 | H | Me |
| 4-3 | H | Et |
| 4-4 | Me | H |
| 4-5 | Me | Me |
| 4-6 | Me | Et |
| 4-7 | Et | H |
| 4-8 | Et | Me |
| 4-9 | Et | Et |
| 4-10 | n-Pr | H |
| 4-11 | n-Pr | Me |
| 4-12 | n-Pr | Et |
| 4-13 | i-Pr | H |
| 4-14 | i-Pr | Me |
| 4-15 | i-Pr | Et |
| 4-16 | c-Pr | H |
| 4-17 | c-Pr | Me |
| 4-18 | c-Pr | Et |
| 4-19 | n-Bu | H |

(fortgesetzt)

| Beispiel Nr. | $R^6$ | $R^7$ |
|---|---|---|
| 4-20 | n-Bu | Me |
| 4-21 | n-Bu | Et |
| 4-22 | i-Bu | H |
| 4-23 | i-Bu | Me |
| 4-24 | i-Bu | Et |
| 4-25 | CF3 | H |
| 4-26 | CF3 | Me |
| 4-27 | CF3 | Et |

Tabelle 5: Verbindungen der Formel (I)

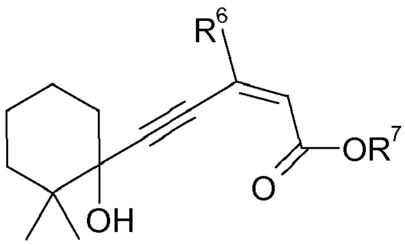

(I)

| Beispiel Nr. | $R^6$ | $R^7$ |
|---|---|---|
| 5-1 | H | H |
| 5-2 | H | Me |
| 5-3 | H | Et |
| 5-4 | Me | H |
| 5-5 | Me | Me |
| 5-6 | Me | Et |
| 5-7 | Et | H |
| 5-8 | Et | Me |
| 5-9 | Et | Et |
| 5-10 | n-Pr | H |
| 5-11 | n-Pr | Me |
| 5-12 | n-Pr | Et |
| 5-13 | i-Pr | H |
| 5-14 | i-Pr | Me |
| 5-15 | i-Pr | Et |
| 5-16 | c-Pr | H |
| 5-17 | c-Pr | Me |
| 5-18 | c-Pr | Et |
| 5-19 | n-Bu | H |
| 5-20 | n-Bu | Me |
| 5-21 | n-Bu | Et |

(fortgesetzt)

| Beispiel Nr. | $R^6$ | $R^7$ |
|---|---|---|
| 5-22 | i-Bu | H |
| 5-23 | i-Bu | Me |
| 5-24 | i-Bu | Et |
| 5-25 | CF3 | H |
| 5-26 | CF3 | Me |
| 5-27 | CF3 | Et |

Die $^1$H-NMR- und $^{13}$C-NMR- NMR-spektroskopischen Daten, die für die in den nachfolgenden Abschnitten beschriebenen chemischen Beispiele angegeben sind, (400 MHz bei $^1$H-NMR und 150 MHz bei $^{13}$C-NMR, Lösungsmittel $CDCl_3$, $CD_3OD$ oder $d_6$-DMSO, interner Standard: Tetramethylsilan $\delta$ = 0.00 ppm), wurden mit einem Gerät der Firma Bruker erhalten, und die bezeichneten Signale haben die nachfolgend aufgeführten Bedeutungen: br = breit(es); s = Singulett, d = Dublett, t = Triplett, dd = Doppeldublett, ddd = Dublett eines Doppeldubletts, m = Multiplett, q = Quartett, quint = Quintett, sext = Sextett, sept = Septett, t = Triplett, dq = Doppelquartett, dt = Doppeltriplett.

Spektroskopische Daten ausgewählter Tabellenbeispiele:

Tabelle 2:

Beispiel Nr. 2-9:

$^1$H-NMR (400 MHz, $CDCl_3$ $\delta$, ppm) 5.98 (s, 1H), 4.20 (q, 2H), 2.30 (m, 2H), 1.60 (m, 3H), 1.48 (m, 2H), 1.36 (m, 3H), 1.29 (t, 3H), 1.18 (m, 3H), 1.11 (d, 6H).

Beispiel Nr. 2-12:

Diastereomer Nr. 1:

$^1$H-NMR (400 MHz, $CDCl_3$ $\delta$, ppm) 5.98 (s, 1H), 4.20 (q, 2H), 2.24 (m, 2H), 1.63 (m, 4H), 1.46 (m, 3H), 1.32 (m, 3H), 1.29 (t, 3H), 1.12 (d, 3H), 1.10 (d, 3H), 0.92 (t, 3H).

Diastereomer Nr. 2:

$^1$H-NMR (400 MHz, $CDCl_3$ $\delta$, ppm) 5.98 (s, 1H), 4.20 (q, 2H), 2.25 (m, 2H), 1.63 (m, 5H), 1.48 (m, 2H), 1.34 (m, 4H), 1.29 (t, 3H), 1.10 (m, 6H), 0.93 (t, 3H).

Beispiel Nr. 2-27:

Diastereomer Nr. 1:

$^1$H-NMR (400 MHz, $CDCl_3$ $\delta$, ppm) 6.60 (s, 1H), 4.28 (q, 2H), 1.73 (m, 2H), 1.63 (m, 2H), 1.48 (m, 2H), 1.31 (t, 3H), 1.28 (m, 2H), 1.13/1.10 (d, 3H), 1.11/1.09 (d, 3H).

Diastereomer Nr. 2:

$^1$H-NMR (400 MHz, $CDCl_3$ ,$\delta$ ppm) 6.60 (s, 1H), 4.28 (q, 2H), 1.65 (m, 4H), 1.48 (m, 2H), 1.31 (t, 3H), 1.28 (m, 2H), 1.10 (t, 6H).

Tabelle 3:

Beispiel Nr. 3-1:

$^1$H-NMR (400 MHz, $CDCl_3$ $\delta$, ppm) 6.29 (d, 1H); 6.12 (d, 1H); 1.93 (m, 1H); 1.68-1.20 (m, 5H); 1.11 (s, 3H); 1.04 (d, 3H); 1.02 (s, 3H).

Beispiel Nr. 3-2:

$^1$H-NMR (400 MHz, $CDCl_3$ δ, ppm) 6.21 (d, 1H); 6.09 (d, 1H); 3.76 (s, 3H); 2.13 (s, 1H); 1.93 (m, 1H); 1.69-1.33 (m, 5H); 1.15 (s, 3H); 1.11 (d, 3H); 1.04 (s, 3H).

Beispiel Nr. 3-3:

$^1$H-NMR (400 MHz, $CDCl_3$ δ, ppm) 6.18 (d, 1H); 6.07 (d, 1H); 4.22 (q, 2H); 1.92 (m, 1H); 1.70-1.30 (m,6H); 1.30(t, 3H); 1.14 (s, 3H); 1.06 (d, 3H); 1.03 (s, 3H).

Beispiel Nr. 3-4:

$^1$H-NMR (400 MHz, $CDCl_3$ δ, ppm) 6.01 (s, 1H); 2.09 (s, 3H); 1.92 (m, 1H); 1.69-1.31 (m, 6H); 1,12 (s, 3H); 1.06 (d, 3H); 1.02 (s, 3H).

Beispiel Nr. 3-5:

$^1$H-NMR (400 MHz, $CDCl_3$ δ, ppm) 5.97 (s, 1H); 3.72 (s, 3H); 2.06 (s, 3H); 1.93 (m, 1H); 1.69-1.33 (m, 6H); 1.15 (s, 3H); 1.07 (d, 3H); 1.03 (s, 3H).

Beispiel Nr. 3-6:

$^1$H-NMR (400 MHz, $CDCl_3$ δ, ppm) 5.98 (s, 1H), 4.19 (q, 2H), 2.30 (s, 1H), 2.04 (s, 3H), 1.91 (m, 1H), 1.65 (m, 2H), 1.51-1.35 (m, 4H), 1.28 (t, 3H), 1.12 (s, 3H), 1.07 (d, 3H), 1.03 (s, 3H).

Beispiel Nr. 3-7:

$^1$H-NMR (400 MHz, $CDCl_3$ δ, ppm) 6.02 (s, 1H), 2.35 (q, 2H), 1.93 (m, 1H), 1.72 (m, 1H), 1.62-1.53 (m, 3H), 1.49-1.40 (m, 3H), 1.20 (t, 3H), 1.14 (s, 3H), 1.08 (d, 3H), 1.03 (s, 3H).

Beispiel Nr. 3-9:

$^1$H-NMR (400 MHz, $CDCl_3$ δ, ppm) 5.96 (s, 1H), 4.19 (q, 2H), 2.31 (q, 2H), 1.93 (m, 2H), 1.61 (m, 2H), 1.48 (m, 2H), 1.35 (m, 2H), 1.28 (t, 3H), 1.18 (t, 6H), 1.08 (d, 3H), 1.04 (s, 3H).

Beispiel Nr. 3-10:

$^1$H-NMR (400 MHz, $CDCl_3$ δ, ppm) 6.00 (s, 1H), 2.30 (q, 2H), 1.92 (m, 1H), 1.63 (m, 2H), 1.48-1.38 (m, 3H), 1.37-1.30 (m, 3H), 1.12 (s, 3H), 1.08 (d, 3H), 1.03 (s, 3H), 0.94 (t, 3H).

Beispiel Nr. 3-12:

$^1$H-NMR (400 MHz, $CDCl_3$ δ, ppm) 5.96 (s, 1H), 4.19 (q, 2H), 2.27 (t, 2H), 1.93 (m, 1H), 1.62 (m, 4H), 1.48 (m, 2H), 1.35 (m, 2H), 1.28 (t, 3H), 1.14 (s, 3H), 1.11 (m, 1H), 1.08 (d, 3H), 1.03 (s, 3H), 0.92 (t, 3H).

Beispiel Nr. 3-15:

$^1$H-NMR (400 MHz, $CDCl_3$ δ, ppm) 6.00 (s, 1H), 4.19 (q, 2H), 2.54 (m, 1H), 1.94 (m, 2H), 1.46 (m, 2H), 1.35 (m, 2H), 1.30 (t, 3H), 1.19 (m, 9H), 1.12 (m, 1H), 1.09 (d, 3H), 1.05 (s, 3H).

Beispiel Nr. 3-18:

$^1$H-NMR (400 MHz, $CDCl_3$ δ, ppm) 6.12 (s, 1H), 4.19 (q, 2H), 2.62 (m, 2H), 1.47 (m, 2H), 1.35 (m, 2H), 1.28 (t, 3H), 1.11 (s, 3H), 1.08 (m, 1H), 1.06 (d, 3H), 1.03 (s, 3H), 0.92 (m, 2H), 0.83 (m, 2H).

Beispiel Nr. 3-21:

$^1$H-NMR (400 MHz, $CDCl_3$ δ, ppm) 5.96 (s, 1H), 4.19 (q, 2H), 2.29 (t, 2H), 1.93 (m, 1H), 1.60 (m, 3H), 1.45 (m,

2H), 1.35 (m, 4H), 1.29 (t, 3H), 1.14 (s, 3H), 1.12 (dd, 1H), 1.08 (d, 3H), 1.05 (s, 3H), 0.90 (t, 3H).

Beispiel Nr. 3-24:

$^1$H-NMR (400 MHz, $CDCl_3$ $\delta$, ppm) 5.98 (s, 1H), 3.72 (s, 3H), 2.25 (m, 1H), 1.60 (m, 2H), 1.46 (m, 2H), 1.35 (m, 2H), 1.14 (m, 9H), 1.10 (dd, 1H), 1.04 (m, 3H), 1.00 (s, 2H), 0.87 (t, 3H).

Beispiel Nr. 3-25:

$^1$H-NMR (400 MHz, $CDCl_3$ $\delta$, ppm) 6.62 (s, 1H), 1.94 (m, 1H), 1.60 (m, 2H), 1.47 (m, 2H), 1.37 (m, 2H), 1.13 (s, 3H), 1.08 (d, 3H), 1.05 (s, 3H).

Beispiel Nr. 3-27:

$^1$H-NMR (400 MHz, $CDCl_3$ $\delta$, ppm) 6.60 (s, 1H), 4.28 (q, 2H), 1.93 (m, 1H), 1.60 (m, 2H), 1.45 (m, 2H), 1.34 (m, 2H), 1.32 (t, 3H), 1.15 (s, 3H), 1.10 (m, 1H), 1.08 (d, 3H), 1.04 (s, 3H).

Tabelle 4:

Beispiel Nr. 4-9:

$^1$H-NMR (400 MHz, $CDCl_3$ $\delta$, ppm) 5.98 (s, 1H), 4.19 (q, 2H), 2.31 (q, 2H), 1.55 (m, 3H), 1.35 (m, 3H), 1.28 (t, 3H), 1.20 (s, 6H), 1.16 (m, 3H), 1.12 (s, 6H).

Beispiel Nr. 4-12:

$^1$H-NMR (400 MHz, $CDCl_3$ $\delta$, ppm) 5.98 (s, 1H), 4.20 (q, 2H), 2.27 (t, 2H), 1.62 (m, 2H), 1.57 (m, 4H), 1.33 (m, 2H), 1.28 (t, 3H), 1.20 (s, 6H), 1.12 (s, 6H), 0.94 (t, 3H).

Beispiel Nr. 4-27:

$^1$H-NMR (400 MHz, $CDCl_3$ $\delta$, ppm) 6.19 (s, 1H), 4.28 (q, 2H), 1.55 (m, 4H), 1.36 (m, 2H), 1.32 (t, 3H), 1.20 (d, 6H), 1.11 (d, 6H).

Gegenstand der vorliegenden Erfindung sind demnach die Verfahren zur Herstellung von (2Z)-5(1-Hydroxycyclohexyl)pent-2-en-4-insäure-Derivaten und deren Analoge der allgemeinen Formel (I), und deren Verwendung der sowie von beliebigen Mischungen dieser erfindungsgemäßen mit agrochemischen Wirkstoffen entsprechend der unten stehenden Definition, zur Steigerung der Widerstandsfähigkeit von Pflanzen gegenüber abiotischen Stressfaktoren, bevorzugt Trockenstress, sowie zur Stärkung des Pflanzenwachstums und/oder zur Erhöhung des Pflanzenertrags.

Weiterer Gegenstand der vorliegenden Erfindung ist eine Sprühlösung zur Behandlung von Pflanzen, enthaltend eine zur Steigerung der Widerstandsfähigkeit von Pflanzen gegenüber abiotischen Stressfaktoren wirksame Menge von mindestens einer Verbindung, ausgewählt aus der Gruppe, bestehend aus (2Z)-5(1-Hydroxycyclohexyl)pent-2-en-4-insäure-Derivate der allgemeinen Formel (I). Zu den dabei relativierbaren abiotischen Streßbedingungen können zum Beispiel Dürre, Kälte-und Hitzebedingungen, osmotischer Streß, Staunässe, erhöhter Bodensalzgehalt, erhöhtes Ausgesetztsein an Mineralien, Ozonbedingungen, Starklichtbedingungen, beschränkte Verfügbarkeit von Stickstoffnährstoffen, beschränkte Verfügbarkeit von Phosphornährstoffen zählen.

In einer Ausführungsform kann beispielsweise vorgesehen sein, dass die erfindungsgemäß vorgesehenen Verbindungen, d. h. die entsprechenden substituierten (2Z)-5(1-Hydroxycyclohexyl)pent-2-en-4-insäure-Derivate, durch eine Sprühapplikation auf entsprechende zu behandelnde Pflanzen oder Pflanzenteile aufgebracht werden. Die erfindungsgemäß vorgesehene Verwendung der erfindungsgemäßen Verbindungen (I) erfolgt vorzugsweise mit einer Dosierung zwischen 0,00005 und 3 kg/ha, besonders bevorzugt zwischen 0,0001 und 2 kg/ha, insbesondere bevorzugt zwischen 0,0005 und 1 kg/ha. Wenn im Rahmen der vorliegenden Erfindung Abscisinsäure gleichzeitig mit substituierten (2Z)-5(1-Hydroxycyclohexyl)pent-2-en-4-insäure-Derivaten, beispielsweise in Rahmen einer gemeinsamen Zubereitung oder Formulierung verwendet wird, so erfolgt die Zumischung von Abscisinsäure dabei vorzugsweise in einer Dosierung zwischen 0,001 und 3 kg/ha, besonders bevorzugt zwischen 0,005 und 2 kg/ha, insbesondere bevorzugt zwischen 0,01 und 1 kg/ha.

Unter der Bezeichnung Resistenz bzw. Widerstandsfähigkeit gegenüber abiotischem Stress werden im Rahmen der vorliegenden Erfindung verschiedenartige Vorteile für Pflanzen verstanden. Solche vorteilhaften Eigenschaften äußern sich beispielsweise in den nachfolgend genannten verbesserten Pflanzencharakteristika: verbessertes Wurzelwachstum hinsichtlich Oberfläche und Tiefe, vermehrte Ausläuferbildung oder Bestockung, stärkere und produktivere Ausläufer und Bestockungstriebe, Verbesserung des Sproßwachstums, erhöhte Standfestigkeit, vergrößerte Sprossbasisdurchmesser, vergrößerte Blattfläche, höhere Erträge an Nähr- und Inhaltsstoffen, wie z.B. Kohlenhydrate, Fette, Öle, Proteine, Vitamine, Mineralstoffe, ätherische Öle, Farbstoffe, Fasern, bessere Faserqualität, früheres Blühen, gesteigerte Blütenanzahl, reduzierter Gehalt an toxischen Produkten wie Mycotoxine, reduzierter Gehalt an Rückständen oder unvorteilhaften Bestandteilen jeglicher Art oder bessere Verdaulichkeit, verbesserte Lagerstabilität des Erntegutes, verbesserter Toleranz gegenüber unvorteilhaften Temperaturen, verbesserter Toleranz gegenüber Dürre und Trockenheit, wie auch Sauerstoffmangel durch Wasserüberschuß, verbesserte Toleranz gegenüber erhöhten Salzgehalten in Böden und Wasser, gesteigerte Toleranz gegenüber Ozonstress, verbesserte Verträglichkeit gegenüber Herbiziden und anderen Pflanzenbehandlungsmitteln, verbesserte Wasseraufnahme und Photosyntheseleistung, vorteilhafte Pflanzeneigenschaften, wie beispielsweise Beschleunigung der Reifung, gleichmäßigere Abreife, größere Anziehungskraft für Nützlinge, verbesserte Bestäubung oder andere Vorteile, die einem Fachmann durchaus bekannt sind.

Insbesondere zeigt die erfindungsgemäße Verwendung in der Sprühapplikation auf Pflanzen und Pflanzenteilen die beschriebenen Vorteile. Kombinationen von den entsprechenden substituierten (2Z)-5(1-Hydroxycyclohexyl)pent-2-en-4-insäure-Derivaten der allgemeinen Formel (I) unter anderem mit Insektiziden, Lockstoffen, Akariziden, Fungiziden, Nematiziden, Herbiziden, wachstumsregulierenden Stoffen, Safenern, die Pflanzenreife beeinflussenden Stoffen und Bakteriziden können bei der Bekämpfung von Pflanzenkrankheiten im Rahmen der vorliegenden Erfindung ebenfalls Anwendung finden. Die kombinierte Verwendung von entsprechenden substituierten (2Z)-5(1-Hydroxycyclohexyl)pent-2-en-4-insäure-Derivaten der allgemeinen Formel (I) mit gentechnisch veränderten Sorten in Bezug auf erhöhte abiotische Stresstoleranz ist darüber hinaus ebenfalls möglich.

Die weiter oben genannten verschiedenartigen Vorteile für Pflanzen lassen sich bekannterweise partiell zusammenfassen und mit allgemein gültigen Begriffen belegen. Soche Begriffe sind beispielsweise die nachfolgend aufgeführten Bezeichnungen: phytotonischer Effekt, Widerstandsfähigkeit gegenüber Stressfaktoren, weniger Pflanzenstress, Pflanzengesundheit, gesunde Pflanzen, Pflanzenfitness, ("Plant Fitness"), "Plant Wellness", "Plant Concept", "Vigor Effect", "Stress Shield", Schutzschild, "Crop Health", "Crop Health Properties", "Crop Health Products", "Crop Health Management", "Crop Health Therapy", "Plant Health", Plant Health Properties", Plant Health Products", "Plant Health Management", "Plant Health Therapy", Grünungseffekt ("Greening Effect" oder "Re-greening Effect"), "Freshness" oder andere Begriffe, die einem Fachmann durchaus bekannt sind.

Im Rahmen der vorliegenden Erfindung wird unter einem guten Effekt auf die Widerstandsfähigkeit gegenüber abiotischem Stress nicht beschränkend

- mindestens ein um im Allgemeinen 3 %, insbesondere größer als 5 % besonders bevorzugt größer als 10 % verbessertes Auflaufen,
- mindestens einen im Allgemeinen 3 %, insbesondere größer als 5 % besonders bevorzugt größer als 10 % gesteigerten Ertrag,
- mindestens eine um im Allgemeinen 3 %, insbesondere größer als 5 % besonders bevorzugt größer als 10 % verbesserte Wurzelentwicklung,
- mindestens eine um im Allgemeinen 3 %, insbesondere größer als 5 % besonders bevorzugt größer als 10 % ansteigende Sproßgröße,
- mindestens eine um im Allgemeinen 3 %, insbesondere größer als 5 % besonders bevorzugt größer als 10 % vergrößerte Blattfläche,
- mindestens eine um im Allgemeinen 3 %, insbesondere größer als 5 % besonders bevorzugt größer als 10 % verbesserte Photosyntheseleistung und/oder
- mindestens eine um im Allgemeinen 3 %, insbesondere größer als 5 % besonders bevorzugt größer als 10 % verbesserte Blütenausbildung

verstanden, wobei die Effekte einzeln oder aber in beliebiger Kombination von zwei oder mehreren Effekten auftreten können.

Weiterer Gegenstand der vorliegenden Erfindung ist eine Sprühlösung zur Behandlung von Pflanzen, enthaltend eine zur Steigerung der Widerstandsfähigkeit von Pflanzen gegenüber abiotischen Stressfaktoren wirksame Menge von mindestens einer Verbindung der Formel (I). Die Sprühlösung kann andere übliche Bestandteile aufweisen, wie Lösungsmittel, Formulierhilfsstoffe, insbesondere Wasser, enthalten. Weitere Bestandteile können unter anderem agrochemische Wirkstoffe sein, welche unten noch weiter beschrieben werden.

Weiterer Gegenstand der vorliegenden Erfindung ist die Verwendung von entsprechenden Sprühlösungen zur Steigerung der Widerstandsfähigkeit von Pflanzen gegenüber abiotischen Stressfaktoren. Die nachfolgenden Ausführungen gelten sowohl für die erfindungsgemäße Verwendung der Verbindungen der Formel I) an sich als auch für die entsprechenden Sprühlösungen.

Erindunsgemäß wurde darüber hinaus gefunden, dass die Anwendung der Verbindungen der allgemeinen

Formel (I) in Kombination mit mindestens einem Düngemittel wie weiter unten stehend definiert auf Pflanzen oder in deren Umgebung möglich ist.

Düngemittel, die erfindungsgemäß zusammen mit den oben näher erläuterten Verbindungen der allgemeinen Formel (I) verwendet werden können, sind im Allgemeinen organische und anorganische Stickstoff-haltige Verbindungen wie beispielsweise Harnstoffe, Harnstoff-Formaldehyd-Kondensationsprodukte, Aminosäuren, Ammoniumsalze und -nitrate, Kaliumsalze (bevorzugt Chloride, Sulfate, Nitrate), Phosphorsäuresalze und/oder Salze von Phosphoriger Säure (bevorzugt Kaliumsalze und Ammoniumsalze). Insbesondere zu nennen sind in diesem Zusammenhang die NPK-Dünger, d.h. Düngemittel, die Stickstoff, Phosphor und Kalium enthalten, Kalkammonsalpeter, d.h. Düngemittel, die noch Calcium enthalten, Ammonsulfatsalpeter (Allgemeine Formel $(NH_4)_2SO_4$ $NH_4NO_3$), Ammonphosphat und Ammonsulfat. Diese Düngemittel sind dem Fachmann allgemein bekannt, siehe auch beispielsweise Ullmann's Encyclopedia of Industrial Chemistry, 5. Edition, Vol. A 10, Seiten 323 bis 431, Verlagsgesellschaft, Weinheim, 1987.

Die Düngemittel können auch Salze aus Mikronährstoffen (bevorzugt Calcium, Schwefel, Bor, Mangan, Magnesium, Eisen, Bor, Kupfer, Zink, Molybdän und Kobalt) und Phytohormonen (z. B. Vitamin B1 und Indol-(III)essigsäure) oder Gemische davon enthalten. Erfindungsgemäß eingesetzte Düngemittel können auch weitere Salze wie Monoammoniumphosphat (MAP), Diammoniumphosphat (DAP), Kaliumsulfat, Kaliumchlorid, Magnesiumsulfat enthalten. Geeignete Mengen für die sekundären Nährstoffe oder Spurenelemente sind Mengen von 0,5 bis 5 Gew.-%, bezogen auf das gesamte Düngemittel. Weitere mögliche Inhaltsstoffe sind Pflanzenschutzmittel, Insektizide oder Fungizide, Wachstumsregulatoren oder Gemische davon. Hierzu folgen weiter unten weitergehende Ausführungen.

Die Düngemittel können beispielsweise in Form von Pulvern, Granulaten, Prills oder Kompaktaten eingesetzt werden. Die Düngemittel können jedoch auch in flüssiger Form, gelöst in einem wässrigen Medium, eingesetzt werden. In diesem Fall kann auch verdünnter wässriger Ammoniak als Stickstoffdüngemittel eingesetzt werden. Weitere mögliche Inhaltsstoffe für Düngemittel sind beispielsweise in Ullmann's Encyclopedia of Industrial Chemistry, 5. Auflage, 1987, Band A 10, Seiten 363 bis 401, DE-A 41 28 828, DE-A 19 05 834 und DE-A 196 31 764 beschrieben. Die allgemeine Zusammensetzung der Düngemittel, bei welchen es sich im Rahmen der vorliegenden Erfindung um Einzelnährstoff- und/oder Mehrnährstoffdünger handeln kann, beispielsweise aus Stickstoff, Kalium oder Phosphor, kann innerhalb eines breiten Bereichs variieren. Im Allgemeinen ist ein Gehalt von 1 bis 30 Gew.-% Stickstoff (bevorzugt 5 bis 20 Gew.-%), von 1 bis 20 Gew.-% Kalium (bevorzugt 3 bis 15 Gew.-%) und ein Gehalt von 1 bis 20 Gew.-% Phosphor (bevorzugt 3 bis 10 Gew.-%) vorteilhaft. Der Gehalt von Mikroelementen ist üblicherweise im ppm-Bereich, bevorzugt im Bereich von von 1 bis 1000 ppm.

Im Rahmen der vorliegenden Erfindung kann das Düngemittel sowie die Verbindungen der allgemeinen Formel (I) zeitgleich verabreicht werden. Es ist jedoch auch möglich, zunächst das Düngemittel und dann eine Verbindung der allgemeinen Formel (I) oder zunächst eine Verbindung der allgemeinen Formel (I) und dann das Düngemittel anzuwenden. Bei nicht zeitgleicher Anwendung einer Verbindung der allgemeinen Formel (I) und des Düngemittels erfolgt im Rahmen der vorliegenden Erfindung jedoch die Anwendung in funktionellem Zusammenhang, insbesondere innerhalb eines Zeitraums von im Allgemeinen 24 Stunden, bevorzugt 18 Stunden, besonders bevorzugt 12 Stunden, speziell 6 Stunden, noch spezieller 4 Stunden, noch weiter spezieller innerhalb 2 Stunden. In ganz besonderen Ausführungsformen der vorliegenden Erfindung erfolgt die Anwendung der erfindungsgemäßen Verbindung der Formel (I) und des Düngemittels in einem zeitlichen Rahmen von weniger als 1 Stunden, vorzugsweise weniger als 30 Minuten, besonders bevorzugt weniger als 15 Minuten.

Die erfindungsgemäß zu verwendenden Wirkstoffe der allgmeinen Formel (I) können, gegebenenfalls in Kombination mit Düngemitteln, bevorzugt an folgenden Pflanzen angewendet werden, wobei die folgende Aufzählung nicht beschränkend ist.

Bevorzugt sind Pflanzen aus der Gruppe der Nutzpflanzen, Zierpflanzen, Rasenarten, allgemein genutzte Bäume, die in öffentlichen und privaten Bereichen als Zierpflanzen Verwendungen finden, und Forstbestand. Der Forstbestand umfasst Bäume für die Herstellung von Holz, Zellstoff, Papier und Produkten die aus Teilen der Bäume hergestellt werden. Der Begriff Nutzpflanzen, wie hier verwendet, bezeichnet Kulturpflanzen, die als Pflanzen für die Gewinnung von Nahrungsmitteln, Futtermitteln, Treibstoffe oder für technische Zwecke eingesetzt werden.

Zu den Nutzpflanzen zählen z. B. folgende Pflanzenarten: Triticale, Durum (Hartweizen), Turf, Reben, Getreide, beispielsweise Weizen, Gerste, Roggen, Hafer, Reis, Mais und Hirse; Rüben, beispielsweise Zuckerrüben und Futterrüben; Früchte, beispielsweise Kernobst, Steinobst und Beerenobst, beispielsweise Äpfel, Birnen, Pflaumen, Pfirsiche, Mandeln, Kirschen und Beeren, z. B. Erdbeeren, Himbeeren, Brombeeren; Hülsenfrüchte, beispielsweise Bohnen, Linsen, Erbsen und Sojabohnen; Ölkulturen, beispielsweise Raps, Senf, Mohn, Oliven, Sonnenblumen, Kokos, Castorölpflanzen, Kakaobohnen und Erdnüsse; Gurkengewächse, beispielsweise Kürbis, Gurken und Melonen; Fasergewächse, beispielsweise Baumwolle, Flachs, Hanf und Jute; Citrusfrüchte, beispielsweise Orangen, Zitronen, Pampelmusen und Mandarinen; Gemüsesorten, beispielsweise Spinat, (Kopf)-Salat, Spargel, Kohlarten, Möhren, Zwiebeln, Tomaten, Kartoffeln und Paprika; Lorbeergewächse, beispielsweise Avocado, Cinnamomum, Kampfer, oder ebenso Pflanzen wie Tabak, Nüsse, Kaffee, Aubergine, Zuckerrohr, Tee, Pfeffer, Weinreben, Hopfen, Bananen, Naturkautschukgewächse sowie Zierpflanzen, beispielsweise Blumen, Sträucher, Laubbäume und Nadelbäume wie Koniferen. Diese Aufzählung

stellt keine Limitierung dar.

Als besonders geeignete Zielkulturen für die Anwendung des erfindungsgemäßen Verfahrens sind folgende Pflanzen anzusehen: Hafer, Roggen, Triticale, Durum, Baumwolle, Aubergine, Turf, Kernobst, Steinobst, Beerenobst, Mais, Weizen, Gerste, Gurke, Tabak, Reben, Reis, Getreide, Birne, Pfeffer, Bohnen, Sojabohnen, Raps, Tomate, Paprika, Melonen, Kohl, Kartoffel und Apfel.

Als Bäume, die entsprechend dem erfindungsgemäßen Verfahren verbessert werden können, seien beispielhaft genannt: Abies sp., Eucalyptus sp., Picea sp., Pinus sp., Aesculus sp., Platanus sp., Tilia sp., Acer sp., Tsuga sp., Fraxinus sp., Sorbus sp., Betula sp., Crataegus sp., Ulmus sp., Quercus sp., Fagus sp., Salix sp., Populus sp..

Als bevorzugte Bäume, die entsprechend dem erfindungsgemäßen Verfahren verbessert werden können, können genannt werden: Aus der Baumart Aesculus: A. hippocastanum, A. pariflora, A. carnea; aus der Baumart Platanus: P. aceriflora, P. occidentalis, P. racemosa; aus der Baumart Picea: P. abies; aus der Baumart Pinus: P. radiate, P. ponderosa, P. contorta, P. sylvestre, P. elliottii, P. montecola, P. albicaulis, P. resinosa, P. palustris, P. taeda, P. flexilis, P. jeffregi, P. baksiana, P. strobes; aus der Baumart Eucalyptus: E. grandis, E. globulus, E. camadentis, E. nitens, E. obliqua, E. regnans, E. pilularus.

Als besonders bevorzugte Bäume, die entsprechend dem erfindungsgemäßen Verfahren verbessert werden können, können genannt werden: Aus der Baumart Pinus: P. radiate, P. ponderosa, P. contorta, P. sylvestre, P. strobes; aus der Baumart Eucalyptus: E. grandis, E. globulus und E. camadentis.

Als besonders bevorzugte Bäume, die entsprechend dem erfindungsgemäßen Verfahren verbessert werden können, können genannt werden: Rosskastanie, Platanengewächs, Linde und Ahornbaum.

Die vorliegende Erfindung kann auch an beliebigen Rasenarten ("turfgrasses") durchgeführt werden, einschließlich "cool season turfgrasses" und "warm season turfgrasses". Beispiele für Rasenarten für die kalte Jahreszeit sind Blaugräser ("blue grasses"; Poa spp.), wie "Kentucky bluegrass" (Poa pratensis L.), "rough bluegrass" (Poa trivialis L.), "Canada bluegrass" (Poa compressa L.), "annual bluegrass" (Poa annua L.), "upland bluegrass" (Poa glaucantha Gaudin), "wood bluegrass" (Poa nemoralis L.) und "bulbous bluegrass" (Poa bulbosa L.); Straussgräser ("Bentgrass", Agrostis spp.), wie "creeping bentgrass" (Agrostis palustris Huds.), "colonial bentgrass" (Agrostis tenuis Sibth.), "velvet bentgrass" (Agrostis canina L.), "South German Mixed Bentgrass" (Agrostis spp. einschließlich Agrostis tenius Sibth., Agrostis canina L., und Agrostis palustris Huds.), und "redtop" (Agrostis alba L.);

Schwingel ("Fescues", Festucu spp.), wie "red fescue" (Festuca rubra L. spp. rubra), "creeping fescue" (Festuca rubra L.), "chewings fescue" (Festuca rubra commutata Gaud.), "sheep fescue" (Festuca ovina L.), "hard fescue" (Festuca longifolia Thuill.), "hair fescue" (Festucu capillata Lam.), "tall fescue" (Festuca arundinacea Schreb.) und "meadow fescue" (Festuca elanor L.);

Lolch ("ryegrasses", Lolium spp.), wie "annual ryegrass" (Lolium multiflorum Lam.), "perennial ryegrass" (Lolium perenne L.) und "italian ryegrass" (Lolium multiflorum Lam.);

und Weizengräser ("wheatgrasses", Agropyron spp..), wie "fairway wheatgrass" (Agropyron cristatum (L.) Gaertn.), "crested wheatgrass" (Agropyron desertorum (Fisch.) Schult.) und "western wheatgrass" (Agropyron smithii Rydb.).

Beispiele für weitere "cool season turfgrasses" sind "beachgrass" (Ammophila breviligulata Fern.), "smooth bromegrass" (Bromus inermis Leyss.), Schilf ("cattails") wie "Timothy" (Phleum pratense L.), "sand cattail" (Phleum subulatum L.), "orchardgrass" (Dactylis glomerata L.), "weeping alkaligrass" (Puccinellia distans (L.) Parl.) und "crested dog's-tail" (Cynosurus cristatus L.).

Beispiele für "warm season turfgrasses" sind "Bermudagrass" (Cynodon spp. L. C. Rich), "zoysiagrass" (Zoysia spp. Willd.), "St. Augustine grass" (Stenotaphrum secundatum Walt Kuntze), "centipedegrass" (Eremochloa ophiuroides Munro Hack.), "carpetgrass" (Axonopus affinis Chase), "Bahia grass" (Paspalum notatum Flugge), "Kikuyugrass" (Pennisetum clandestinum Hochst. ex Chiov.), "buffalo grass" (Buchloe dactyloids (Nutt.) Engelm.), "Blue gramma" (Bouteloua gracilis (H.B.K.) Lag. ex Griffiths), "seashore paspalum" (Paspalum vaginatum Swartz) und "sideoats grama" (Bouteloua curtipendula (Michx. Torr.). "Cool season turfgrasses" sind für die erfindungsgemäße Verwendung im Allgemeinen bevorzugt. Besonders bevorzugt sind Blaugras, Straussgras und "redtop", Schwingel und Lolch. Straussgras ist insbesondere bevorzugt.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder mit Hilfe rekombinanter DNA-Techniken, gezüchtet worden sind. Kulturpflanzen können demnach Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten.

Das erfindungsgemäße Behandlungsverfahren kann somit auch für die Behandlung von genetisch modifizierten Organismen (GMOs), z. B. Pflanzen oder Samen, verwendet werden. Genetisch modifizierte Pflanzen (oder transgene Pflanzen) sind Pflanzen, bei denen ein heterologes Gen stabil in das Genom integriert worden ist. Der Begriff "heterologes

Gen" bedeutet im wesentlichen ein Gen, das außerhalb der Pflanze bereitgestellt oder assembliert wird und das bei Einführung in das Zellkerngenom, das Chloroplastengenom oder das Hypochondriengenom der transformierten Pflanze dadurch neue oder verbesserte agronomische oder sonstige Eigenschaften verleiht, dass es ein interessierendes Protein oder Polypeptid exprimiert oder dasses ein anderes Gen, das in der Pflanze vorliegt bzw. andere Gene, die in der Pflanze vorliegen, herunterreguliert oder abschaltet (zum Beispiel mittels Antisense-Technologie, Co-suppressionstechnologie oder RNAi-Technologie [RNA Interference]). Ein heterologes Gen, das im Genom vorliegt, wird ebenfalls als Transgen bezeichnet. Ein Transgen, das durch sein spezifisches Vorliegen im Pflanzengenom definiert ist, wird als Transformations- bzw. transgenes Event bezeichnet.

Zu Pflanzen und Pflanzensorten, die vorzugsweise erfindungsgemäß behandelt werden, zählen alle Pflanzen, die über Erbgut verfügen, das diesen Pflanzen besonders vorteilhafte, nützliche Merkmale verleiht (egal, ob dies durch Züchtung und/oder Biotechnologie erzielt wurde).

Pflanzen und Pflanzensorten, die ebenfalls erfindungsgemäß behandelt werden können, sind solche Pflanzen, die gegen einen oder mehrere abiotische Streßfaktoren resistent sind. Zu den abiotischen Streßbedingungen können zum Beispiel Dürre, Kälte- und Hitzebedingungen, osmotischer Streß, Staunässe, erhöhter Bodensalzgehalt, erhöhtes Ausgesetztsein an Mineralien, Ozonbedingungen, Starklichtbedingungen, beschränkte Verfügbarkeit von Stickstoffnährstoffen, beschränkte Verfügbarkeit von Phosphornährstoffen oder Vermeidung von Schatten zählen.

Pflanzen und Pflanzensorten, die ebenfalls erfindungsgemäß behandelt werden können, sind solche Pflanzen, die durch erhöhte Ertragseigenschaften gekennzeichnet sind. Ein erhöhter Ertrag kann bei diesen Pflanzen z. B. auf verbesserter Pflanzenphysiologie, verbessertem Pflanzenwuchs und verbesserter Pflanzenentwicklung, wie Wasserverwertungseffizienz, Wasserhalteeffizienz, verbesserter Stickstoffverwertung, erhöhter Kohlenstoffassimilation, verbesserter Photosynthese, verstärkter Keimkraft und beschleunigter Abreife beruhen. Der Ertrag kann weiterhin durch eine verbesserte Pflanzenarchitektur (unter Streß- und nicht-Streß-Bedingungen) beeinflußt werden, darunter frühe Blüte, Kontrolle der Blüte für die Produktion von Hybridsaatgut, Keimpflanzenwüchsigkeit, Pflanzengröße, Internodienzahl und -abstand, Wurzelwachstum, Samengröße, Fruchtgröße, Schotengröße, Schoten- oder Ährenzahl, Anzahl der Samen pro Schote oder Ähre, Samenmasse, verstärkte Samenfüllung, verringerter Samenausfall, verringertes Schotenplatzen sowie Standfestigkeit. Zu weiteren Ertragsmerkmalen zählen Samenzusammensetzung wie Kohlenhydratgehalt, Proteingehalt, Ölgehalt und Ölzusammensetzung, Nährwert, Verringerung der nährwidrigen Verbindungen, verbesserte Verarbeitbarkeit und verbesserte Lagerfähigkeit.

Pflanzen, die erfindungsgemäß ebenfalls behandelt werden können, sind Hybridpflanzen, die bereits die Eigenschaften der Heterosis bzw. des Hybrideffekts exprimieren, was im allgemeinen zu höherem Ertrag, höherer Wüchsigkeit, besserer Gesundheit und besserer Resistenz gegen biotische und abiotische Streßfaktoren führt. Solche Pflanzen werden typischerweise dadurch erzeugt, dass man eine ingezüchtete pollensterile Elternlinie (den weiblichen Kreuzungspartner) mit einer anderen ingezüchteten pollenfertilen Elternlinie (dem männlichen Kreuzungspartner) kreuzt. Das Hybridsaatgut wird typischerweise von den pollensterilen Pflanzen geerntet und an Vermehrer verkauft. Pollensterile Pflanzen können manchmal (z. B. beim Mais) durch Entfahnen (d. h. mechanischem Entfernen der männlichen Geschlechtsorgane bzw. der männlichen Blüten), produziert werden; es ist jedoch üblicher, dass die Pollensterilität auf genetischen Determinanten im Pflanzengenom beruht. In diesem Fall, insbesondere dann, wenn es sich bei dem gewünschten Produkt, da man von den Hybridpflanzen ernten will, um die Samen handelt, ist es üblicherweise günstig, sicherzustellen, dass die Pollenfertilität in Hybridpflanzen, die die für die Pollensterilität verantwortlichen genetischen Determinanten enthalten, völlig restoriert wird. Dies kann erreicht werden, indem sichergestellt wird, dass die männlichen Kreuzungspartner entsprechende Fertilitätsrestorergene besitzen, die in der Lage sind, die Pollenfertilität in Hybridpflanzen, die die genetischen Determinanten, die für die Pollensterilität verantwortlich sind, enthalten, zu restorieren. Genetische Determinanten für Pollensterilität können im Cytoplasma lokalisiert sein. Beispiele für cytoplasmatische Pollensterilität (CMS) wurden zum Beispiel für Brassica-Arten beschrieben (WO 92/005251, WO 95/009910, WO 98/27806, WO 05/002324, WO 06/021972 und US 6,229,072). Genetische Determinanten für Pollensterilität können jedoch auch im Zellkerngenom lokalisiert sein. Pollensterile Pflanzen können auch mit Methoden der pflanzlichen Biotechnologie, wie Gentechnik, erhalten werden. Ein besonders günstiges Mittel zur Erzeugung von pollensterilen Pflanzen ist in WO 89/10396 beschrieben, wobei zum Beispiel eine Ribonuklease wie eine Barnase selektiv in den Tapetumzellen in den Staubblättern exprimiert wird. Die Fertilität kann dann durch Expression eines Ribonukleasehemmers wie Barstar in den Tapetumzellen restoriert werden (z. B. WO 91/002069).

Pflanzen oder Pflanzensorten (die mit Methoden der Pflanzenbiotechnologie, wie der Gentechnik, erhalten werden), die erfindungsgemäß ebenfalls behandelt werden können, sind herbizidtolerante Pflanzen, d. h. Pflanzen, die gegenüber einem oder mehreren vorgegebenen Herbiziden tolerant gemacht worden sind. Solche Pflanzen können entweder durch genetische Transformation oder durch Selektion von Pflanzen, die eine Mutation enthalten, die solch eine Herbizidtoleranz verleiht, erhalten werden.

Herbizidtolerante Pflanzen sind zum Beispiel glyphosatetolerante Pflanzen, d. h. Pflanzen, die gegenüber dem Herbizid Glyphosate oder dessen Salzen tolerant gemacht worden sind. So können zum Beispiel glyphosatetolerante Pflanzen durch Transformation der Pflanze mit einem Gen, das für das Enzym 5-Enolpyruvylshikimat-3-phosphatsynthase (EPSPS) kodiert, erhalten werden. Beispiele für solche EPSPS-Gene sind das AroA-Gen (Mutante CT7) des Bakterium Salmonella typhimurium (Comai et al., Science (1983), 221, 370-371), das CP4-Gen des Bakteriums Agrobacterium sp. (Barry et al., Curr. Topics Plant Physiol. (1992), 7, 139-145), die Gene, die für eine EPSPS aus der Petunie (Shah et al., Science (1986), 233, 478-481), für eine EPSPS aus der Tomate (Gasser et al., J. Biol. Chem. (1988), 263, 4280-4289) oder für eine EPSPS aus Eleusine (WO 01/66704) kodieren. Es kann sich auch um eine mutierte EPSPS handeln, wie sie zum Beispiel in EP-A 0837944, WO 00/066746, WO 00/066747 oder WO 02/026995 beschrieben ist. Glyphosatetolerante Pflanzen können auch dadurch erhalten werden, dass man ein Gen exprimiert, das für ein Glyphosate-Oxidoreduktase-Enzym, wie es in US 5,776,760 und US 5,463,175 beschrieben ist, kodiert. Glyphosatetolerante Pflanzen können auch dadurch erhalten werden, dass man ein Gen exprimiert, das für ein Glyphosate-acetyltransferase-Enzym, wie es in z. B. WO 02/036782, WO 03/092360, WO 05/012515 und WO 07/024782 beschrieben ist, kodiert. Glyphosatetolerante Pflanzen können auch dadurch erhalten werden, dass man Pflanzen, die natürlich vorkommende Mutationen der oben erwähnten Gene, wie sie zum Beispiel in WO 01/024615 oder WO 03/013226 beschrieben sind, enthalten, selektiert.

Sonstige herbizidresistente Pflanzen sind zum Beispiel Pflanzen, die gegenüber Herbiziden, die das Enzym Glutaminsynthase hemmen, wie Bialaphos, Phosphinotricin oder Glufosinate, tolerant gemacht worden sind. Solche Pflanzen können dadurch erhalten werden, dass man ein Enzym exprimiert, das das Herbizid oder eine Mutante des Enzyms Glutaminsynthase, das gegenüber Hemmung resistent ist, entgiftet. Solch ein wirksames entgiftendes Enzym ist zum Beispiel ein Enzym, das für ein Phosphinotricin-acetyltransferase kodiert (wie zum Beispiel das bar- oder pat-Protein aus Streptomyces-Arten). Pflanzen, die eine exogene Phosphinotricin-acetyltransferase exprimieren, sind zum Beispiel in US 5,561,236; US 5,648,477; US 5,646,024; US 5,273,894; US 5,637,489; US 5,276,268; US 5,739,082; US 5,908,810 und US 7,112,665 beschrieben.

Weitere herbizidtolerante Pflanzen sind auch Pflanzen, die gegenüber den Herbiziden, die das Enzym Hydroxyphenylpyruvatdioxygenase (HPPD) hemmen, tolerant gemacht worden sind. Bei den Hydroxyphenylpyruvatdioxygenasen handelt es sich um Enzyme, die die Reaktion, in der para-Hydroxyphenylpyruvat (HPP) zu Homogentisat umgesetzt wird, katalysieren. Pflanzen, die gegenüber HPPD-Hemmern tolerant sind, können mit einem Gen, das für ein natürlich vorkommendes resistentes HPPD-Enzym kodiert, oder einem Gen, das für ein mutiertes HPPD-Enzym gemäß WO 96/038567, WO 99/024585 und WO 99/024586 kodiert, transformiert werden. Eine Toleranz gegenüber HPPD-Hemmern kann auch dadurch erzielt werden, dass man Pflanzen mit Genen transformiert, die für gewisse Enzyme kodieren, die die Bildung von Homogentisat trotz Hemmung des nativen HPPD-Enzyms durch den HPPD-Hemmer ermöglichen. Solche Pflanzen und Gene sind in WO 99/034008 und WO 02/36787 beschrieben. Die Toleranz von Pflanzen gegenüber HPPD-Hemmern kann auch dadurch verbessert werden, dass man Pflanzen zusätzlich zu einem Gen, das für ein HPPD-tolerantes Enzym kodiert, mit einem Gen transformiert, das für ein Prephenatdehydrogenase-Enzym kodiert, wie dies in WO 04/024928 beschrieben ist.

Weitere herbizidresistente Pflanzen sind Pflanzen, die gegenüber Acetolactatsynthase (ALS)-Hemmern tolerant gemacht worden sind. Zu bekannten ALS-Hemmern zählen zum Beispiel Sulfonylharnstoff, Imidazolinon, Triazolopyrimidine, Pyrimidinyloxy(thio)benzoate und/oder Sulfonylaminocarbonyltriazolinon-Herbizide. Es ist bekannt, dass verschiedene Mutationen im Enzym ALS (auch als Acetohydroxysäure-Synthase, AHAS, bekannt) eine Toleranz gegenüber unterschiedlichen Herbiziden bzw. Gruppen von Herbiziden verleihen, wie dies zum Beispiel bei Tranel und Wright, Weed Science (2002), 50, 700-712, jedoch auch in US 5,605,011, US 5,378,824, US 5,141,870 und US 5,013,659, beschrieben ist. Die Herstellung von sulfonylharnstofftoleranten Pflanzen und imidazolinontoleranten Pflanzen ist in US 5,605,011; US 5,013,659; US 5,141,870; US 5,767,361; US 5,731,180; US 5,304,732; US 4,761,373; US 5,331,107; US 5,928,937; und US 5,378,824; sowie in der internationalen Veröffentlichung WO 96/033270 beschrieben. Weitere imidazolinontolerante Pflanzen sind auch in z. B. WO 04/040012, WO 04/106529, WO 05/020673, WO 05/093093, WO 06/007373, WO 06/015376, WO 06/024351 und WO 06/060634 beschrieben. Weitere sulfonylharnstoff- und imidazolinontolerante Pflanzen sind auch in z.B. WO 07/024782 beschrieben.

Weitere Pflanzen, die gegenüber ALS-Inhibitoren, insbesondere gegenüber Imidazolinonen, Sulfonylharnstoffen und/oder Sulfamoylcarbonyltriazolinonen tolerant sind, können durch induzierte Mutagenese, Selektion in Zellkulturen in Gegenwart des Herbizids oder durch Mutationszüchtung erhalten werden, wie dies zum Beispiel für die Sojabohne in US 5,084,082, für Reis in WO 97/41218, für die Zuckerrübe in US 5,773,702 und WO 99/057965, für Salat in US 5,198,599 oder für die Sonnenblume in WO 01/065922 beschrieben ist.

Pflanzen oder Pflanzensorten (die nach Methoden der pflanzlichen Biotechnologie, wie der Gentechnik, erhalten wurden), die ebenfalls erfindungsgemäß behandelt werden können, sind insektenresistente transgene Pflanzen, d.h. Pflanzen, die gegen Befall mit gewissen Zielinsekten resistent gemacht wurden. Solche Pflanzen können durch genetische Transformation oder durch Selektion von Pflanzen, die eine Mutation enthalten, die solch eine Insektenresistenz verleiht, erhalten werden.

Der Begriff "insektenresistente transgene Pflanze" umfaßt im vorliegenden Zusammenhang jegliche Pflanze, die mindestens ein Transgen enthält, das eine Kodiersequenz umfaßt, die für folgendes kodiert:

1) ein insektizides Kristallprotein aus Bacillus thuringiensis oder einen insektiziden Teil davon, wie die insektiziden Kristallproteine, die von Crickmore et al., Microbiology and Molecular Biology Reviews (1998), 62, 807-813, zusammengestellt wurden, von Crickmore et al. (2005) in der Bacillus thuringiensis-Toxinnomenklatur aktualisiert (online bei: http://www.lifesci.sussex.ac.uk/Home/Neil-Crickmore/Bt/), oder insektizide Teile davon, z.B. Proteine der Cry-Proteinklassen Cry1Ab, Cry1Ac, Cry1 F, Cry2Ab, Cry3Ae oder Cry3Bb oder insektizide Teile davon; oder

2) ein Kristallprotein aus Bacillus thuringiensis oder einen Teil davon, der in Gegenwart eines zweiten, anderen Kristallproteins als Bacillus thuringiensis oder eines Teils davon insektizid wirkt, wie das binäre Toxin, das aus den Kristallproteinen Cy34 und Cy35 besteht (Moellenbeck et al., Nat. Biotechnol. (2001), 19, 668-72; Schnepf et al., Applied Environm. Microb. (2006), 71, 1765-1774); oder

3) ein insektizides Hybridprotein, das Teile von zwei unterschiedlichen insektiziden Kristallproteinen aus Bacillus thuringiensis umfaßt, wie zum Beispiel ein Hybrid aus den Proteinen von 1) oben oder ein Hybrid aus den Proteinen von 2) oben, z. B. das Protein Cry1A.105, das von dem Mais-Event MON98034 produziert wird (WO 07/027777); oder

4) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden, wie das Protein Cry3Bb1 in Mais-Events MON863 oder MON88017 oder das Protein Cry3A im Mais-Event MIR 604; oder

5) ein insektizides sezerniertes Protein aus Bacillus thuringiensis oder Bacillus cereus oder einen insektiziden Teil davon, wie die vegetativ wirkenden insektentoxischen Proteine (vegetative insecticidal proteins, VIP), die unter folgendem Link angeführt sind, z. B. Proteine der Proteinklasse VIP3Aa: http://www.lifesci.sussex.ac.uk/Home/Neil_Crickmore/Bt/vip.html oder

6) ein sezerniertes Protein aus Bacillus thuringiensis oder Bacillus cereus, das in Gegenwart eines zweiten sezernierten Proteins aus Bacillus thuringiensis oder B. cereus insektizid wirkt, wie das binäre Toxin, das aus den Proteinen VIP1A und VIP2A besteht (WO 94/21795); oder

7) ein insektizides Hybridprotein, das Teile von verschiedenen sezernierten Proteinen von Bacillus thuringiensis oder Bacillus cereus umfaßt, wie ein Hybrid der Proteine von 1) oder ein Hybrid der Proteine von 2) oben; oder

8) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden (wobei die Kodierung für ein insektizides Protein erhalten bleibt), wie das Protein VIP3Aa im Baumwoll-Event COT 102.

Natürlich zählt zu den insektenresistenten transgenen Pflanzen im vorliegenden Zusammenhang auch jegliche Pflanze, die eine Kombination von Genen umfaßt, die für die Proteine von einer der oben genannten Klassen 1 bis 8 kodieren. In einer Ausführungsform enthält eine insektenresistente Pflanze mehr als ein Transgen, das für ein Protein nach einer der oben genannten 1 bis 8 kodiert, um das Spektrum der entsprechenden Zielinsektenarten zu erweitern oder um die Entwicklung einer Resistenz der Insekten gegen die Pflanzen dadurch hinauszuzögern, dass man verschiedene Proteine einsetzt, die für dieselbe Zielinsektenart insektizid sind, jedoch eine unterschiedliche Wirkungsweise, wie Bindung an unterschiedliche Rezeptorbindungsstellen im Insekt, aufweisen.

Pflanzen oder Pflanzensorten (die nach Methoden der pflanzlichen Biotechnologie, wie der Gentechnik, erhalten wurden), die ebenfalls erfindungsgemäß behandelt werden können, sind gegenüber abiotischen Streßfaktoren tolerant. Solche Pflanzen können durch genetische Transformation oder durch Selektion von Pflanzen, die eine Mutation enthalten, die solch eine Streßresistenz verleiht, erhalten werden. Zu besonders nützlichen Pflanzen mit Streßtoleranz zählen folgende:

a. Pflanzen, die ein Transgen enthalten, das die Expression und/oder Aktivität des Gens für die Poly(ADP-ribose)polymerase (PARP) in den Pflanzenzellen oder Pflanzen zu reduzieren vermag, wie dies in WO 00/004173 oder EP 04077984.5 oder EP 06009836.5 beschrieben ist.

b. Pflanzen, die ein streßtoleranzförderndes Transgen enthalten, das die Expression und/oder Aktivität der für PARG kodierenden Gene der Pflanzen oder Pflanzenzellen zu reduzieren vermag, wie dies z.B. in WO 04/090140 beschrieben ist;

c. Pflanzen, die ein streßtoleranzförderndes Transgen enthalten, das für ein in Pflanzen funktionelles Enzym des Nicotinamidadenindinukleotid-Salvage-Biosynthesewegs kodiert, darunter Nicotinamidase, Nicotinatphosphoribosyltransferase, Nicotinsäuremononukleotid-adenyltransferase, Nicotinamidadenindinukleotidsynthetase oder Nicotinamidphosphoribosyl-transferase, wie dies z. B. in EP 04077624.7 oder WO 06/133827 oder PCT/EP07/002433 beschrieben ist.

Pflanzen oder Pflanzensorten (die nach Methoden der pflanzlichen Biotechnologie, wie der Gentechnik, erhalten wurden), die ebenfalls erfindungsgemäß behandelt werden können, weisen eine veränderte Menge, Qualität und/oder Lagerfähigkeit des Ernteprodukts und/oder veränderte Eigenschaften von bestimmten Bestandteilen des Ernteprodukts auf, wie zum Beispiel:

1) Transgene Pflanzen, die eine modifizierte Stärke synthetisieren, die bezüglich ihrer chemisch-physikalischen Eigenschaften, insbesondere des Amylosegehalts oder des Amylose/Amylopektin-Verhältnisses, des Verzweigungsgrads, der durchschnittlichen Kettenlänge, der Verteilung der Seitenketten, des Viskositätsverhaltens, der Gelfestigkeit, der Stärkekorngröße und/oder Stärkekornmorphologie im Vergleich mit der synthetisierten Stärke in Wildtyppflanzenzellen oder -pflanzen verändert ist, so dass sich diese modifizierte Stärke besser für bestimmte Anwendungen eignet. Diese transgenen Pflanzen, die eine modifizierte Stärke synthetisieren, sind zum Beispiel in EP 0571427, WO 95/004826, EP 0719338, WO 96/15248, WO 96/19581, WO 96/27674, WO 97/11188, WO 97/26362, WO 97/32985, WO 97/42328, WO 97/44472, WO 97/45545, WO 98/27212, WO 98/40503, WO 99/58688, WO 99/58690, WO 99/58654, WO 00/008184, WO 00/008185, WO 00/28052, WO 00/77229, WO 01/12782, WO 01/12826, WO 02/101059, WO 03/071860, WO 04/056999, WO 05/030942, WO 05/030941, WO 05/095632, WO 05/095617, WO 05/095619, WO 05/095618, WO 05/123927, WO 06/018319, WO 06/103107, WO 06/108702, WO 07/009823, WO 00/22140, WO 06/063862, WO 06/072603, WO 02/034923, EP 06090134.5, EP 06090228.5, EP 06090227.7, EP 07090007.1, EP 07090009.7, WO 01/14569, WO 02/79410, WO 03/33540, WO 04/078983, WO 01/19975, WO 95/26407, WO 96/34968, WO 98/20145, WO 99/12950, WO 99/66050, WO 99/53072, US 6,734,341, WO 00/11192, WO 98/22604, WO 98/32326, WO 01/98509, WO 01/98509, WO 05/002359, US 5,824,790, US 6,013,861, WO 94/004693, WO 94/009144, WO 94/11520, WO 95/35026 bzw. WO 97/20936 beschrieben.

2) Transgene Pflanzen, die Nichtstärkekohlenhydratpolymere synthetisieren, oder Nichtstärkekohlenhydratpolymere, deren Eigenschaften im Vergleich zu Wildtyppflanzen ohne genetische Modifikation verändert sind. Beispiele sind Pflanzen, die Polyfructose, insbesondere des Inulin- und Levantyps, produzieren, wie dies in EP 0663956, WO 96/001904, Wo 96/021023, WO 98/039460 und WO 99/024593 beschrieben ist, Pflanzen, die alpha-1,4-Glucane produzieren, wie dies in WO 95/031553, US 2002/031826, US 6,284,479, US 5,712,107, WO 97/047806, WO 97/047807, WO 97/047808 und WO 00/14249 beschrieben ist, Pflanzen, die alpha-1,6-verzweigte alpha-1,4-Glucane produzieren, wie dies in WO 2000/73422 beschrieben ist, und Pflanzen, die Alternan produzieren, wie dies in WO 00/047727, EP 06077301.7, US 5,908,975 und EP 0728213 beschrieben ist.

3) Transgene Pflanzen, die Hyaluronan produzieren, wie dies zum Beispiel in WO 06/032538, WO 07/039314, WO 07/039315, WO 07/039316, JP 2006/304779 und WO 05/012529 beschrieben ist.

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Baumwollpflanzen mit veränderten Fasereigenschaften. Solche Pflanzen können durch genetische Transformation oder durch Selektion von Pflanzen, die eine Mutation enthalten, die solche veränderten Fasereigenschaften verleiht, erhalten werden; dazu zählen:

a) Pflanzen wie Baumwollpflanzen, die eine veränderte Form von Cellulosesynthasegenen enthalten, wie dies in WO 98/000549 beschrieben ist,

b) Pflanzen wie Baumwollpflanzen, die eine veränderte Form von rsw2- oder rsw3-homologen Nukleinsäuren enthalten, wie dies in WO 04/053219 beschrieben ist;

c) Pflanzen wie Baumwollpflanzen mit einer erhöhten Expression der Saccharosephosphatsynthase, wie dies in WO 01/017333 beschrieben ist;

d) Pflanzen wie Baumwollpflanzen mit einer erhöhten Expression der Saccharosesynthase, wie dies in WO 02/45485 beschrieben ist;

e) Pflanzen wie Baumwollpflanzen bei denen der Zeitpunkt der Durchlaßsteuerung der Plasmodesmen an der Basis der Faserzelle verändert ist, z. B. durch Herunterregulieren der faserselektiven β-1,3-Glucanase, wie dies in WO 05/017157 beschrieben ist;

f) Pflanzen wie Baumwollpflanzen mit Fasern mit veränderter Reaktivität, z. B. durch Expression des N-Acetylglucosamintransferasegens, darunter auch nodC, und von Chitinsynthasegenen, wie dies in WO 06/136351 beschrieben ist.

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Raps oder verwandte Brassica-Pflanzen mit veränderten Eigenschaften der Ölzusammensetzung. Solche Pflanzen können durch genetische Transformation oder durch Selektion von Pflanzen, die eine Mutation enthalten, die solche veränderten Öleigenschaften verleiht, erhalten werden; dazu zählen:

a) Pflanzen wie Rapspflanzen, die Öl mit einem hohen Ölsäuregehalt produzieren, wie dies zum Beispiel in US 5,969,169, US 5,840,946 oder US 6,323,392 oder US 6,063, 947 beschrieben ist;

b) Pflanzen wie Rapspflanzen, die Öl mit einem niedrigen Linolensäuregehalt produzieren, wie dies in US 6,270828, US 6,169,190 oder US 5,965,755 beschrieben ist.

c) Pflanzen wie Rapspflanzen, die Öl mit einem niedrigen gesättigten Fettsäuregehalt produzieren, wie dies z. B. in US 5,434,283 beschrieben ist.

Besonders nützliche transgene Pflanzen, die erfindungsgemäß behandelt werden können, sind Pflanzen mit einem oder mehreren Genen, die für ein oder mehrere Toxine kodieren, sind die transgenen Pflanzen, die unter den folgenden Handelsbezeichnungen angeboten werden: YIELD GARD® (zum Beispiel Mais, Baumwolle, Sojabohnen), KnockOut® (zum Beispiel Mais), BiteGard® (zum Beispiel Mais), BT-Xtra® (zum Beispiel Mais), StarLink® (zum Beispiel Mais), Bollgard® (Baumwolle), Nucotn® (Baumwolle), Nucotn 33B® (Baumwolle), NatureGard® (zum Beispiel Mais), Protecta® und NewLeaf® (Kartoffel). Herbizidtolerante Pflanzen, die zu erwähnen sind, sind zum Beispiel Maissorten, Baumwollsorten und Sojabohnensorten, die unter den folgenden Handelsbezeichnungen angeboten werden: Roundup Ready® (Glyphosatetoleranz, zum Beispiel Mais, Baumwolle, Sojabohne), Liberty Link® (Phosphinotricintoleranz, zum Beispiel Raps), IMI® (Imidazolinontoleranz) und SCS® (Sylfonylharnstofftoleranz), zum Beispiel Mais. Zu den herbizidresistenten Pflanzen (traditionell auf Herbizidtoleranz gezüchtete Pflanzen), die zu erwähnen sind, zählen die unter der Bezeichnung Clearfield® angebotenen Sorten (zum Beispiel Mais).

Besonders nützliche transgene Pflanzen, die erfindungsgemäß behandelt werden können, sind Pflanzen, die Transformations-Events, oder eine Kombination von Transformations-Events, enthalten und die zum Beispiel in den Dateien von verschiedenen nationalen oder regionalen Behörden angeführt sind.

Die erfindungsgemäß zu verwendenden Verbindungen der Formel (I) können in üblichen Formulierungen überführt werden, wie Lösungen, Emulsionen, Spritzpulver, wasser- und ölbasierte Suspensionen, Pulver, Stäubemittel, Pasten, lösliche Pulver, lösliche Granulate, Streugranulate, Suspensions-Emulsions-Konzentrate, Wirkstoff-imprägnierte Naturstoffe, Wirkstoff-imprägnierte synthetische Stoffe, Düngemittel sowie Feinstverkapselungen in polymeren Stoffen. Im Rahmen der vorliegenden Erfindung ist es insbesondere bevorzugt, wenn die Verbindungen der allgemeinen Formel (I) in der Form einer Sprühformulieruing verwendet werden.

Die vorliegende Erfindung betrifft daher darüber hinaus auch eine Sprühformulierung zur Steigerung der Widerstandsfähigkeit von Pflanzen gegenüber abiotischem Stress. Im Folgenden wird eine Sprühformulierung näher beschrieben:

Die Formulierungen zur Sprühapplikation werden in bekannter Weise hergestellt, z.B. durch Vermischen der erfindungsgemäß zu verwendenden Verbindungen der allgmeinen Formel (I) mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Weitere übliche Zusatzstoffe, wie zum Beispiel übliche Streckmittel sowie Lösungs-oder Verdünnungsmittel, Farbstoffe, Netzmittel, Dispergiermittel, Emulgatoren, Entschäumer, Konservierungsmittel, sekundäre Verdickungsmittel, Kleber, Gibberelline und auch Wasser, können gegebenenfalls auch verwendet werden. Die Herstellung der Formulierungen erfolgt entweder in geeigneten Anlagen oder auch vor oder während der Anwendung.

Als Hilfsstoffe können solche Stoffe Verwendung finden, die geeignet sind, dem Mittel selbst oder und/oder davon abgeleitete Zubereitungen (z.B. Spritzbrühen) besondere Eigenschaften zu verleihen, wie bestimmte technische

Eigenschaften und/oder auch besondere biologische Eigenschaften. Als typische Hilfsmittel kommen in Frage: Streckmittel, Lösemittel und Trägerstoffe.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (Poly-)Ether, der einfachen und substituierten Amine, Amide, Lactame (wie N-Alkylpyrrolidone) und Lactone, der Sulfone und Sulfoxide (wie Dimethylsysulfoxid).

Im Falle der Benutzung von Wasser als Streckmittel können z.B. auch organische Lösemittel als Hilfslösungsmittel verwendet werden. Als flüssige Lösemittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylsulfoxid, sowie Wasser.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Als Netzmittel, die in den erfindungsgemäß verwendbaren Formulierungen enthalten sein können, kommen alle zur Formulierung von agrochemischen Wirkstoffen üblichen, die Benetzung fördernden Stoffe in Frage. Vorzugsweise verwendbar sind Alkylnaphthalin-Sulfonate, wie Diisopropyl- oder Diisobutylnaphthalin-Sulfonate.

Als Dispergiermittel und/oder Emulgatoren, die in den erfindungsgemäß verwendbaren Formulierungen enthalten sein können, kommen alle zur Formulierung von agrochemischen Wirkstoffen üblichen nichtionischen, anionischen und kationischen Dispergiermittel in Betracht. Vorzugsweise verwendbar sind nichtionische oder anionische Dispergiermittel oder Gemische von nichtionischen oder anionischen Dispergiermitteln. Als geeignete nichtionische Dispergiermittel sind insbesondere Ethylenoxid-Propylenoxid-Blockpolymere, Alkylphenolpolyglykolether sowie Tristryrylphenolpolyglykolether und deren phosphatierte oder sulfatierte Derivate zu nennen. Geeignete anionische Dispergiermittel sind insbesondere Ligninsulfonate, Polyacrylsäuresalze und Arylsulfonat-Formaldehydkondensate.

Als Entschäumer können in den erfindungsgemäß verwendbaren Formulierungen alle zur Formulierung von agrochemischen Wirkstoffen üblichen schaumhemmenden Stoffe enthalten sein. Vorzugsweise verwendbar sind Silikonentschäumer und Magnesiumstearat.

Als Konservierungsmittel können in den erfindungsgemäß verwendbaren Formulierungen alle für derartige Zwecke in agrochemischen Mitteln einsetzbaren Stoffe vorhanden sein. Beispielhaft genannt seien Dichlorophen und Benzylalkoholhemiformal.

Als sekundäre Verdickungsmittel, die in den erfindungsgemäß verwendbaren Formulierungen enthalten sein können, kommen alle für derartige Zwecke in agrochemischen Mitteln einsetzbaren Stoffe in Frage. Vorzugsweise in Betracht kommen Cellulosederivate, Acrylsäurederivate, Xanthan, modifizierte Tone und hochdisperse Kieselsäure.

Als Kleber, die in den erfindungsgemäß verwendbaren Formulierungen enthalten sein können, kommen alle üblichen in Beizmitteln einsetzbaren Bindemittel in Frage. Vorzugsweise genannt seien Polyvinylpyrrolidon, Polyvinylacetat, Polyvinylalkohol und Tylose. Als Gibberelline, die in den erfindungsgemäß verwendbaren Formulierungen enthalten sein können, kommen vorzugsweise die Gibberelline A1, A3 (= Gibberellinsäure), A4 und A7 infrage, besonders bevorzugt verwendet man die Gibberellinsäure. Die Gibberelline sind bekannt (vgl. R. Wegler "Chemie der Pflanzenschutz- und Schädlingsbekämpfungsmittel", Bd. 2, Springer Verlag, 1970, S. 401-412).

Weitere Additive können Duftstoffe, mineralische oder vegetabilische gegebenenfalls modifizierte Öle, Wachse und Nährstoffe (auch Spurennährstoffe), wie Salze von Eisen, Mangan, Bor, Kupfer, Kobalt, Molybdän und Zink sein. Weiterhin enthalten sein können Stabilisatoren wie Kältestabilisatoren, Oxidationsschutzmittel, Lichtschutzmittel oder andere die chemische und / oder physikalische Stabilität verbessernde Mittel. Die Formulierungen enthalten im allgemeinen zwischen 0,01 und 98 Gew.-%, vorzugsweise zwischen 0,5 und 90 %, der Verbindung der allgemeinen Formel (I).

Der erfindungsgemäße Wirkstoff kann in seinen handelsüblichen Formulierungen sowie in den aus diesen Formulierungen bereiteten Anwendungsformen in Mischung mit anderen Wirkstoffen wie Insektiziden, Lockstoffen, Sterilantien, Bakteriziden, Akariziden, Nematiziden, Fungiziden, wachstumsregulierenden Stoffen, Herbiziden, Safenern, Düngemitteln oder Semiochemicals vorliegen.

Ferner lässt sich die beschriebene positive Wirkung der Verbindungen der Formel (I) auf die pflanzeneigenen Abwehrkräfte durch eine zusätzliche Behandlung mit insektziden, fungiziden oder bakteriziden Wirkstoffen unterstützen.

Bevorzugte Zeitpunkte für die Applikation von Verbindungen der allgemeinen Formel (I) zur Seigerung der Resistanz gegenüber abiotischem Stress sind Boden-, Stamm-und/oder Blattbehandlungen mit den zugelassenen Aufwandmengen.

Die Wirkstoffe der allgemeinen Formel (I) können im Allgemeinen darüber hinaus in ihren handelsüblichen Formulierungen sowie in den aus diesen Formulierungen bereiteten Anwendungsformen in Mischungen mit anderen Wirkstoffen, wie Insektiziden, Lockstoffen, Sterilantien, Akariziden, Nematiziden, Fungiziden, Bakteriziden, wachstumsregulierenden Stoffen, die Pflanzenreife beeinflussenden Stoffen, Safenern oder Herbiziden vorliegen und erfindungsgemäß verwendet werden. Besonders günstige Mischpartner sind beispielsweise die nachfolgend gruppenweise genannten Wirkstoffe der verschiedenen Klassen, ohne dass durch deren Reihenfolge eine Präferenz gesetzt wird:

Fungizide:

F1) Inhibitoren der Nucleinsäure Synthese, z. B. Benalaxyl, Benalaxyl-M, Bupirimat, Chiralaxyl, Clozylacon, Dimethirimol, Ethirimol, Furalaxyl, Hymexazol, Metalaxyl, Metalaxyl-M, Ofurace, Oxadixyl, Oxolinsäure;

F2) Inhibitoren der Mitose und Zellteilung, z. B. Benomyl, Carbendazim, Diethofencarb, Fuberidazole, Fluopicolid, Pencycuron, Thiabendazol, Thiophanat-methyl, Zoxamid und Chlor-7-(4-methylpiperidin-1-yl)-6-(2,4,6-Trifluorphenyl [1,2,4]triazolo[1,5-a]pyrimidin;

F3) Inhibitoren der Atmungskette Komplex I/II, z. B. Diflumetorim, Bixafen, Boscalid, Carboxin, Diflumethorim Fenfuram, Fluopyram, Flutolanil, Furametpyr, Mepronil, Oxycarboxin, Penflufen, Penthiopyrad, Thifluzamid, N-[2-(1,3-Dimethylbutyl)phenyl]-5-fluor-1,3-dimethyl-1H-pyrazol-4-carboxamid, Isopyrazam, Sedaxan, 3-(Difluormethyl)-1-methyl-N-(3',4',5'-trifluorbiphenyl-2-yl)-1H-pyrazol-4-carboxamid, 3-(Difluormethyl)-1-methyl-N-[2-(1,1,2,2-tetrafluorethoxy)phenyl]-1H-pyrazol-4-carboxamid, 3-(Difluormethyl)-N-[4-fluoro-2-(1,1,2,3,3,3-hexafluorpropoxy)phenyl]-1-methyl-1H-pyrazol-4-carboxamid, N-[1-(2,4-Dichlorphenyl)-1-methoxypropan-2-yl]-3-(difluormethyl)-1-methyl-1H-pyrazol-4-carboxamid und entsprechende Salze;

F4) Inhibitoren der Atmungskette Komplex III, z. B.Amisulbrom, Azoxystrobin, Cyazofamid, Dimoxystrobin, Enestrobin, Famoxadon, Fenamidon, Fluoxastrobin, Kresoximmethyl, Metominostrobin, Orysastrobin, Pyraclostrobin, Pyribencarb, Picoxystrobin, Trifloxystrobin, (2E)-2-(2-{[6-(3-Chlor-2-methylphenoxy)-5-fluorpyrimidin-4-yl]oxy}phenyl)-2-(methoxyimino)-N-methylethanamid, (2E)-2-(Ethoxyimino)-N-methyl-2-(2-{[({(1E)-1-[3-(trifluoromethyl)-phenyl]ethyliden}amino)oxy]methyl}phenyl)ethanamid und entsprechende Salze, (2E)-2-(Methoxyimino)-N-methyl-2-{2-[(E)-({1-[3-(trifluormethyl)phenyl]ethoxy}-imino)methyl]phenyl}ethanamid, (2E)-2-{2-[({[(1E)-1-(3-{[(E)-1-Fluor-2-phenylethenyl]-oxy}phenyl)ethyliden]amino}oxy)methyl]phenyl}-2-(methoxyimino)-N-methylethanamid, (2E)-2-{2-[({[(2E,3E)-4-(2,6-Dichlorophenyl)but-3-en-2-yliden]amino}oxy)methyl]phenyl}-2-(methoxyimino)-N-methylethanamid, 2-Chlor-N-(1,1,3-trimethyl-2,3-dihydro-1H-inden-4-yl)pyridin-3-carboxamid, 5-Methoxy-2-methyl-4-(2-{[({(1E)-1-[3-(trifluormethyl)phenyl]ethyliden}amino)oxy]methyl}phenyl)-2,4-dihydro-3H-1,2,4-triazol-3-on, 2-Methyl-{2-[({cyclopropyl[(4-methoxyphenyl)-imino]methyl}sulfanyl)methyl]phenyl}-3-methoxyacrylat, N-(3-Ethyl-3,5,5-trimethylcyclohexyl)-3-(formylamino)-2-hydroxybenzamid und entsprechende Salze;

F5) Entkoppler, z. B. Dinocap, Fluazinam;

F6) Inhibitoren der ATP Produktion, z. B. Fentinacetat, Fentinchlorid, Fentinhydroxid, Silthiofam

F7) Inhibitoren der Aminosäure- und Proteinbiosynthese, z.B. Andoprim, Blasticidin-S, Cyprodinil, Kasugamycin, Kasugamycinhydrochlorid Hydrat, Mepanipyrim, Pyrimethanil

F8) Inhibitoren der Signal-Transduktion, z. B. Fenpiclonil, Fludioxonil, Quinoxyfen

F9) Inhibitoren der Fett- und Membran Synthese, z. B. Chlozolinat, Iprodion, Procymidon, Vinclozolin, Ampropylfos, Kalium-Ampropylfos, Edifenphos, Iprobenfos (IBP), Isoprothiolan, Pyrazophos, Tolclofos-methyl, Biphenyl, Iodocarb, Propamocarb, Propamocarb hydrochlorid

F10) Inhibitoren der Ergosterol Biosynthese, z. B. Fenhexamid, Azaconazol, Bitertanol, Bromuconazol, Diclobutrazol, Difenoconazol, Diniconazol, Diniconazol-M, Etaconazol, Fenbuconazol, Fluquinconazol, Flusilazol, Flutriafol, Furconazol, Furconazol-cis, Hexaconazol, Imibenconazol, Ipconazol, Metconazol, Myclobutanil, Paclobutrazol, Penconazol, Propiconazol, Prothioconazol, Simeconazol, Spiroxamin, Tebuconazol, Triadimefon, Triadimenol, Triticonazol, Uniconazol, Voriconazol, Imazalil, Imazalilsulfat, Oxpoconazol, Fenarimol, Flurprimidol, Nuarimol, Pyrifenox, Triforin, Pefurazoat, Prochloraz, Triflumizol, Viniconazol, Aldimorph, Dodemorph, Dodemorphacetat, Fenpropimorph, Tridemorph, Fenpropidin, Naftifin, Pyributicarb, Terbinafin, 1-(4-Chlorophenyl)-2-(1H-1,2,4-triazol-1-yl)cycloheptanol, Methyl-1-(2,2-dimethyl-2,3-dihydro-1H-inden-1-yl)-1H-imidazol-5-carboxylat, N'-{5-(Difluormethyl)-2-methyl-4-[3-(trimethyl-silyl)propoxy]phenyl}-N-ethyl-N-methylimidoformamid, N-Ethyl-N-methyl-N'-{2-methyl-5-(trifluormethyl)-4-[3-(trimethylsilyl)propoxy]phenyl}imidoformamid und O-{1-[(4-Methoxy-phenoxy)methyl]-2,2-dimethylpropyl}-1H-imidazol-1-carbothioat;

F11) Inhibitoren der Zellwand Synthese, z. B. Benthiavalicarb, Bialaphos, Dimethomorph, Flumorph, Iprovalicarb, Polyoxins, Polyoxorim, Validamycin A

F12) Inhibitoren der Melanin Biosynthese, z. B. Capropamid, Diclocymet, Fenoxanil, Phtalid, Pyroquilon, Tricyclazol

F13) Resistenzinduktion, z. B. Acibenzolar-S-methyl, Probenazol, Tiadinil

F14) Multisite, z. B. Captafol, Captan, Chlorothalonil, Kupfersalze wie: Kupferhydroxid, Kupfernaphthenat, Kupferoxychlorid, Kupfersulfat, Kupferoxid, Oxin-Kupfer und Bordeaux Mischung, Dichlofluanid, Dithianon, Dodin, Dodin freie Base, Ferbam, Folpet, Fluorofolpet, Guazatin, Guazatinacetat, Iminoctadin, Iminoctadinalbesilat, Iminoctadintriacetat, Mankupfer, Mancozeb, Maneb, Metiram, Metiram Zink, Propineb, Schwefel und Schwefelpräparate enthaltend Calciumpolysulphid, Thiram, Tolylfluanid, Zineb, Ziram

F15) Unbekannter Mechanismus, z. B. Amibromdol, Benthiazol, Bethoxazin, Capsimycin, Carvon, Chinomethionat, Chloropicrin, Cufraneb, Cyflufenamid, Cymoxanil, Dazomet, Debacarb, Diclomezine, Dichlorophen, Dicloran, Difenzoquat, Difenzoquat Methylsulphat, Diphenylamin, Ethaboxam, Ferimzon, flumetover, Flusulfamid, Fluopicolid, Fluoroimid, Fosatyl-Al, Hexachlorobenzol, 8-Hydroxychinolinsulfat, Iprodione, Irumamycin, Isotianil, Methasulphocarb, Metrafenon, Methyl Isothiocyanat, Mildiomycin, Natamycin, Nickel dimethyldithiocarbamat, Nitrothalisopropyl, Octhilinon, Oxamocarb, Oxyfenthiin, Pentachlorophenol und Salze, 2-Phenylphenol und Salze, Piperalin, Propanosin -Natrium, Proquinazid, Pyrrolnitrin, Quintozen, Tecloftalam, Tecnazen, Triazoxid, Trichlamid, Zarilamid und 2,3,5,6-Tetrachlor-4-(methylsulfonyl)-pyridin, N-(4-Chlor-2-nitrophenyl)-N-ethyl-4-methyl-benzenesulfonamid, 2-Amino-4-methyl-N-phenyl-5-thiazolecarboxamid, 2-Chlor-N-(2,3-dihydro-1,1,3-trimethyl-1H-inden-4-yl)-3-pyridincarboxamid, 3-[5-(4-Chlorphenyl)-2,3-dimethylisoxazolidin-3-yl]pyridin, cis-1-(4-Chlorphenyl)-2-(1H-1,2,4-triazol-1-yl)-cycloheptanol, 2,4-Dihydro-5-methoxy-2-methyl-4-[[[[1-[3-(trifluoromethyl)-phenyl]-ethyliden]-amino]-oxy]-methyl]-phenyl]-3H-1,2,3-triazol-3-on (185336-79-2), Methyl 1-(2,3-dihydro-2,2-dimethyl-1H-inden-1-yl)-1H-imidazole-5-carboxylat, 3,4,5-Trichlor-2,6-pyridindicarbonitril, Methyl 2-[[[cyclopropyl[(4-methoxyphenyl) imino]methyl]thio]methyl]-.alpha.-(methoxymethylen)- benzacetat, 4-Chlor-alpha-propinyloxy-N-[2-[3-methoxy-4-(2-propinyloxy)phenyl]ethyl]-benzacetamide, (2S)-N-[2-[4-[[3-(4-chlorophenyl)-2-propinyl]oxy]-3-methoxyphenyl]ethyl]-3-methyl-2-[(methylsulfonyl)amino]-butanamid, 5-Chlor-7-(4-methylpiperidin-1-yl)-6-(2,4,6-trifluorophenyl)-[1,2,4]triazolo[1,5-a]pyrimidin, 5-Chlor-6-(2,4,6-trifluorophenyl)-N-[(1R)-1,2,2-trimethyl-propyl][1,2,4]triazolo[1,5-a]pyrimidin-7-amin, 5-Chlor-N-[(1R)-1 ,2-dimethylpropyl]-6-(2,4,6-trifluorophenyl) [1,2,4]triazolo[1,5-a]pyrimidin-7-amine, N-[1-(5-Brom-3-chloro-pyridin-2-yl)ethyl]-2,4-dichloronicotinamid, N-(5-Brom-3-chlorpyridin-2-yl)methyl-2,4-dichlornicotinamid, 2-Butoxy-6-iod-3-propyl-benzopyranon-4-on, N-{(Z)-[(cyclopropyl-methoxy) imino][6-(difluormethoxy)-2,3-difluorphenyl]methyl}-2-benzacetamid, N-(3-Ethyl-3,5,5-trimethyl-cyclohexyl)-3-formylamino-2-hydroxy-benzamid, 2-[[[[1-[3(1Fluor-2-phenylethyl)oxy] phenyl] ethyliden]amino]oxy]methyl]-alpha-(methoxyimino)-N-methyl-alphaE-benzacetamid, N-{2-[3-Chlor-5-(trifluormethyl)pyridin-2-yl]ethyl}-2-(trifluoromethyl)benzamid, N-(3',4'-dichlor-5-fluorbiphenyl-2-yl)-3-(difluormethyl)-1-methyl-1H-pyrazol-4-carboxamid, N-(6-Methoxy-3-pyridinyl)-cyclopropan carboxamid, 1-[(4-Methoxyphenoxy)methyl]-2,2-dimethylpropyl-1H-imidazol-1- carbonsäure, O-[1-[(4-Methoxyphenoxy)methyl]-2,2-dimethylpropyl]-1H-imidazol- 1- carbothioic acid, 2-(2-{[6-(3-Chlor-2-methylphenoxy)-5-fluorpyrimidin-4-yl]oxy}phenyl)-2-(methoxyimino)-N-methylacetam id

Bakterizide:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

Insektizide / Akarizide / Nematizide:

I1) Acetylcholinesterase (AChE) Inhibitoren, a) aus der Stoffgruppe der Carbamate, zum Beispiel Alanycarb, Aldicarb, Aldoxycarb, Allyxycarb, Aminocarb, Bendiocarb, Benfuracarb, Bufencarb, Butacarb, Butocarboxim, Butoxycarboxim, Carbaryl, Carbofuran, Carbosulfan, Cloethocarb, Dimetilan, Ethiofencarb, Fenobucarb, Fenothiocarb, Fenoxycarb, Formetanate, Furathiocarb, Isoprocarb, Metam-sodium, Methiocarb, Methomyl, Metolcarb, Oxamyl, Pirimicarb, Promecarb, Propoxur, Thiodicarb, Thiofanox, Trimethacarb, XMC, Xylylcarb, Triazamate, b) aus der Gruppe der Organophosphate, zum Beispiel Acephate, Azamethiphos, Azinphos (-methyl, -ethyl), Bromophos-ethyl, Bromfenvinfos (-methyl), Butathiofos, Cadusafos, Carbophenothion, Chlorethoxyfos, Chlorfenvinphos, Chlormephos, Chlorpyrifos (-methyl/-ethyl), Coumaphos, Cyanofenphos, Cyanophos, Chlorfenvinphos, Demeton-S-methyl,

Demeton-S-methylsulphon, Dialifos, Diazinon, Dichlofenthion, Dichlorvos/DDVP, Dicrotophos, Dimethoate, Dimethylvinphos, Dioxabenzofos, Disulfoton, EPN, Ethion, Ethoprophos, Etrimfos, Famphur, Fenamiphos, Fenitrothion, Fensulfothion, Fenthion, Flupyrazofos, Fonofos, Formothion, Fosmethilan, Fosthiazate, Heptenophos, Iodofenphos, Iprobenfos, Isazofos, Isofenphos, Isopropyl O-salicylate, Isoxathion, Malathion, Mecarbam, Methacrifos, Methamidophos, Methidathion, Mevinphos, Monocrotophos, Naled, Omethoate, Oxydemeton-methyl, Parathion (-methyl/-ethyl), Phenthoate, Phorate, Phosalone, Phosmet, Phosphamidon, Phosphocarb, Phoxim, Pirimiphos (-methyl/-ethyl), Profenofos, Propaphos, Propetamphos, Prothiofos, Prothoate, Pyraclofos, Pyridaphenthion, Pyridathion, Quinalphos, Sebufos, Sulfotep, Sulprofos, Tebupirimfos, Temephos, Terbufos, Tetrachlorvinphos, Thiometon, Triazophos, Triclorfon, Vamidothion

12) Natrium-Kanal-Modulatoren / Spannungsabhängige Natrium-Kanal-Blocker, a) aus der Gruppe der Pyrethroide, zum Beispiel Acrinathrin, Allethrin (d-cis-trans, d-trans), Beta-Cyfluthrin, Bifenthrin, Bioallethrin, Bioallethrin-S-cyclopentyl-isomer, Bioethanomethrin, Biopermethrin, Bioresmethrin, Chlovaporthrin, Cis-Cypermethrin, Cis-Resmethrin, Cis-Permethrin, Clocythrin, Cycloprothrin, Cyfluthrin, Cyhalothrin, Cypermethrin (alpha-, beta-, theta-, zeta-), Cyphenothrin, Deltamethrin, Eflusilanate, Empenthrin (1 R-isomer), Esfenvalerate, Etofenprox, Fenfluthrin, Fenpropathrin, Fenpyrithrin, Fenvalerate, Flubrocythrinate, Flucythrinate, Flufenprox, Flumethrin, Fluvalinate, Fubfenprox, Gamma-Cyhalothrin, Imiprothrin, Kadethrin, Lambda-Cyhalothrin, Metofluthrin, Permethrin (cis-, trans-), Phenothrin (1 R-trans isomer), Prallethrin, Profluthrin, Protrifenbute, Pyresmethrin, Pyrethrin, Resmethrin, RU 15525, Silafluofen, Tau-Fluvalinate, Tefluthrin, Terallethrin, Tetramethrin (-1 R- isomer), Tralomethrin, Transfluthrin, ZXI 8901, Pyrethrins (pyrethrum), b) DDT, c) Oxadiazine, zum Beispiel Indoxacarb, d) Semicarbazone, zum Beispiel Metaflumizon (BAS3201)

13) Acetylcholin-Rezeptor-Agonisten/-Antagonisten, a) aus der Gruppe der Chloronicotinyle,
zum Beispiel Acetamiprid, AKD 1022, Clothianidin, Dinotefuran, Imidacloprid, Imidaclothiz, Nitenpyram, Nithiazine, Thiacloprid, Thiamethoxam, b) Nicotine, Bensultap, Cartap, c) Sulfoxaflor (N-[methyloxido[1-[6-(trifluoromethyl)-3-pyridinyl]ethyl]-λ4-sulfanyliden]-cyanamid)

14) Acetylcholin-Rezeptor-Modulatoren aus der Gruppe der Spinosyne, zum Beispiel Spinosad

15) GABA-gesteuerte Chlorid-Kanal-Antagonisten, a) aus der Gruppe der Organochlorine, zum Beispiel Camphechlor, Chlordane, Endosulfan, Gamma-HCH, HCH, Heptachlor, Lindane, Methoxychlor, b) Fiprole, zum Beispiel Acetoprole, Ethiprole, Fipronil, Pyrafluprole, Pyriprole, Vaniliprole;

16) Chlorid-Kanal-Aktivatoren, zum Beispiel Abamectin, Emamectin, Emamectinbenzoate, Ivermectin, Lepimectin, Milbemycin;

17) Juvenilhormon-Mimetika, zum Beispiel Diofenolan, Epofenonane, Fenoxycarb, Hydroprene, Kinoprene, Methoprene, Pyriproxifen, Triprene;

18) Ecdysonagonisten/disruptoren, zum Beispiel Chromafenozide, Halofenozide, Methoxyfenozide, Tebufenozide;

19) Inhibitoren der Chitinbiosynthese, zum Beispiel Bistrifluron, Chlofluazuron, Diflubenzuron, Fluazuron, Flucycloxuron, Flufenoxuron, Hexaflumuron, Lufenuron, Novaluron, Noviflumuron, Penfluron, Teflubenzuron, Triflumuron, Buprofezin, Cyromazine;

I10) Inhibitoren der oxidativen Phosphorylierung, a) ATP-Disruptoren, z. B. Diafenthiuron, b) Organozinnverbindungen, zum Beispiel Azocyclotin, Cyhexatin, Fenbutatin-oxide;

I11) Entkoppler der oxidativen Phoshorylierung durch Unterbrechung des H-Protongradienten, a) aus der Gruppe der Pyrrole, zum Beispiel Chlorfenapyr, b) aus der Klasse der Dinitrophenole, zum Beispiel Binapacyrl, Dinobuton, Dinocap, DNOC, Meptyldinocap;

I12) Seite-I-Elektronentransportinhibitoren, beispielsweise METI's, insbesondere als Beispiele Fenazaquin, Fenpyroximate, Pyrimidifen, Pyridaben, Tebufenpyrad, Tolfenpyrad oder auch Hydramethylnon, Dicofol;

I13) Seite-II-Elektronentransportinhibitoren, z. B. Rotenone;

I14) Seite-III-Elektronentransportinhibitoren, z. B. Acequinocyl, Fluacrypyrim;

I15) Mikrobielle Disruptoren der Insektendarmmembran, z. B. Bacillus thuringiensis-Stämme;

I16) Inhibitoren der Fettsynthese, a) aus der Gruppe der Tetronsäuren, zum Beispiel Spirodiclofen, Spiromesifen, b) aus der Klasse der Tetramsäuren, zum Beispiel Spirotetramat, cis-3-(2,5-dimethylphenyl)-4-hydroxy-8-methoxy-1-azaspiro[4.5]dec-3-en-2-on;

I17) Oktopaminerge Agonisten, zum Beispiel Amitraz;

I18) Inhibitoren der Magnesium-stimulierten ATPase, z. B. Propargite;

I19) Nereistoxin-Analoge, zum Beispiel Thiocyclam hydrogen oxalate, Thiosultapsodium;

I20) Agonisten des Ryanodin-Rezeptors, a) aus der Gruppe der Benzoesäuredicarboxamide, zum Beispiel Flubendiamide, b) aus der Gruppe der Anthranilamide, zum Beispiel Rynaxypyr (3-Bromo-N-{4-chloro-2-methyl-6-[(methylamino)carbonyl]phenyl}-1-(3-chloropyridin-2-yl)-1H-pyrazole-5-carboxamide), Cyazypyr (ISO-proposed) (3-Bromo-N-{4-cyan-2-methyl-6-[(methylamino)carbonyl]phenyl}-1-(3-chlorpyridin-2-yl)-1H-pyrazol-5-carboxamid) (bekannt aus WO 2004067528);

I21) Biologika, Hormone oder Pheromone, z. B. Azadirachtin, Bacillus spec., Beauveria spec., Codlemone, Metarrhizium spec., Paecilomyces spec., Thuringiensin, Verticillium spec.;

I22) Wirkstoffe mit unbekannten oder nicht spezifischen Wirkmechanismen, a) Begasungsmittel, zum Beispiel Aluminium phosphide, Methyl bromide, Sulfuryl fluoride, b) Fraßhemmer, zum Beispiel Cryolite, Flonicamid, Pymetrozine, c) Milbenwachstums-inhibitoren, zum Beispiel Clofentezine, Etoxazole, Hexythiazox, d) Amidoflumet, Benclothiaz, Benzoximate, Bifenazate, Bromopropylate, Buprofezin, Chinomethionat, Chlordimeform, Chlorobenzilate, Chloropicrin, Clothiazoben, Cycloprene, Cyflumetofen, Dicyclanil, Fenoxacrim, Fentrifanil, Flubenzimine, Flufenerim, Flutenzin, Gossyplure, Hydramethylnone, Japonilure, Metoxadiazone, Petroleum, Piperonyl butoxide, Potassium oleate, Pyridalyl, Sulfluramid, Tetradifon, Tetrasul, Triarathene, Verbutin oder Lepimectin.

Safener sind vorzugsweise ausgewählt aus der Gruppe bestehend aus:

S1) Verbindungen der Formel (S1),

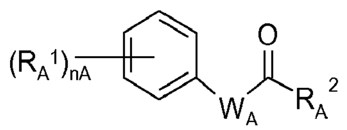

**(S1)**

wobei die Symbole und Indizes folgende Bedeutungen haben:

$n_A$ ist eine natürliche Zahl von 0 bis 5, vorzugsweise 0 bis 3;

$R_A^1$ ist Halogen, $(C_1-C_4)$Alkyl, $(C_1-C_4)$Alkoxy, Nitro oder $(C_1-C_4)$Haloalkyl,

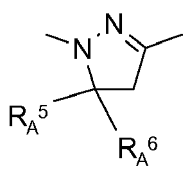

**($W_A^1$)**

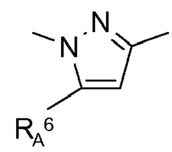

**($W_A^2$)**

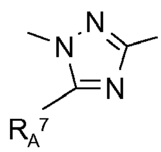

**($W_A^3$)**

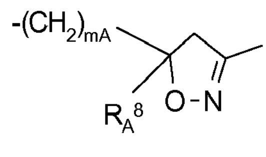

**($W_A^4$)**

$W_A$ ist ein unsubstituierter oder substituierter divalenter heterocyclischer Rest aus der Gruppe der teilungesättigten oder aromatischen Fünfring-Heterocyclen mit 1 bis 3 Heteroringatomen aus der Gruppe N und O, wobei mindestens ein N-Atom und höchstens ein O-Atom im Ring enthalten ist, vorzugsweise ein Rest aus der Gruppe ($W_A^1$) bis ($W_A^4$),

$m_A$ ist 0 oder 1;

$R_A^2$ ist $OR_A^3$, $SR_A^3$ oder $NR_A^3R_A^4$ oder ein gesättigter oder ungesättigter 3- bis 7-gliedriger Heterocyclus mit mindestens einem N-Atom und bis zu 3 Heteroatomen, vorzugsweise aus der Gruppe O und S, der über das N-Atom mit der Carbonylgruppe in (S1) verbunden ist und unsubstituiert oder durch Reste aus der Gruppe $(C_1-C_4)$Alkyl, $(C_1-C_4)$Alkoxy oder gegebenenfalls substituiertes Phenyl substituiert ist, vorzugsweise ein Rest der Formel $OR_A^3$, $NHR_A^4$ oder $N(CH_3)_2$, insbesondere der Formel $OR_A^3$;

$R_A^3$ ist Wasserstoff oder ein unsubstituierter oder substituierter aliphatischer Kohlenwasserstoffrest, vorzugsweise mit insgesamt 1 bis 18 C-Atomen;

$R_A^4$ ist Wasserstoff, $(C_1-C_6)$Alkyl, $(C_1-C_6)$Alkoxy oder substituiertes oder unsubstituiertes Phenyl;

$R_A^5$ ist H, $(C_1-C_8)$Alkyl, $(C_1-C_8)$Haloalkyl, $(C_1-C_4)$Alkoxy$(C_1-C_8)$Alkyl, Cyano oder $COOR_A^9$, worin $R_A^9$ Wasserstoff, $(C_1-C_8)$Alkyl, $(C_1-C_8)$Haloalkyl, $(C_1-C_4)$Alkoxy-$(C_1-C_4)$alkyl, $(C_1-C_6)$Hydroxyalkyl, $(C_3-C_{12})$Cycloalkyl oder Tri-$(C_1-C_4)$-alkyl-silyl ist;

$R_A^6$, $R_A^7$, $R_A^8$ sind gleich oder verschieden Wasserstoff, $(C_1-C_8)$Alkyl, $(C_1-C_8)$Haloalkyl, $(C_3-C_{12})$Cycloalkyl oder substituiertes oder unsubstituiertes Phenyl;

vorzugsweise:

a) Verbindungen vom Typ der Dichlorphenylpyrazolin-3-carbonsäure ($S1^a$), vorzugsweise Verbindungen wie 1-(2,4-Dichlorphenyl)-5-(ethoxycarbonyl)-5-methyl- 2-pyrazolin-3-carbonsäure, 1-(2,4-Dichlorphenyl)-5-(ethoxycarbonyl)-5-methyl-2-pyrazolin-3-carbonsäure-ethylester (S1-1) ("Mefenpyr-diethyl"), und verwandte Verbindungen, wie sie in der WO-A-91/07874 beschrieben sind;
b) Derivate der Dichlorphenylpyrazolcarbonsäure ($S1^b$), vorzugsweise Verbindungen wie 1-(2,4-Dichlorphenyl)-5-methyl-pyrazol-3-carbonsäureethylester (S1-2), 1-(2,4-Di-chlorphenyl)-5-isopropyl-pyrazol-3-carbonsäureethylester (S1-3), 1-(2,4-Dichlor-phenyl)-5-(1,1-dimethyl-ethyl)pyrazol-3-carbonsäureethylester (S1-4) und verwandte Verbindungen, wie sie in EP-A-333 131 und EP-A-269 806 beschrieben sind;
c) Derivate der 1,5-Diphenylpyrazol-3-carbonsäure ($S1^c$), vorzugsweise Verbindungen wie 1-(2,4-Dichlorphenyl)-5-phenylpyrazol-3-carbonsäureethylester (S1-5), 1-(2-Chlorphenyl)-5-phenylpyrazol-3-carbonsäuremethylester (S1-6) und verwandte Verbindungen wie sie beispielsweise in der EP-A-268554 beschrieben sind;
d) Verbindungen vom Typ der Triazolcarbonsäuren ($S1^d$), vorzugsweise Verbindungen wie Fenchlorazol(-ethylester), d.h. 1-(2,4-Dichlorphenyl)-5-trichlormethyl-(1H)-1,2,4-triazol-3-carbonsäureethylester (S1-7), und verwandte Verbindungen wie sie in EP-A-174 562 und EP-A-346 620 beschrieben sind;
e) Verbindungen vom Typ der 5-Benzyl- oder 5-Phenyl-2-isoxazolin-3- carbonsäure oder der 5,5-Diphenyl-2-isoxazolin-3-carbonsäure ($S1^e$), vorzugsweise Verbindungen wie 5-(2,4-Dichlorbenzyl)-2-isoxazolin-3-carbonsäureethylester (S1-8) oder 5-Phenyl-2-isoxazolin-3-carbonsäureethylester (S1-9) und verwandte Verbindungen, wie sie in WO-A-91/08202 beschrieben sind, bzw. 5,5-Diphenyl-2-isoxazolin-3-carbonsäure (S1-10) oder 5,5-Diphenyl-2-isoxazolin-3-carbonsäureethylester (S1-11) ("Isoxadifen-ethyl") oder -n-propylester (S1-12) oder der 5-(4-Fluorphenyl)-5-phenyl-2-isoxazolin-3-carbonsäureethylester (S1-13), wie sie in der Patentanmeldung WO-A-95/07897 beschrieben sind.

S2) Chinolinderivate der Formel (S2),

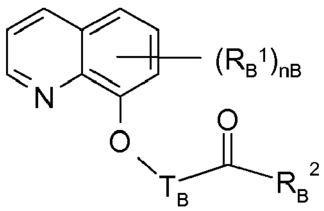

**(S2)**

wobei die Symbole und Indizes folgende Bedeutungen haben:

$R_B^1$ ist Halogen, $(C_1-C_4)$Alkyl, $(C_1-C_4)$Alkoxy, Nitro oder $(C_1-C_4)$Haloalkyl,

$n_B$ ist eine natürliche Zahl von 0 bis 5, vorzugsweise 0 bis 3;

$R_B^2$ ist $OR_B^3$, $SR_B^3$ oder $NR_B^3R_B^4$ oder ein gesättigter oder ungesättigter 3- bis 7-gliedriger Heterocyclus mit mindestens einem N-Atom und bis zu 3 Heteroatomen, vorzugsweise aus der Gruppe O und S, der über das N-Atom mit der Carbonylgruppe in (S2) verbunden ist und unsubstituiert oder durch Reste aus der Gruppe $(C_1-C_4)$Alkyl, $(C_1-C_4)$Alkoxy oder gegebenenfalls substituiertes Phenyl substituiert ist, vorzugsweise ein Rest der Formel $OR_B^3$, $NHR_B^4$ oder $N(CH_3)_2$, insbesondere der Formel $OR_B^3$;

$R_B^3$ ist Wasserstoff oder ein unsubstituierter oder substituierter aliphatischer Kohlenwasserstoffrest, vorzugsweise mit insgesamt 1 bis 18 C-Atomen;

$R_B^4$ ist Wasserstoff, $(C_1-C_6)$Alkyl, $(C_1-C_6)$Alkoxy oder substituiertes oder unsubstituiertes Phenyl;

$T_B$ ist eine ($C_1$ oder $C_2$)-Alkandiylkette, die unsubstituiert oder mit einem oder zwei $(C_1-C_4)$Alkylresten oder mit $[(C_1-C_3)$-Alkoxy]-carbonyl substituiert ist;

vorzugsweise:

a) Verbindungen vom Typ der 8-Chinolinoxyessigsäure ($S2^a$), vorzugsweise (5-Chlor-8-chinolinoxy)essigsäure-(1-methylhexyl)ester ("Cloquintocet-mexyl") (S2-1), (5-Chlor-8-chinolinoxy)essigsäure-(1,3-dimethyl-but-1-yl)ester (S2-2), (5-Chlor-8-chinolinoxy)essigsäure-4-allyloxy-butylester (S2-3), (5-Chlor-8-chinolin-oxy)essigsäure-1-allyloxy-prop-2-ylester (S2-4), (5-Chlor-8-chinolinoxy)essigsäure-ethylester (S2-5), (5-Chlor-8-chinolinoxy)essigsäuremethylester (S2-6), (5-Chlor-8-chinolinoxy)essigsäureallylester (S2-7), (5-Chlor-8-chinolinoxy)essigsäure-2-(2-propyliden-iminoxy)-1-ethylester (S2-8), (5-Chlor-8-chinolinoxy)essigsäure-2-oxo-prop-1-ylester (S2-9) und verwandte Verbindungen, wie sie in EP-A-86 750, EP-A-94 349 und EP-A-191 736 oder EP-A-0 492 366 beschrieben sind, sowie (5-Chlor-8-chinolinoxy)essigsäure (S2-10), deren Hydrate und Salze, beispielsweise deren Lithium-, Natrium- Kalium-, Kalzium-, Magnesium-, Aluminium-, Eisen-, Ammonium-, quartäre Ammonium-, Sulfonium-, oder Phosphoniumsalze wie sie in der WO-A-2002/34048 beschrieben sind;

b) Verbindungen vom Typ der (5-Chlor-8-chinolinoxy)malonsäure ($S2^b$), vorzugsweise Verbindungen wie (5-Chlor-8-chinolinoxy)malonsäurediethylester, (5-Chlor- 8-chinolinoxy)malonsäurediallylester, (5-Chlor-8-chinolinoxy)malonsäure-methyl-ethylester und verwandte Verbindungen, wie sie in EP-A-0 582 198 beschrieben sind.

S3) Verbindungen der Formel (S3)

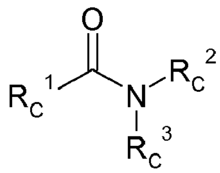

**(S3)**

wobei die Symbole und Indizes folgende Bedeutungen haben:

$R_C^1$ ist $(C_1-C_4)$Alkyl, $(C_1-C_4)$Haloalkyl, $(C_2-C_4)$Alkenyl, $(C_2-C_4)$Haloalkenyl, $(C_3-C_7)$Cycloalkyl, vorzugsweise Dichlormethyl;

$R_C^2$ $R_C^3$ sind gleich oder verschieden Wasserstoff, $(C_1-C_4)$Alkyl, $(C_2-C_4)$Alkenyl, $(C_2-C_4)$Alkinyl, $(C_1-C_4)$Haloalkyl, $(C_2-C_4)$Haloalkenyl, $(C_1-C_4)$Alkylcarbamoyl-$(C_1-C_4)$alkyl, $(C_2-C_4)$Alkenylcarbamoyl-$(C_1-C_4)$alkyl, $(C_1-C_4)$Alkoxy-$(C_1-C_4)$alkyl, Dioxolanyl-$(C_1-C_4)$alkyl, Thiazolyl, Furyl, Furylalkyl, Thienyl, Piperidyl, substituiertes oder unsubstituiertes Phenyl, oder $R_C^2$ und $R_C^3$ bilden zusammen einen substituierten oder unsubstituierten heterocyclischen Ring, vorzugsweise einen Oxazolidin-, Thiazolidin-, Piperidin-, Morpholin-, Hexahydropyrimidin- oder Benzoxazinring; vorzugsweise: Wirkstoffe vom Typ der Dichloracetamide, die häufig als Vorauflaufsafener (bodenwirksame Safener) angewendet werden, wie z. B. "Dichlormid" (N,N-Diallyl-2,2-dichloracetamid) (S3-1), "R-29148" (3-Dichloracetyl-2,2,5-trimethyl-1,3-oxazolidin) der Firma Stauffer (S3-2), "R-28725" (3-Dichloracetyl-2,2,-dimethyl-1,3-oxazolidin) der Firma Stauffer (S3-3), "Benoxacor" (4-Dichloracetyl-3,4-dihydro-3-methyl-2H-1,4-benzoxazin) (S3-4), "PPG-1292" (N-Allyl-N-[(1,3-dioxolan-2-yl)-methyl]-dichloracetamid) der Firma PPG Industries (S3-5), "DKA-24" (N-Allyl-N-[(allylaminocarbonyl)methyl]-dichloracetamid) der Firma Sagro-Chem (S3-6), "AD-67" oder "MON 4660" (3-Dichloracetyl-1-oxa-3-aza-spiro[4,5]decan) der Firma Nitrokemia bzw. Monsanto (S3-7), "TI-35" (1-Dichloracetyl-azepan) der Firma TRI-Chemical RT (S3-8), "Diclonon" (Dicyclonon) oder "BAS145138" oder "LAB145138" (S3-9) ((RS)-1-Dichloracetyl-3,3,8a-trimethylperhydropyrrolo[1,2-a]pyrimidin-6-on) der Firma BASF, "Furilazol" oder "MON 13900" ((RS)-3-Dichloracetyl-5-(2-furyl)-2,2-dimethyloxazolidin) (S3-10); sowie dessen (R)-Isomer (S3-11).

S4) N-Acylsulfonamide der Formel (S4) und ihre Salze,

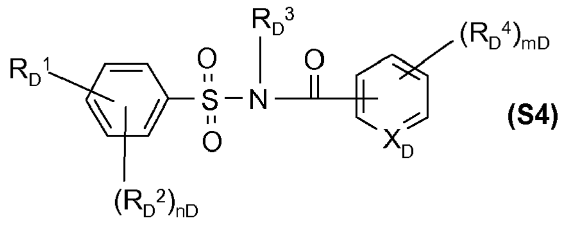

**(S4)**

worin die Symbole und Indizes folgende Bedeutungen haben:

$X_D$ ist CH oder N;

$R_D^1$ ist CO-$NR_D^5R_D^6$ oder NHCO-$R_D^7$;

$R_D^2$ ist Halogen, $(C_1-C_4)$Haloalkyl, $(C_1-C_4)$Haloalkoxy, Nitro, $(C_1-C_4)$Alkyl, $(C_1-C_4)$Alkoxy, $(C_1-C_4)$Alkylsulfonyl, $(C_1-C_4)$Alkoxycarbonyl oder $(C_1-C_4)$Alkylcarbonyl;

$R_D^3$ ist Wasserstoff, $(C_1-C_4)$Alkyl, $(C_2-C_4)$Alkenyl oder $(C_2-C_4)$Alkinyl;

$R_D^4$ ist Halogen, Nitro, $(C_1-C_4)$Alkyl, $(C_1-C_4)$Haloalkyl, $(C_1-C_4)$Haloalkoxy, $(C_3-C_6)$Cycloalkyl, Phenyl, $(C_1-C_4)$Alkoxy, Cyano, $(C_1-C_4)$Alkylthio, $(C_1-C_4)$Alkylsulfinyl, $(C_1-C_4)$Alkylsulfonyl, $(C_1-C_4)$Alkoxycarbonyl oder $(C_1-C_4)$Alkylcarbonyl,

$R_D^5$ ist Wasserstoff, $(C_1-C_6)$Alkyl, $(C_3-C_6)$Cycloalkyl, $(C_2-C_6)$Alkenyl, $(C_2-C_6)$Alkinyl, $(C_5-C_6)$Cycloalkenyl, Phenyl oder 3- bis 6-gliedriges Heterocyclyl enthaltend $v_D$ Heteroatome aus der Gruppe Stickstoff, Sauerstoff und Schwefel, wobei die sieben letztgenannten Reste durch $v_D$ Substituenten aus der Gruppe Halogen, $(C_1-C_6)$Alkoxy, $(C_1-C_6)$Haloalkoxy, $(C_1-C_2)$Alkylsulfinyl, $(C_1-C_2)$Alkylsulfonyl, $(C_3-C_6)$Cycloalkyl, $(C_1-C_4)$Alkoxycarbonyl, $(C_1-C_4)$Alkylcarbonyl und Phenyl und im Falle cyclischer Reste auch $(C_1-C_4)$ Alkyl und $(C_1-C_4)$Haloalkyl substituiert sind;

$R_D^6$ ist Wasserstoff, $(C_1-C_6)$Alkyl, $(C_2-C_6)$Alkenyl oder $(C_2-C_6)$Alkinyl, wobei die drei letztgenannten Reste durch $v_D$ Reste aus der Gruppe Halogen, Hydroxy, $(C_1-C_4)$Alkyl, $(C_1-C_4)$Alkoxy und $(C_1-C_4)$Alkylthio substituiert sind, oder

$R_D^5$ und $R_D^6$ gemeinsam mit dem dem sie tragenden Stickstoffatom einen Pyrrolidinyl-oder Piperidinyl-Rest bilden;

$R_D^7$ ist Wasserstoff, $(C_1-C_4)$Alkylamino, Di-$(C_1-C_4)$alkylamino, $(C_1-C_6)$Alkyl, $(C_3-C_6)$Cycloalkyl, wobei die 2 letztgenannten Reste durch $v_D$ Substituenten aus der Gruppe Halogen, $(C_1-C_4)$Alkoxy, $(C_1-C_6)$Haloalkoxy und $(C_1-C_4)$Alkylthio und im Falle cyclischer Reste auch $(C_1-C_4)$Alkyl und $(C_1-C_4)$Haloalkyl substituiert sind;

$n_D$ ist 0, 1 oder 2;

$m_D$ ist 1 oder 2;

$v_D$ ist 0, 1, 2 oder 3;

davon bevorzugt sind Verbindungen vom Typ der N-Acylsulfonamide, z.B. der nachfolgenden Formel ($S4^a$), die z. B. bekannt sind aus WO-A-97/45016

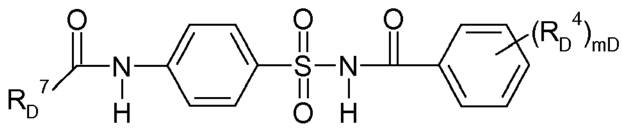

**($S4^a$)**

worin

$R_D^7$ $(C_1-C_6)$Alkyl, $(C_3-C_6)$Cycloalkyl, wobei die 2 letztgenannten Reste durch $v_D$ Substituenten aus der Gruppe Halogen, $(C_1-C_4)$Alkoxy, $(C_1-C_6)$Haloalkoxy und $(C_1-C_4)$Alkylthio und im Falle cyclischer Reste auch $(C_1-C_4)$Alkyl und $(C_1-C_4)$Haloalkyl substituiert sind;

$R_D^4$ Halogen, $(C_1-C_4)$Alkyl, $(C_1-C_4)$Alkoxy, $CF_3$;

$m_D$ 1 oder 2;

$v_D$ ist 0, 1, 2 oder 3 bedeutet; sowie Acylsulfamoylbenzoesäureamide, z.B. der nachfolgenden Formel ($S4^b$), die z.B. bekannt sind aus WO-A-99/16744,

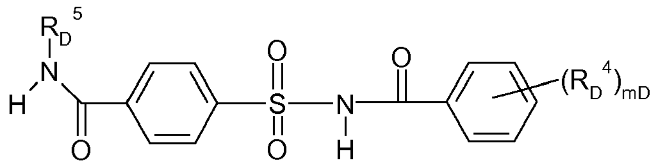

**(S4b)**

z.B. solche worin
$R_D^5$ = Cyclopropyl und ($R_D^4$) = 2-OMe ist ("Cyprosulfamide", S4-1),
$R_D^5$ = Cyclopropyl und ($R_D^4$) = 5-Cl-2-OMe ist (S4-2),
$R_D^5$ = Ethyl und ($R_D^4$) = 2-OMe ist (S4-3),
$R_D^5$ = Isopropyl und ($R_D^4$) = 5-Cl-2-OMe ist (S4-4) und
$R_D^5$ = Isopropyl und ($R_D^4$) = 2-OMe ist (S4-5).
sowie Verbindungen vom Typ der N-Acylsulfamoylphenylharnstoffe der Formel (S4c), die z.B. bekannt sind aus der EP-A-365484,

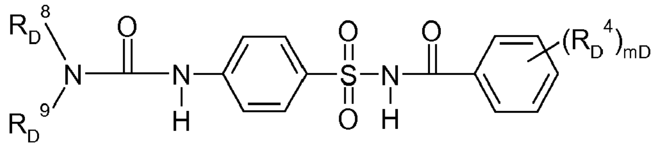

**(S4c)**

worin

| | |
|---|---|
| $R_D^8$ und $R_D^9$ | unabhängig voneinander Wasserstoff, $(C_1-C_8)$Alkyl, $(C_3-C_8)$Cycloalkyl, $(C_3-C_6)$Alkenyl, $(C_3-C_6)$Alkinyl, |
| $R_D^4$ | Halogen, $(C_1-C_4)$Alkyl, $(C_1-C_4)$Alkoxy, $CF_3$ |
| $m_D$ | 1 oder 2 bedeutet; |

beispielsweise
1-[4-(N-2-Methoxybenzoylsulfamoyl)phenyl]-3-methylharnstoff,
1-[4-(N-2-Methoxybenzoylsulfamoyl)phenyl]-3,3-dimethylharnstoff,
1-[4-(N-4,5-Dimethylbenzoylsulfamoyl)phenyl]-3-methylharnstoff.

S5) Wirkstoffe aus der Klasse der Hydroxyaromaten und der aromatischaliphatischen Carbonsäurederivate (S5), z.B. 3,4,5-Triacetoxybenzoesäureethylester, 3,5-Di-methoxy-4-hydroxybenzoesäure, 3,5-Dihydroxybenzoesäure, 4-Hydroxysalicylsäure, 4-Fluorsalicyclsäure, 2-Hydroxyzimtsäure, 2,4-Dichlorzimtsäure, wie sie in der WO-A-2004/084631, WO-A-2005/015994, WO-A-2005/016001 beschrieben sind.

S6) Wirkstoffe aus der Klasse der 1,2-Dihydrochinoxalin-2-one (S6), z.B. 1-Methyl-3-(2-thienyl)-1,2-dihydrochinoxalin-2-on, 1-Methyl-3-(2-thienyl)-1,2-dihydrochinoxalin-2-thion, 1-(2-Aminoethyl)-3-(2-thienyl)-1,2-dihydro-chinoxalin-2-on-hydrochlorid, 1-(2-Methylsulfonylaminoethyl)-3-(2-thienyl)-1,2-dihydrochinoxa-lin-2-on, wie sie in der WO-A-2005/112630 beschrieben sind.

S7) Verbindungen der Formel (S7),wie sie in der WO-A-1998/38856 beschrieben

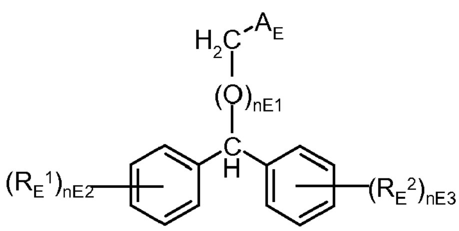

**(S7)**

sind
worin die Symbole und Indizes folgende Bedeutungen haben:
$R_E^1$, $R_E^2$ sind unabhängig voneinander Halogen, $(C_1-C_4)$Alkyl, $(C_1-C_4)$Alkoxy, $(C_1-C_4)$Haloalkyl, $(C_1-C_4)$Alkylamino,

Di-($C_1$-$C_4$)Alkylamino, Nitro;
$A_E$ ist $COOR_E^3$ oder $COSR_E^4$
$R_E^3$, $R_E^4$ sind unabhängig voneinander Wasserstoff, ($C_1$-$C_4$)Alkyl, ($C_2$-$C_6$)Alkenyl, ($C_2$-$C_4$)Alkinyl, Cyanoalkyl, ($C_1$-$C_4$)Haloalkyl, Phenyl, Nitrophenyl, Benzyl, Halobenzyl, Pyridinylalkyl und Alkylammonium,
$n_E^1$ ist 0 oder 1
$n_E^2$, $n_E^3$ sind unabhängig voneinander 0, 1 oder 2,
vorzugsweise Diphenylmethoxyessigsäure, Diphenylmethoxyessigsäureethylester, Diphenyl-methoxyessigsäuremethylester (CAS-Reg.Nr. 41858-19-9) (S7-1).

S8) Verbindungen der Formel (S8),wie sie in der WO-A-98/27049 beschrieben sind

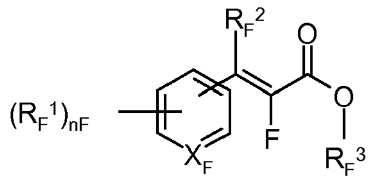

**(S8)**

Worin

$X_F$ CH oder N,
$n_F$ für den Fall, dass $X_F$=N ist, eine ganze Zahl von 0 bis 4 und für den Fall, dass $X_F$=CH ist, eine ganze Zahl von 0 bis 5 ,
$R_F^1$ Halogen, ($C_1$-$C_4$)Alkyl, ($C_1$-$C_4$)Haloalkyl, ($C_1$-$C_4$)Alkoxy, ($C_1$-$C_4$)Haloalkoxy, Nitro, ($C_1$-$C_4$)Alkylthio, ($C_1$-$C_4$)-Alkylsulfonyl, ($C_1$-$C_4$)Alkoxycarbonyl, ggf. substituiertes. Phenyl, ggf. substituiertes Phenoxy,
$R_F^2$ Wasserstoff oder ($C_1$-$C_4$)Alkyl
$R_F^3$ Wasserstoff, ($C_1$-$C_8$)Alkyl, ($C_2$-$C_4$)Alkenyl, ($C_2$-$C_4$)Alkinyl, oder Aryl, wobei jeder der vorgenannten C-haltigen Reste unsubstituiert oder durch einen oder mehrere, vorzugsweise bis zu drei gleiche oder verschiedene Reste aus der Gruppe, bestehend aus Halogen und Alkoxy substituiert ist; bedeuten, oder deren Salze,

vorzugsweise Verbindungen worin

$X_F$ CH,
$n_F$ eine ganze Zahl von 0 bis 2 ,
$R_F^1$ Halogen, ($C_1$-$C_4$)Alkyl, ($C_1$-$C_4$)Haloalkyl, ($C_1$-$C_4$)Alkoxy, ($C_1$-$C_4$)Haloalkoxy,
$R_F^2$ Wasserstoff oder ($C_1$-$C_4$)Alkyl,
$R_F^3$ Wasserstoff, ($C_1$-$C_8$)Alkyl, ($C_2$-$C_4$)Alkenyl, ($C_2$-$C_4$)Alkinyl, oder Aryl, wobei jeder der vorgenannten C-haltigen Reste unsubstituiert oder durch einen oder mehrere, vorzugsweise bis zu drei gleiche oder verschiedene Reste aus der Gruppe, bestehend aus Halogen und Alkoxy substituiert ist, bedeuten, oder deren Salze.

S9) Wirkstoffe aus der Klasse der 3-(5-Tetrazolylcarbonyl)-2-chinolone (S9), z.B. 1,2-Dihydro-4-hydroxy-1-ethyl-3-(5-tetrazolylcarbonyl)-2-chinolon (CAS-Reg.Nr. 219479-18-2), 1,2-Dihydro-4-hydroxy-1-methyl-3-(5-tetrazolyl-carbonyl)-2-chinolon (CAS-Reg.Nr. 95855-00-8), wie sie in der WO-A-1999/000020 beschrieben sind.

S10) Verbindungen der Formeln ($S10^a$) oder ($S10^b$) wie sie in der WO-A-2007/023719 und WO-A-2007/023764 beschrieben sind

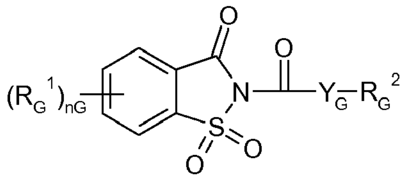

**($S10^a$)**

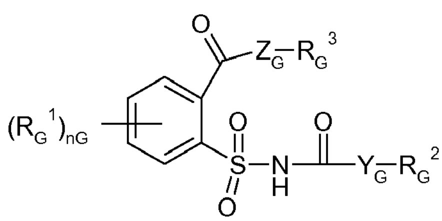

**($S10^b$)**

worin

$R_G^1$ Halogen, $(C_1-C_4)$Alkyl, Methoxy, Nitro, Cyano, $CF_3$, $OCF_3$
$Y_G$, $Z_G$ unabhängig voneinander O oder S,
$n_G$ eine ganze Zahl von 0 bis 4,
$R_G^2$ $(C_1-C_{16})$Alkyl, $(C_2-C_6)$Alkenyl, $(C_3-C_6)$Cycloalkyl, Aryl; Benzyl, Halogenbenzyl,
$R_G^3$ Wasserstoff oder $(C_1-C_6)$Alkyl bedeutet.

S11) Wirkstoffe vom Typ der Oxyimino-Verbindungen (S11), die als Saatbeizmittel bekannt sind, wie z. B. "Oxabetrinil" ((Z)-1,3-Dioxolan-2-ylmethoxyimino-(phenyl)acetonitril) (S11-1), das als Saatbeiz-Safener für Hirse gegen Schäden von Metolachlor bekannt ist, "Fluxofenim" (1-(4-Chlorphenyl)-2,2,2-trifluor-1-ethanon-O-(1,3-dioxolan-2-ylmethyl)-oxim) (S11-2), das als Saatbeiz-Safener für Hirse gegen Schäden von Metolachlor bekannt ist, und "Cyometrinil" oder "CGA-43089" ((Z)-Cyanomethoxyimino(phenyl)acetonitril) (S11-3), das als Saatbeiz-Safener für Hirse gegen Schäden von Metolachlor bekannt ist.

S12) Wirkstoffe aus der Klasse der Isothiochromanone (S12), wie z.B. Methyl-[(3-oxo-1H-2-benzothiopyran-4(3H)-yliden)methoxy]acetat (CAS-Reg.Nr. 205121-04-6) (S12-1) und verwandte Verbindungen aus WO-A-1998/13361.

S13) Eine oder mehrere Verbindungen aus Gruppe (S13): "Naphthalic anhydrid" (1,8-Naphthalindicarbonsäureanhydrid) (S13-1), das als Saatbeiz-Safener für Mais gegen Schäden von Thiocarbamatherbiziden bekannt ist, "Fenclorim" (4,6-Dichlor-2-phenylpyrimidin) (S13-2), das als Safener für Pretilachlor in gesätem Reis bekannt ist, "Flurazole" (Benzyl-2-chlor-4-trifluormethyl-1,3-thiazol-5-carboxylat) (S13-3), das als Saatbeiz-Safener für Hirse gegen Schäden von Alachlor und Metolachlor bekannt ist, "CL 304415" (CAS-Reg.Nr. 31541-57-8) (4-Carboxy-3,4-dihydro-2H-1-benzopyran-4-essigsäure) (S13-4) der Firma American Cyanamid, das als Safener für Mais gegen Schäden von Imidazolinonen bekannt ist, "MG 191" (CAS-Reg.Nr. 96420-72-3) (2-Dichlormethyl-2-methyl-1,3-dioxolan) (S13-5) der Firma Nitrokemia, das als Safener für Mais bekannt ist, "MG-838" (CAS-Reg.Nr. 133993-74-5) (2-propenyl 1-oxa-4-azaspiro[4.5]decan-4-carbodithioat) (S13-6) der Firma Nitrokemia, "Disulfoton" (O,O-Diethyl S-2-ethylthioethyl phosphordithioat) (S13-7), "Dietholate" (O,O-Diethyl-O-phenylphosphorothioat) (S13-8), "Mephenate" (4-Chlorphenyl-methylcarbamat) (S13-9).

S14) Wirkstoffe, die neben einer herbiziden Wirkung gegen Schadpflanzen auch Safenerwirkung an Kulturpflanzen wie Reis aufweisen, wie z. B. "Dimepiperate" oder "MY-93" (S-1-Methyl-1-phenylethyl-piperidin-1-carbothioat), das als Safener für Reis gegen Schäden des Herbizids Molinate bekannt ist, "Daimuron" oder "SK 23" (1-(1-Methyl-1-phenylethyl)-3-p-tolyl-harnstoff), das als Safener für Reis gegen Schäden des Herbizids Imazosulfuron bekannt ist, "Cumyluron" = "JC-940" (3-(2-Chlorphenylmethyl)-1-(1-methyl-1-phenylethyl)harnstoff, siehe JP-A-60087254), das als Safener für Reis gegen Schäden einiger Herbizide bekannt ist, "Methoxyphenon" oder "NK 049" (3,3'-Dimethyl-4-methoxy-benzophenon), das als Safener für Reis gegen Schäden einiger Herbizide bekannt ist, "CSB" (1-Brom-4-(chlormethylsulfonyl)benzol) von Kumiai, (CAS-Reg.Nr. 54091-06-4), das als Safener gegen Schäden einiger Herbizide in Reis bekannt ist.

S15) Verbindungen der Formel (S15) oder deren Tautomere wie sie in der WO-A-2008/131861 und WO-A-2008/131860 beschrieben sind

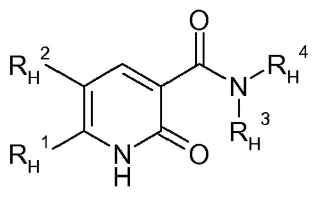

**(S15)**

worin
$R_H^1$ einen $(C_1-C_6)$Haloalkylrest bedeutet und
$R_H^2$ Wasserstoff oder Halogen bedeutet und
$R_H^3$, $R_H^4$ unabhängig voneinander Wasserstoff, $(C_1-C_{16})$Alkyl, $(C_2-C_{16})$Alkenyl oder $(C_2-C_{16})$Alkinyl, wobei jeder der letztgenannten 3 Reste unsubstituiert oder durch einen oder mehrere Reste aus der Gruppe Halogen, Hydroxy, Cyano, $(C_1-C_4)$Alkoxy, $(C_1-C_4)$Haloalkoxy, $(C_1-C_4)$Alkylthio, $(C_1-C_4)$Alkylamino, Di[$(C_1-C_4)$alkyl]-amino, [$(C_1-C_4)$Alkoxy]-carbonyl, [$(C_1-C_4)$Haloalkoxy]-carbonyl, $(C_3-C_6)$Cycloalkyl, das unsubstituiert oder substituiert ist, Phenyl, das unsubstituiert oder substituiert ist, und Heterocyclyl, das unsubstituiert oder substituiert ist, substituiert ist, oder $(C_3-C_6)$Cycloalkyl, $(C_4-C_6)$Cycloalkenyl, $(C_3-C_6)$Cycloalkyl, das an einer Seite des Rings mit einem 4 bis

6-gliedrigen gesättigten oder ungesättigten carbocyclischen Ring kondensiert ist, oder $(C_4-C_6)$Cycloalkenyl, das an einer Seite des Rings mit einem 4 bis 6-gliedrigen gesättigten oder ungesättigten carbocyclischen Ring kondensiert ist, wobei jeder der letztgenannten 4 Reste unsubstituiert oder durch einen oder mehrere Reste aus der Gruppe Halogen, Hydroxy, Cyano, (C1-C4)Alkyl, (C1-C4)Haloalkyl, (C1-C4)Alkoxy, (C1-C4)Haloalkoxy, (C1-C4)Alkylthio, (C1-C4)Alkylamino, Di[(C1-C4)alkyl]-amino, [(C1-C4)Alkoxy]-carbonyl, [(C1-C4)Haloalkoxy]-carbonyl, (C3-C6)Cycloalkyl, das unsubstituiert oder substituiert ist, Phenyl, das unsubstituiert oder substituiert ist, und Heterocyclyl, das unsubstituiert oder substituiert ist, substituiert ist,
bedeutet oder
$R_H^3$ $(C_1-C_4)$-Alkoxy, $(C_2-C_4)$Alkenyloxy, $(C_2-C_6)$Alkinyloxy oder $(C_2-C_4)$Haloalkoxy bedeutet und
$R_H^4$ Wasserstoff oder $(C_1-C_4)$-Alkyl bedeutet oder
$R_H^3$ und $R_H^4$ zusammen mit dem direkt gebundenen N-Atom einen vier- bis achtgliedrigen heterocyclischen Ring, der neben dem N-Atom auch weitere Heteroringatome, vorzugsweise bis zu zwei weitere Heteroringatome aus der Gruppe N, O und S enthalten kann und der unsubstituiert oder durch einen oder mehrere Reste aus der Gruppe Halogen, Cyano, Nitro, $(C_1-C_4)$Alkyl, $(C_1-C_4)$Haloalkyl, $(C_1-C_4)$Alkoxy, $(C_1-C_4)$Haloalkoxy und $(C_1-C_4)$Alkylthio substituiert ist, bedeutet.

S16) Wirkstoffe, die vorrangig als Herbizide eingesetzt werden, jedoch auch Safenerwirkung auf Kulturpflanzen aufweisen, z.B. (2,4-Dichlorphenoxy)essigsäure (2,4-D), (4-Chlorphenoxy)essigsäure, (R,S)-2-(4-Chlor-o-tolyloxy)propionsäure (Mecoprop), 4-(2,4-Dichlorphenoxy)buttersäure (2,4-DB), (4-Chlor-o-tolyloxy)-essigsäure (MCPA), 4-(4-Chlor-o-tolyloxy)buttersäure, 4-(4-Chlorphenoxy)-buttersäure, 3,6-Dichlor-2-methoxybenzoesäure (Dicamba), 1-(Ethoxycarbonyl)ethyl-3,6-dichlor-2-methoxybenzoat (Lactidichlor-ethyl).

Pflanzenreife beeinflussende Stoffe:

Als Kombinationspartner für die Verbindungen der allgemeinen Formel (I) in Mischungsformulierungen oder im Tank-Mix sind beispielsweise bekannte Wirkstoffe, die auf einer Inhibition von beispielsweise 1-Aminocyclopropan-1-carboxylatsynthase, 1-aminocyclopropane-1-carboxylatoxidase und den Ethylenrezeptoren, z. B. ETR1, ETR2, ERS1, ERS2 oder EIN4, beruhen, einsetzbar, wie sie z. B. in Biotechn. Adv. 2006, 24, 357-367; Bot. Bull. Acad. Sin. 199, 40, 1-7 oder Plant Growth Reg. 1993, 13, 41-46 und dort zitierter Literatur beschrieben sind.
Als bekannte die Pflanzenreife beeinflussende Stoffe, die mit den Verbindungen der allgemeinen Formel (I) kombiniert werden können, sind z.B. folgende Wirkstoffe zu nennen (die Verbindungen sind entweder mit dem "common name" nach der International Organization for Standardization (ISO) oder mit dem chemischen Namen oder mit der Codenummer bezeichnet) und umfassen stets sämtliche Anwendungsformen wie Säuren, Salze, Ester und Isomere wie Stereoisomere und optische Isomere. Dabei sind beispielhaft eine und zum Teil auch mehrere Anwendungsformen genannt:
Rhizobitoxin, 2-Amino-ethoxy-vinylglycin (AVG), Methoxyvinylglycin (MVG), Vinylglycin, Aminooxyessigsäure, Sinefungin, S-Adenosylhomocystein, 2-Keto-4-Methylthiobutyrat, (Isopropyliden)-aminooxyessigsäure-2-(methoxy)-2-oxoethylester, (Isopropyliden)-aminooxyessigsäure-2-(hexyloxy)-2-oxoethylester, (Cyclohexylidene)-aminooxyessigsäure-2-(isopropyloxy)-2-oxoethylester, Putrescin, Spermidin, Spermin, 1,8-Diamino4-aminoethyloctan, L-Canalin, Daminozid, 1-Aminocyclopropyl-1-carbonsäure-methylester, N-Methyl-1-aminocyclopropyl-1-carbonsäure, 1-Aminocyclopropyl-1-carbonsäureamid, Substituierte 1-Aminocyclopropyl-1-carbonsäurederivate wie sie in DE3335514, EP30287, DE2906507 oder US5123951 beschrieben werden, 1-Aminocyclopropyl-1-hydroxamsäure, 1-Methylcyclopropen, 3-Methylcyclopropen, 1-Ethylcyclopropen, 1-n-Propylcyclopropen, 1-Cyclopropenyl-Methanol, Carvon, Eugenol, Natriumcycloprop-1-en-1-ylacetat, Natriumcycloprop-2-en-1-ylacetat, Natrium-3-(cycloprop-2-en-1-yl)propanoat, Natrium-3-(cycloprop-1-en-1-yl)propanoat.

Herbizide oder Pflanzenwachstumsregulatoren:

Als Kombinationspartner für die Verbindungen der allgemeinen Formel (I) in Mischungsformulierungen oder im Tank-Mix sind beispielsweise bekannte Wirkstoffe, die auf einer Inhibition von beispielsweise Acetolactat-Synthase, Acetyl-CoA-Carboxylase, Cellulose-Synthase, Enolpyruvylshikimat-3-phosphat-Synthase, Glutamin-Synthetase, p-Hydroxyphenylpyruvat-Dioxygenase, Phytoendesaturase, Photosystem I, Photosystem II, Protoporphyrinogen-Oxidase beruhen, einsetzbar, wie sie z.B. aus Weed Research 26 (1986) 441-445 oder "The Pesticide Manual", 15th edition, The British Crop Protection Council and the Royal Soc. of Chemistry, 2009 und dort zitierter Literatur beschrieben sind.
Als bekannte Herbizide oder Pflanzenwachstumsregulatoren, die mit Verbindungen der allgemeinen Formel (I) kombiniert werden können, sind z.B. folgende Wirkstoffe zu nennen (die Verbindungen sind entweder mit dem "common name" nach der International Organization for Standardization (ISO) oder mit dem chemischen Namen oder mit der Codenummer bezeichnet) und umfassen stets sämtliche Anwendungsformen wie Säuren, Salze, Ester und Isomere

wie Stereoisomere und optische Isomere. Dabei sind beispielhaft eine und zum Teil auch mehrere Anwendungsformen genannt:

Acetochlor, Acibenzolar, Acibenzolar-S-methyl, Acifluorfen, Acifluorfen-sodium, Aclonifen, Alachlor, Allidochlor, Alloxydim, Alloxydim-sodium, Ametryn, Amicarbazone, Amidochlor, Amidosulfuron, Aminocyclopyrachlor, Aminopyralid, Amitrole, Ammoniumsulfamat, Ancymidol, Anilofos, Asulam, Atrazine, Azafenidin, Azimsulfuron, Aziprotryn, Beflubutamid, Benazolin, Benazolin-ethyl, Bencarbazone, Benfluralin, Benfuresate, Bensulide, Bensulfuron, Bensulfuron-methyl, Bentazone, Benzfendizone, Benzobicyclon, Benzofenap, Benzofluor, Benzoylprop, Bicyclopyrone, Bifenox, Bilanafos, Bilanafos-natrium, Bispyribac, Bispyribac-natrium, Bromacil, Bromobutide, Bromofenoxim, Bromoxynil, Bromuron, Buminafos, Busoxinone, Butachlor, Butafenacil, Butamifos, Butenachlor, Butralin, Butroxydim, Butylate, Cafenstrole, Carbetamide, Carfentrazone, Carfentrazone-ethyl, Chlomethoxyfen, Chloramben, Chlorazifop, Chlorazifop-butyl, Chlorbromuron, Chlorbufam, Chlorfenac, Chlorfenacnatrium, Chlorfenprop, Chlorflurenol, Chlorflurenol-methyl, Chloridazon, Chlorimuron, Chlorimuron-ethyl, Chlormequat-chlorid, Chlornitrofen, Chlorophthalim, Chlorthaldimethyl, Chlorotoluron, Chlorsulfuron, Cinidon, Cinidon-ethyl, Cinmethylin, Cinosulfuron, Clethodim, Clodinafop, Clodinafop-propargyl, Clofencet, Clomazone, Clomeprop, Cloprop, Clopyralid, Cloransulam, Cloransulam-methyl, Cumyluron, Cyanamide, Cyanazine, Cyclanilide, Cycloate, Cyclosulfamuron, Cycloxydim, Cycluron, Cyhalofop, Cyhalofop-butyl, Cyperquat, Cyprazine, Cyprazole, 2,4-D, 2,4-DB, Daimuron/Dymron, Dalapon, Daminozid, Dazomet, n-Decanol, Desmedipham, Desmetryn, Detosyl-Pyrazolate (DTP), Diallate, Dicamba, Dichlobenil, Dichlorprop, Dichlorprop-P, Diclofop, Diclofop-methyl, Diclofop-P-methyl, Diclosulam, Diethatyl, Diethatyl-ethyl, Difenoxuron, Difenzoquat, Diflufenican, Diflufenzopyr, Diflufenzopyrnatrium, Dimefuron, Dikegulac-sodium, Dimefuron, Dimepiperate, Dimethachlor, Dimethametryn, Dimethenamid, Dimethenamid-P, Dimethipin, Dimetrasulfuron, Dinitramine, Dinoseb, Dinoterb, Diphenamid, Dipropetryn, Diquat, Diquat-dibromide, Dithiopyr, Diuron, DNOC, Eglinazine-ethyl, Endothal, EPTC, Esprocarb, Ethalfluralin, Ethametsulfuron, Ethametsulfuron-methyl, Ethephon, Ethidimuron, Ethiozin, Ethofumesate, Ethoxyfen, Ethoxyfen-ethyl, Ethoxysulfuron, Etobenzanid, F-5331, d.h. N-[2-Chlor-4-fluor-5-[4-(3-fluorpropyl)-4,5-dihydro-5-oxo-1H-tetrazol-1-yl]-phenyl]-ethansulfonamid, F-7967, d. h. 3-[7-Chlor-5-fluor-2-(trifluormethyl)-1H-benzimidazol-4-yl]-1-methyl-6-(trifluormethyl)pyrimidin-2,4(1H,3H)-dion, Fenoprop, Fenoxaprop, Fenoxaprop-P, Fenoxaprop-ethyl, Fenoxaprop-P-ethyl, Fenoxasulfone, Fentrazamide, Fenuron, Flamprop, Flamprop-M-isopropyl, Flamprop-M-methyl, Flazasulfuron, Florasulam, Fluazifop, Fluazifop-P, Fluazifop-butyl, Fluazifop-P-butyl, Fluazolate, Flucarbazone, Flucarbazone-sodium, Flucetosulfuron, Fluchloralin, Flufenacet (Thiafluamide), Flufenpyr, Flufenpyr-ethyl, Flumetralin, Flumetsulam, Flumiclorac, Flumiclorac-pentyl, Flumioxazin, Flumipropyn, Fluometuron, Fluorodifen, Fluoroglycofen, Fluoroglycofen-ethyl, Flupoxam, Flupropacil, Flupropanate, Flupyrsulfuron, Flupyrsulfuron-methyl-sodium, Flurenol, Flurenol-butyl, Fluridone, Flurochloridone, Fluroxypyr, Fluroxypyr-meptyl, Flurprimidol, Flurtamone, Fluthiacet, Fluthiacet-methyl, Fluthiamide, Fomesafen, Foramsulfuron, Forchlorfenuron, Fosamine, Furyloxyfen, Gibberellinsäure, Glufosinate, Glufosinate-ammonium, Glufosinate-P, Glufosinate-P-ammonium, Glufosinate-P-natrium, Glyphosate, Glyphosate-isopropylammonium, H-9201, d. h. O-(2,4-Dimethyl-6-nitrophenyl)-O-ethyl-isopropylphosphoramidothioat, Halosafen, Halosulfuron, Halosulfuron-methyl, Haloxyfop, Haloxyfop-P, Haloxyfop-ethoxyethyl, Haloxyfop-P-ethoxyethyl, Haloxyfopmethyl, Haloxyfop-P-methyl, Hexazinone, HW-02, d. h. 1-(Dimethoxyphosphoryl)-ethyl(2,4-dichlorphenoxy)acetat, Imazamethabenz, Imazamethabenz-methyl, Imazamox, Imazamox-ammonium, Imazapic, Imazapyr, Imazapyrisopropylammonium, Imazaquin, Imazaquin-ammonium, Imazethapyr, Imazethapyrammonium, Imazosulfuron, Inabenfide, Indanofan, Indaziflam, Indolessigsäure (IAA), 4-Indol-3-ylbuttersäure (IBA), Iodosulfuron, Iodosulfuron-methyl-natrium, Ioxynil, Ipfencarbazone, Isocarbamid, Isopropalin, Isoproturon, Isouron, Isoxaben, Isoxachlortole, Isoxaflutole, Isoxapyrifop, KUH-043, d. h. 3-({[5-(Difluormethyl)-1-methyl-3-(trifluormethyl)-1H-pyrazol-4-yl]methyl}sulfonyl)-5,5-dimethyl-4,5-dihydro-1,2-oxazol, Karbutilate, Ketospiradox, Lactofen, Lenacil, Linuron, Maleinsäurehydrazid, MCPA, MCPB, MCPB-methyl, -ethyl und -natrium, Mecoprop, Mecoprop-natrium, Mecoprop-butotyl, Mecoprop-P-butotyl, Mecoprop-P-dimethylammonium, Mecoprop-P-2-ethylhexyl, Mecoprop-P-kalium, Mefenacet, Mefluidide, Mepiquat-chlorid, Mesosulfuron, Mesosulfuron-methyl, Mesotrione, Methabenzthiazuron, Metam, Metamifop, Metamitron, Metazachlor, Metazasulfuron, Methazole, Methiopyrsulfuron, Methiozolin, Methoxyphenone, Methyldymron, 1-Methylcyclopropen, Methylisothiocyanat, Metobenzuron, Metobromuron, Metolachlor, S-Metolachlor, Metosulam, Metoxuron, Metribuzin, Metsulfuron, Metsulfuron-methyl, Molinate, Monalide, Monocarbamide, Monocarbamide-dihydrogensulfat, Monolinuron, Monosulfuron, Monosulfuron-ester, Monuron, MT-128, d. h. 6-Chlor-N-[(2E)-3-chlorprop-2-en-1-yl]-5-methyl-N-phenylpyridazin-3-amin, MT-5950, d. h. N-[3-Chlor-4-(1-methylethyl)-phenyl]-2-methylpentanamid, NGGC-011, Naproanilide, Napropamide, Naptalam, NC-310, d.h. 4-(2,4-Dichlorobenzoyl)-1-methyl-5-benzyloxypyrazole, Neburon, Nicosulfuron, Nipyraclofen, Nitralin, Nitrofen, Nitrophenolat-natrium (Isomerengemisch), Nitrofluorfen, Nonansäure, Norflurazon, Orbencarb, Orthosulfamuron, Oryzalin, Oxadiargyl, Oxadiazon, Oxasulfuron, Oxaziclomefone, Oxyfluorfen, Paclobutrazol, Paraquat, Paraquat-dichlorid, Pelargonsäure (Nonansäure), Pendimethalin, Pendralin, Penoxsulam, Pentanochlor, Pentoxazone, Perfluidone, Pethoxamid, Phenisopham, Phenmedipham, Phenmedipham-ethyl, Picloram, Picolinafen, Pinoxaden, Piperophos, Pirifenop, Pirifenop-butyl, Pretilachlor, Primisulfuron, Primisulfuron-methyl, Probenazole, Profluazol, Procyazine, Prodiamine, Prifluraline, Profoxydim, Prohexadione, Prohexadione-calcium, Prohydrojasmone, Prometon, Prometryn, Propachlor, Propanil, Propaquizafop, Propazine, Propham, Propisochlor, Propoxycarbazone, Propoxycarbazone-natrium, Propyrisulfuron, Propyzamide, Prosulfalin, Prosulfocarb, Prosulfuron, Prynachlor, Pyraclonil, Pyraflufen, Pyraflufen-ethyl, Pyrasulfotole, Pyrazolynate (Pyrazolate), Pyrazosulfuron, Pyrazosulfuron-ethyl, Pyrazoxyfen, Pyribambenz, Pyribambenz-isopropyl, Pyribambenz-propyl, Pyribenzoxim, Pyributicarb, Pyridafol, Pyridate, Pyriftalid, Pyriminobac, Pyriminobac-methyl, Pyrimisulfan, Pyrithiobac, Pyrithiobac-natrium, Pyroxasulfone, Pyroxsulam, Quinclorac, Quinmerac, Quinoclamine, Quizalofop, Quizalofop-ethyl, Quizalofop-P, Quizalofop-P-ethyl, Quizalofop-P-tefuryl, Rimsulfuron, Saflufenacil, Secbumeton, Sethoxydim, Siduron, Simazine, Simetryn, SN-106279, d. h. Methyl-(2R)-2-({7-[2-chlor-4-(trifluormethyl)phenoxy]-2-naphthyl}oxy)-propanoat, Sulcotrione, Sulfallate (CDEC), Sulfentrazone, Sulfometuron, Sulfometuron-methyl, Sulfosate (Glyphosate-trimesium), Sulfosulfuron, SYN-523, SYP-249, d. h. 1-Ethoxy-3-methyl-1-oxobut-3-en-2-yl-5-[2-chlor-4-(trifluormethyl)phenoxy]-2-nitrobenzoat, SYP-300, d. h. 1-[7-Fluor-3-oxo-4-(prop-2-in-1-yl)-3,4-dihydro-2H-1,4-benzoxazin-6-yl]-3-propyl-2-thioxoimidazolidin-4,5-dion, Tebutam, Tebuthiuron, Tecnazene, Tefuryltrione, Tembotrione, Tepraloxydim, Terbacil, Terbucarb, Terbuchlor, Terbumeton, Terbuthylazine, Terbutryn, Thenylchlor, Thiafluamide, Thiazafluron, Thiazopyr, Thidiazimin, Thidiazuron, Thiencarbazone, Thiencarbazone-methyl, Thifensulfuron, Thifensulfuron-methyl, Thiobencarb, Tiocarbazil, Topramezone, Tralkoxydim, Triallate, Triasulfuron, Triaziflam, Triazofenamide, Tribenuron, Tribenuron-methyl, Trichloressigsäure (TCA), Triclopyr, Tridiphane, Trietazine, Trifloxysulfuron, Trifloxysulfuron-natrium, Trifluralin, Triflusulfuron, Triflusulfuron-methyl, Trimeturon, Trinexapac, Trinexapac-ethyl, Tritosulfuron, Tsitodef, Uniconazole, Uniconazole-P, Vernolate, ZJ-0862, d. h. 3,4-Dichlor-N-{2-[(4,6-dimethoxypyrimidin-2-yl)oxy]benzyl}anilin, sowie die folgenden Verbindungen:

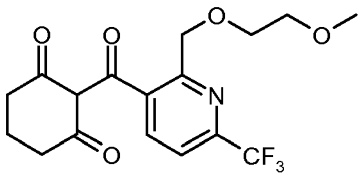
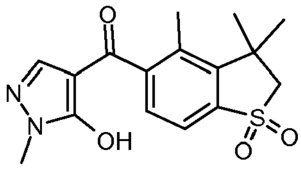
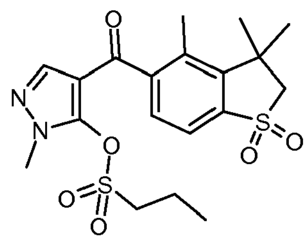
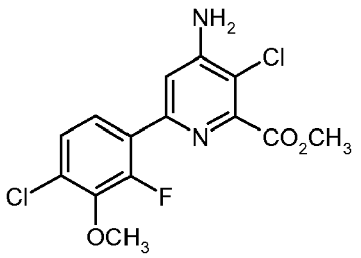
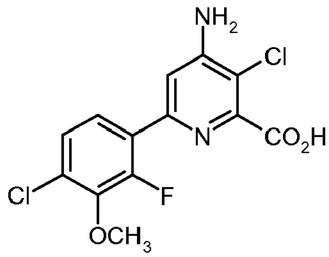
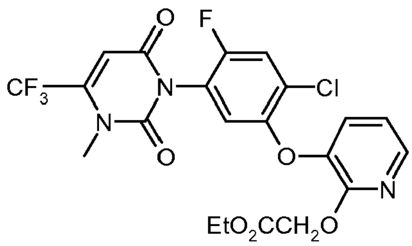

Die Erfindung soll durch die nachfolgenden biologischen Beispiele veranschaulicht werden, ohne sie jedoch darauf einzuschränken.

Biologische Beispiele:

Samen von mono- bzw. dikotylen Kulturpflanzen wurden in Holzfasertöpfen in sandigem Lehmboden ausgelegt, mit Erde abgedeckt und im Gewächshaus unter guten Wachstumsbedingungen angezogen. Die Behandlung der Versuchspflanzen erfolgte im frühen Laubblattstadium (BBCH10 - BBCH13). Zur Gewährleistung einer uniformen Wasserversorgung vor Stressbeginn wurden die bepflanzten Töpfe unmittelbar zuvor durch Anstaubewässerung maximal mit Wasser versorgt und nach Applikation in Plastikeinsätze transferiert, um anschließendes, zu schnelles Abtrocknen zu verhindern. Die in Form von benetzbaren Pulvern (WP), benetzbaren Granulaten (WG), Suspensionskonzentraten (SC) oder Emulsionskonzentraten (EC) formulierten erfindungsgemäßen Verbindungen wurden als wässrige Suspension mit einer Wasseraufwandmenge von umgerechnet 600 l/ha unter Zusatz von 0,2% Netzmittel (Agrotin) auf die grünen Pflanzenteile gesprüht. Unmittelbar nach Substanzapplikation erfolgt die Stressbehandlung der Pflanzen (Kälte- oder Trockenstress). Zur Kältestressbehandlung wurden die Pflanzen unter folgenden kontrollierten Bedingungen gehalten:

"Tag": 12 Stunden beleuchtet bei 8°C
"Nacht": 12 Stunden ohne Beleuchtung bei 1°C.

Der Trockenstreß wurde durch langsames Abtrocknen unter folgenden Bedingungen induziert:

"Tag": 14 Stunden beleuchtet bei 26°C
"Nacht": 10 Stunden ohne Beleuchtung bei 18°C.

Die Dauer der jeweiligen Streßphasen richtete sich hauptsächlich nach dem Zustand der unbehandelten, gestressten Kontrollpflanzen und variierte somit von Kultur zu Kultur. Sie wurde (durch Wiederbewässerung bzw. Transfer in Gewächshaus mit guten Wachstumsbedingungen) beendet, sobald irreversible Schäden an den unbehandelten, gestressten Kontrollpflanzen zu beobachten waren. Bei dikotylen Kulturen wie beispielsweise Raps und Soja variierte die Dauer der Trockenstreßphase zwischen 3 und 5 Tagen, bei monokotylen Kulturen wie beispielweise Weizen, Gerste oder Mais zwischen 6 und 10 Tagen. Die Dauer der Kältestreßphase variierte zwischen 12 und 14 Tagen.

Nach Beendigung der Stressphase folgte eine ca. 5-7 tägige Erholungsphase, während der die Pflanzen abermals unter guten Wachstumsbedingungen im Gewächshaus gehalten wurden. Um auszuschließen, daß die beobachteten Effekte von der ggf. fungiziden Wirkung der Testverbindungen beeinflußt werden, wurde zudem darauf geachtet, daß die Versuche ohne Pilzinfektion bzw. ohne Infektionsdruck ablaufen.

Nach Beendigung der Erholungsphase wurden die Schadintensitäten visuell im Vergleich zu unbehandelten, ungestressten Kontrollen gleichen Alters (bei Trockenstreß) bzw. gleichen Wuchsstadiums (bei Kältestreß) bonitiert. Die Erfassung der Schadintensität erfolgte zunächst prozentual (100% = Pflanzen sind abgestorben, 0 % = wie Kontrollpflanzen). Aus diesen Werten wurde sodann der Wirkungsgrad der Testverbindungen (= prozentuale Reduktion der Schadintensität durch Substanzapplikation) nach folgender Formel ermittelt:

$$WG = \frac{(SW_{ug} - SW_{bg}) \times 100}{SW_{ug}}$$

WG: Wirkungsgrad (%)
$SW_{ug}$: Schadwert der unbehandelten, gestressten Kontrolle
$SW_{bg}$: Schadwert der mit Testverbindung behandelten Pflanzen

In untenstehenden Tabellen sind jeweils Mittelwerte aus drei Ergebniswerten des gleichen Versuches aufgeführt.

Wirkungen ausgewählter Verbindungen der allgemeinen Formel (I) unter Trockenstress:

Tabelle A-1

| No. | Substanz | Dosierung | Einheit | WG (TRZAS) |
|---|---|---|---|---|
| 1 | 2-12 | 250 | g/ha | >5 |
| 2 | 2-27 | 250 | g/ha | >5 |
| 3 | 3-5 | 25 | g/ha | >5 |
| 4 | 3-6 | 250 | g/ha | >5 |
| 5 | 3-7 | 250 | g/ha | >5 |
| 6 | 3-10 | 25 | g/ha | >5 |

Tabelle A-2

| No. | Substanz | Dosierung | Einheit | WG (ZEAMX) |
|---|---|---|---|---|
| 1 | 3-4 | 25 | g/ha | >5 |
| 2 | 3-7 | 250 | g/ha | >5 |
| 3 | 3-10 | 25 | g/ha | >5 |
| 4 | 4-27 | 250 | g/ha | >5 |

Tabelle A-3

| No. | Substanz | Dosierung | Einheit | WG (BRSNS) |
|---|---|---|---|---|
| 1 | 3-4 | 25 | g/ha | >5 |
| 2 | 3-5 | 25 | g/ha | >5 |

(fortgesetzt)

| No. | Substanz | Dosierung | Einheit | WG (BRSNS) |
|---|---|---|---|---|
| 3 | 3-6 | 250 | g/ha | >5 |
| 4 | 3-12 | 250 | g/ha | >5 |

In den zuvor genannten Tabellen bedeuten:

BRSNS = *Brassica napus*
TRZAS = *Triticum aestivum*
ZEAMX = *Zea mays*

Ähnliche Ergebnisse konnten auch noch mit weiteren Verbindungen der allgemeinen Formel (I) auch bei Applikation auf andere Pflanzenarten erzielt werden.

**Patentansprüche**

1. Verwendung substituierter (2Z)-5(1-Hydroxycyclohexyl)pent-2-en-4-insäure-Derivate der allgemeinen Formel (I) oder deren Salze

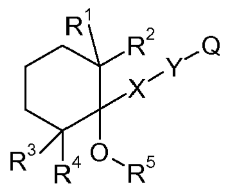

**(I)**

zur Toleranzerhöhung gegenüber abiotischem Stress in Pflanzen, wobei
[X-Y] für die Gruppierungen

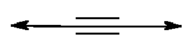
**[X-Y]$^1$** ,
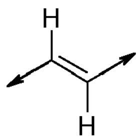
**[X-Y]$^2$** ,
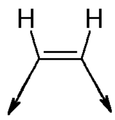
**[X-Y]$^3$** und
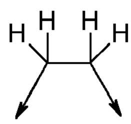
**[X-Y]$^4$** ,

steht,
Q für die Gruppierungen Q-1, Q-2, Q-3, Q-4 und Q-5

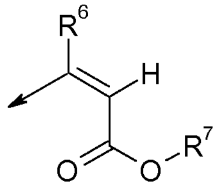 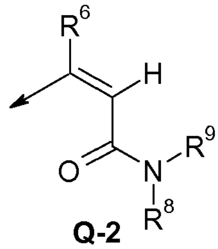 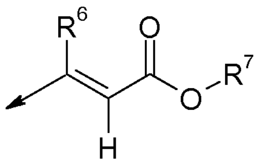 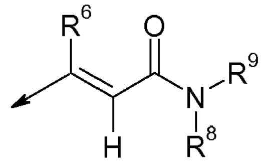

**Q-1** **Q-2** **Q-3** **Q-4**

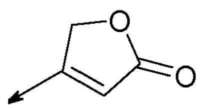

**Q-5**

und
steht

$R^1$ ($C_1$-$C_{20}$)-Alkyl, ($C_2$-$C_{20}$)-Alkenyl, ($C_2$-$C_{20}$)-Alkinyl, ($C_3$-$C_{20}$)-Cycloalkyl, ($C_1$-$C_{20}$)-Alkoxy, ($C_1$-$C_{20}$)-Alkylthio, ($C_1$-$C_{20}$)-Haloalkyl, ($C_2$-$C_{20}$)-Haloalkenyl, ($C_2$-$C_{20}$)-Haloalkinyl, ($C_1$-$C_{20}$)-Haloalkoxy, ($C_1$-$C_{20}$)-Haloalkylthio, ($C_3$-$C_{20}$)-Cycloalkyl, ($C_3$-$C_{20}$)-Cycloalkanyloxy, ($C_3$-$C_{20}$)-Cycloalkanylthio, ($C_3$-$C_{20}$)-Cycloalkenyl, ($C_3$-$C_{20}$)-Cycloalkenyloxy, ($C_3$-$C_{20}$)-Cycloalkenylthio, Phenyl, Heteroaryl, Heterocyclyl, wobei in den vorgenannten Gruppen das Heteroaryl ein aromatischer fünf-, sechs-oder siebengliedriger Heterocyclus und das das Heterocyclyl ein gesättigter oder teilweise ungesättigter fünf-, sechs- oder siebengliedriger Heterocyclus ist, die jeweils ein, zwei, drei oder vier Heteroatome aus der Gruppe O, N und S enthalten, oder $NR^aR^b$, wobei zwei Reste $R^1$, die an dasselbe Kohlenstoffatom gebunden sind, gemeinsam mit dem Kohlenstoffatom, an das sie gebunden sind, auch einen ($C_3$-$C_6$)-Cycloalkylring bilden können; wobei $NR^aR^b$ wie unten definiert sind; wobei jeder der letztgenannten drei Reste unabhängig voneinander unsubstituiert oder durch einen oder mehrere Reste aus der Gruppe Halogen, Hydroxy, ($C_1$-$C_4$)-Alkoxy und ($C_1$-$C_4$)-Alkylthio substituiert ist, bedeutet,

$R^2$ Wasserstoff, ($C_1$-$C_{20}$)-Alkyl, ($C_2$-$C_{20}$)-Alkenyl, ($C_2$-$C_{20}$)-Alkinyl, ($C_3$-$C_{20}$)-Cycloalkyl, ($C_1$-$C_{20}$)-Alkoxy, ($C_1$-$C_{20}$)-Alkylthio, ($C_1$-$C_{20}$)-Haloalkyl, ($C_2$-$C_{20}$)-Haloalkenyl, ($C_2$-$C_{20}$)-Haloalkinyl, ($C_1$-$C_{20}$)-Haloalkoxy, ($C_1$-$C_{20}$)-Haloalkylthio, ($C_3$-$C_{20}$)-Cycloalkanyl, ($C_3$-$C_{20}$)-Cycloalkanyloxy, ($C_3$-$C_{20}$)-Cycloalkanylthio, ($C_3$-$C_{20}$)-Cycloalkenyl, ($C_3$-$C_{20}$)-Cycloalkenyloxy, ($C_3$-$C_{20}$)-Cycloalkenylthio, Phenyl, Heteroaryl, Heterocyclyl, wobei in den vorgenannten Gruppen das Heteroaryl ein aromatischer fünf-, sechs-oder siebengliedriger Heterocyclus und das das Heterocyclyl ein gesättigter oder teilweise ungesättigter fünf-, sechs- oder siebengliedriger Heterocyclus ist, die jeweils ein, zwei, drei oder vier Heteroatome aus der Gruppe O, N und S enthalten, oder $NR^aR^b$, wobei zwei Reste $R^1$, die an dasselbe Kohlenstoffatom gebunden sind, gemeinsam mit dem Kohlenstoffatom, an das sie gebunden sind, auch einen ($C_3$-$C_6$)-Cycloalkylring bilden können; wobei $NR^aR^b$ wie unten definiert sind; wobei jeder der letztgenannten drei Reste unabhängig voneinander unsubstituiert oder durch einen oder mehrere Reste aus der Gruppe Halogen, Hydroxy, ($C_1$-$C_4$)-Alkoxy und ($C_1$-$C_4$)-Alkylthio substituiert ist, bedeutet,

$R^3$ Wasserstoff, ($C_1$-$C_{20}$)-Alkyl, ($C_2$-$C_{20}$)-Alkenyl, ($C_2$-$C_{20}$)-Alkinyl, ($C_3$-$C_{20}$)-Cycloalkyl, ($C_1$-$C_{20}$)-Alkoxy, ($C_1$-$C_{20}$)-Alkylthio, ($C_1$-$C_{20}$)-Haloalkyl, ($C_2$-$C_{20}$)-Haloalkenyl, ($C_2$-$C_{20}$)-Haloalkinyl, ($C_1$-$C_{20}$)-Haloalkoxy, ($C_1$-$C_{20}$)-Haloalkylthio, ($C_3$-$C_{20}$)-Cycloalkanyl, ($C_3$-$C_{20}$)-Cycloalkanyloxy, ($C_3$-$C_{20}$)-Cycloalkanylthio, ($C_3$-$C_{20}$)-Cycloalkenyl, ($C_3$-$C_{20}$)-Cycloalkenyloxy, ($C_3$-$C_{20}$)-Cycloalkenylthio, Phenyl, Heteroaryl, Heterocyclyl, wobei in den vorgenannten Gruppen das Heteroaryl ein aromatischer fünf-, sechs-oder siebengliedriger Heterocyclus und das das Heterocyclyl ein gesättigter oder teilweise ungesättigter fünf-, sechs- oder siebengliedriger Heterocyclus ist, die jeweils ein, zwei, drei oder vier Heteroatome aus der Gruppe O, N und S enthalten, oder $NR^aR^b$, wobei zwei Reste $R^1$, die an dasselbe Kohlenstoffatom gebunden sind, gemeinsam mit dem Kohlenstoffatom, an das sie gebunden sind, auch einen ($C_3$-$C_6$)-Cycloalkylring bilden können; wobei $NR^aR^b$ wie unten definiert sind; wobei jeder der letztgenannten drei Reste unabhängig voneinander unsubstituiert oder durch einen oder mehrere Reste aus der Gruppe Halogen, Hydroxy, ($C_1$-$C_4$)-Alkoxy und ($C_1$-$C_4$)-Alkylthio substituiert ist, bedeutet,

$R^4$ Wasserstoff, ($C_1$-$C_{20}$)-Alkyl, ($C_2$-$C_{20}$)-Alkenyl, ($C_2$-$C_{20}$)-Alkinyl, ($C_3$-$C_{20}$)-Cycloalkyl, ($C_1$-$C_{20}$)-Alkoxy, ($C_1$-$C_{20}$)-Alkylthio, ($C_1$-$C_{20}$)-Haloalkyl, ($C_2$-$C_{20}$)-Haloalkenyl, ($C_2$-$C_{20}$)-Haloalkinyl, ($C_1$-$C_{20}$)-Haloalkoxy,

$(C_1-C_{20})$-Haloalkylthio, $(C_3-C_{20})$-Cycloalkanyl, $(C_3-C_{20})$-Cycloalkanyloxy, $(C_3-C_{20})$-Cycloalkanylthio, $(C_3-C_{20})$-Cycloalkenyl, $(C_3-C_{20})$-Cycloalkenyloxy, $(C_3-C_{20})$-Cycloalkenylthio, Phenyl, Heteroaryl, Heterocyclyl, wobei in den vorgenannten Gruppen das Heteroaryl ein aromatischer fünf-, sechs-oder siebengliedriger Heterocyclus und das das Heterocyclyl ein gesättigter oder teilweise ungesättigter fünf-, sechs- oder siebengliedriger Heterocyclus ist, die jeweils ein, zwei, drei oder vier Heteroatome aus der Gruppe O, N und S enthalten, oder $NR^aR^b$, wobei zwei Reste $R^1$, die an dasselbe Kohlenstoffatom gebunden sind, gemeinsam mit dem Kohlenstoffatom, an das sie gebunden sind, auch einen $(C_3-C_6)$-Cycloalkylring bilden können; wobei jeder der Reste $R^a$, $R^b$, $R^c$, $R^d$, $R^e$, $R^f$ und $R^e$ inklusive Substituenten 1 bis 30 C-Atome, vorzugsweise 1 bis 24 C-Atome, insbesondere 1 bis 20 C-Atome aufweist, bedeutet, jeder der letztgenannten drei Reste unabhängig voneinander unsubstituiert oder durch einen oder mehrere Reste aus der Gruppe Halogen, Hydroxy, $(C_1-C_4)$-Alkoxy und $(C_1-C_4)$-Alkylthio substituiert ist, bedeutet,

$R^5$ Wasserstoff, $(C_1-C_{20})$-Alkyl, $(C_2-C_{20})$-Alkenyl, $(C_2-C_{20})$-Alkinyl, $(C_3-C_{20})$-Cycloalkyl, $(C_1-C_{20})$-Haloalkyl, $(C_2-C_{20})$-Haloalkenyl, $(C_2-C_{20})$-Haloalkinyl, $(C_3-C_{20})$-Cycloalkanyl, $(C_3-C_{20})$-Cycloalkenyl, wobei jeder der letztgenannten drei Reste unabhängig voneinander unsubstituiert oder durch einen oder mehrere Reste aus der Gruppe Halogen, CN, Hydroxy, $(C_1-C_4)$-Alkoxy und $(C_1-C_4)$-Alkylthio substituiert ist, bedeutet, oder einen Rest der Formel $SiR^aR^bR^c$, wobei in der letztgenannten Formel jeder der Reste $R^a$, $R^b$ und $R^c$ unabhängig voneinander Wasserstoff oder einen gegebenenfalls substituierten Kohlenwasserstoffrest bedeutet, der inklusive Substituenten 1 bis 30 C-Atome aufweist,

$R^6$ Wasserstoff, $(C_2-C_{20})$-Alkyl, $(C_2-C_{20})$-Alkenyl, $(C_2-C_{20})$-Alkinyl, $(C_3-C_{20})$-Cycloalkyl, $(C_1-C_{20})$-Haloalkyl, $(C_2-C_{20})$-Haloalkenyl, $(C_2-C_{20})$-Haloalkinyl, $(C_3-C_{20})$-Cycloalkanyl, $(C_3-C_{20})$-Cycloalkanyloxy, $(C_3-C_{20})$-Cycloalkanylthio, $(C_3-C_{20})$-Cycloalkenyl, wobei jeder der letztgenannten drei Reste unabhängig voneinander unsubstituiert oder durch einen oder mehrere Reste aus der Gruppe Halogen, Hydroxy, $(C_1-C_4)$-Alkoxy und $(C_1-C_4)$-Alkylthio substituiert ist, bedeutet,

$R^7$ Wasserstoff, $(C_1-C_{20})$-Alkyl, $(C_2-C_{20})$-Alkenyl, $(C_2-C_{20})$-Alkinyl, $(C_3-C_{20})$-Cycloalkyl, $(C_1-C_{20})$-Haloalkyl, $(C_2-C_{20})$-Haloalkenyl, $(C_2-C_{20})$-Haloalkinyl, $(C_3-C_{20})$-Cycloalkanyl, $(C_3-C_{20})$-Cycloalkenyl, $(C_3-C_{20})$-Cycloalkenyloxy, wobei jeder der letztgenannten drei Reste unabhängig voneinander unsubstituiert oder durch einen oder mehrere Reste aus der Gruppe Halogen, Hydroxy, $(C_1-C_4)$-Alkoxy und $(C_1-C_4)$-Alkylthio substituiert ist, bedeutet, oder einen Rest der Formel $SiR^aR^bR^c$, wobei in der letztgenannten Formel jeder der Reste $R^a$, $R^b$ und $R^c$ unabhängig voneinander Wasserstoff oder einen gegebenenfalls substituierten Kohlenwasserstoffrest bedeutet, der inklusive Substituenten 1 bis 30 C-Atome aufweist,

$R^8$ für Wasserstoff, $(C_1-C_{20})$-Alkyl, $(C_1-C_{20})$-Cycloalkyl, Halogen, $(C_2-C_{20})$-Alkenyl, $(C_2-C_{20})$-Alkinyl, $(C_1-C_{20})$-Haloalkyl, $(C_1-C_{20})$-Cyanoalkyl, $(C_3-C_{20})$Cycloalkylalkyl, Aryl-$(C_1-C_{20})$-alkyl, Heteroaryl-$(C_1-C_{20})$-alkyl, $(C_1-C_{20})$-Alkylcarbonyl, $(C_1-C_{20})$-Alkoxycarbonyl, $(C_1-C_{20})$-Alkenyloxycarbonyl, $(C_2-C_{20})$-Alkenylalkyloxycarbonyl, Aryl-$(C_1-C_{20})$-alkyloxycarbonyl, $(C_3-C_{20})$-Cycloalkoxycarbonyl, $(C_3-C_{20})$-Cycloalkyloxycarbonyl, $(C_1-C_{20})$-Alkylsulfonyl, Arylsulfonyl, $(C_3-C_{20})$-Cycloalkylsulfonyl, $(C_1-C_{20})$-Alkylsulfinyl, Arylsulfinyl, $(C_3-C_{20})$-Cycloalkylsulfinyl, $(C_1-C_{20})$-Alkoxycarbonyl-$(C_1-C_{20})$-alkyl, Hydroxycarbonyl-$(C_1-C_{20})$-alkyl, Arylalkoxycarbonyl-$(C_1-C_{20})$-alkyl, $(C_3-C_{20})$Cycloalkylalkoxycarbonyl-$(C_1-C_{20})$-alkyl, Alkoxycarbonyl-$(C_3-C_{20})$-cycloalkyl, Hydroxycarbonyl-$(C_3-C_{20})$-cycloalkyl, $(C_1-C_{20})$-Alkoxycarbonyl-$(C_3-C_{20})$-cycloalkenyl, Hydroxycarbonyl-$(C_3-C_{20})$-cycloalkenyl, Bis-$(C_1-C_{20})$-Alkylamino-$(C_1-C_{20})$-alkyl steht,

$R^9$ für Wasserstoff, $(C_1-C_{20})$-Alkyl, $(C_3-C_{20})$-Cycloalkyl, Halogen, $(C_1-C_{20})$-Halogenalkyl, $(C_2-C_{20})$-Alkinyl, $(C_2-C_{20})$-Alkenyl, $(C_1-C_{20})$-Cyanoalkyl, Aryl-$(C_1-C_{20})$-alkyl, Heteroaryl-$(C_1-C_{20})$-alkyl, $(C_1-C_{20})$-Alkylcarbonyl, $(C_1-C_{20})$-Alkoxycarbonyl, $(C_1-C_{20})$-Alkylsulfonyl, Arylsulfonyl, $(C_3-C_{20})$-Cycloalkylsulfonyl, $(C_1-C_{20})$-Alkylsulfinyl, Arylsulfinyl, $(C_3-C_{20})$Cycloalkylsulfinyl, $(C_1-C_{20})$-Alkoxycarbonyl-$(C_1-C_{20})$-alkyl steht,

oder

$R^8$ und $R^9$ zusammen Bestandteil einer gegebenenfalls substituierten Sulfilimin- oder Amidingruppe sind oder ein Iminophosphoran bilden.

**2.** Verwendung gemäß Anspruch 1, wobei in Formel (I)
[X-Y] für die Gruppierungen

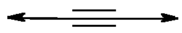

[X-Y]1 ,

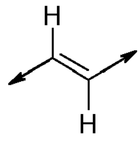

[X-Y]2 und

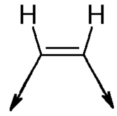

[X-Y]3

steht,
Q für die Gruppierungen Q-1, Q-2, Q-3 und Q-5 steht

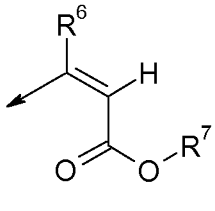

Q-1

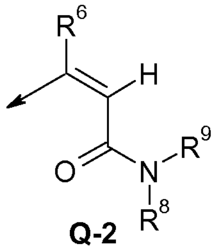

Q-2

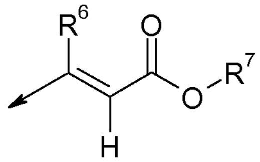

Q-3 und

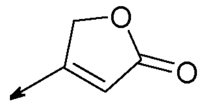

Q-5

$R^1$ $(C_1\text{-}C_{10})$-Alkyl, $(C_2\text{-}C_{10})$-Alkenyl, $(C_2\text{-}C_{10})$-Alkinyl, $(C_3\text{-}C_{10})$-Cycloalkyl, $(C_1\text{-}C_{10})$-Alkoxy, $(C_1\text{-}C_{10})$-Alkylthio, $(C_1\text{-}C_{10})$-Haloalkyl, $(C_1\text{-}C_{10})$-Haloalkenyl, $(C_2\text{-}C_{10})$-Haloalkinyl, $(C_1\text{-}C_{10})$-Haloalkoxy, $(C_1\text{-}C_{10})$-Haloalkylthio, $(C_3\text{-}C_{10})$-Cycloalkyl, $(C_3\text{-}C_{10})$-Cycloalkanyloxy, $(C_3\text{-}C_{10})$-Cycloalkanylthio, $(C_3\text{-}C_{10})$-Cycloalkenyl, $(C_3\text{-}C_{10})$-Cycloalkenyloxy, $(C_3\text{-}C_{10})$-Cycloalkenylthio, Phenyl, Heteroaryl, Heterocyclyl, wobei in den vorgenannten Gruppen das Heteroaryl ein aromatischer fünf-, sechs-oder siebengliedriger Heterocyclus und das das Heterocyclyl ein gesättigter oder teilweise ungesättigter fünf-, sechs- oder siebengliedriger Heterocyclus ist, die jeweils ein, zwei, drei oder vier Heteroatome aus der Gruppe O, N und S enthalten, oder $NR^aR^b$, wobei zwei Reste $R^1$, die an dasselbe Kohlenstoffatom gebunden sind, gemeinsam mit dem Kohlenstoffatom, an das sie gebunden sind, auch einen $(C_3\text{-}C_6)$-Cycloalkylring bilden können; wobei $NR^aR^b$ wie unten definiert sind; wobei jeder der letztgenannten drei Reste unabhängig voneinander unsubstituiert oder durch einen oder mehrere Reste aus der Gruppe Halogen, Hydroxy, $(C_1\text{-}C_4)$-Alkoxy und $(C_1\text{-}C_4)$-Alkylthio substituiert ist, bedeutet,

$R^2$ Wasserstoff, $(C_1\text{-}C_{10})$-Alkyl, $(C_2\text{-}C_{10})$-Alkenyl, $(C_2\text{-}C_{10})$-Alkinyl, $(C_3\text{-}C_{10})$-Cycloalkyl, $(C_1\text{-}C_{10})$-Alkoxy, $(C_1\text{-}C_{10})$-Alkylthio, $(C_1\text{-}C_{10})$-Haloalkyl, $(C_2\text{-}C_{10})$-Haloalkenyl, $(C_2\text{-}C_{10})$-Haloalkinyl, $(C_1\text{-}C_{10})$-Haloalkoxy, $(C_1\text{-}C_{10})$-Haloalkylthio, $(C_3\text{-}C_{10})$-Cycloalkanyloxy, $(C_3\text{-}C_{10})$-Cycloalkanylthio, $(C_3\text{-}C_{10})$-Cycloalkenyl, $(C_3\text{-}C_{10})$-Cycloalkenyloxy, $(C_3\text{-}C_{10})$-Cycloalkenylthio, Phenyl, Heteroaryl, Heterocyclyl, wobei in den vorgenannten Gruppen das Heteroaryl ein aromatischer fünf-, sechs-oder siebengliedriger Heterocyclus und das das Heterocyclyl ein gesättigter oder teilweise ungesättigter fünf-, sechs- oder siebengliedriger Heterocyclus ist, die jeweils ein, zwei, drei oder vier Heteroatome aus der Gruppe O, N und S enthalten, oder $NR^aR^b$, wobei zwei Reste $R^1$, die an dasselbe Kohlenstoffatom gebunden sind, gemeinsam mit dem Kohlenstoffatom, an das sie gebunden sind, auch einen $(C_3\text{-}C_6)$-Cycloalkylring bilden können; wobei $NR^aR^b$ wie unten definiert sind; wobei jeder der letztgenannten drei Reste unabhängig voneinander unsubstituiert oder durch einen oder mehrere Reste aus der Gruppe Halogen, Hydroxy, $(C_1\text{-}C_4)$-Alkoxy und $(C_1\text{-}C_4)$-Alkylthio substituiert ist, bedeutet,

$R^3$ Wasserstoff, $(C_1\text{-}C_{10})$-Alkyl, $(C_2\text{-}C_{10})$-Alkenyl, $(C_2\text{-}C_{10})$-Alkinyl, $(C_3\text{-}C_{10})$-Cycloalkyl, $(C_1\text{-}C_{10})$-Alkoxy, $(C_1\text{-}C_{10})$-Alkylthio, $(C_1\text{-}C_{10})$-Haloalkyl, $(C_2\text{-}C_{10})$-Haloalkenyl, $(C_2\text{-}C_{10})$-Haloalkinyl, $(C_1\text{-}C_{10})$-Haloalkoxy, $(C_1\text{-}C_{10})$-Haloalkylthio, $(C_3\text{-}C_{10})$-Cycloalkanyloxy, $(C_3\text{-}C_{10})$-Cycloalkanylthio, $(C_3\text{-}C_{10})$-Cycloalkenyl, $(C_3\text{-}C_{10})$-Cycloalkenyloxy, $(C_3\text{-}C_{10})$-Cycloalkenylthio, Phenyl, Heteroaryl, Heterocyclyl, wobei in den vorgenannten Gruppen das Heteroaryl ein aromatischer fünf-, sechs-oder siebengliedriger Heterocyclus und das das Heterocyclyl ein gesättigter oder teilweise ungesättigter fünf-, sechs- oder siebengliedriger Heterocyclus ist, die jeweils ein, zwei, drei oder vier Heteroatome aus der Gruppe O, N und S enthalten, oder $NR^aR^b$, wobei zwei Reste $R^1$, die an dasselbe Kohlenstoffatom gebunden sind, gemeinsam mit dem Kohlenstoffatom, an das sie gebunden sind, auch einen $(C_3\text{-}C_6)$-Cycloalkylring bilden können; wobei $NR^aR^b$ wie unten definiert sind; wobei jeder der letztgenannten drei Reste unabhängig voneinander unsubstituiert oder durch einen oder mehrere Reste aus der Gruppe Halogen, Hydroxy, $(C_1\text{-}C_4)$-Alkoxy und $(C_1\text{-}C_4)$-Alkylthio substituiert ist, bedeutet,

$R^4$ Wasserstoff, $(C_1\text{-}C_{10})$-Alkyl, $(C_2\text{-}C_{10})$-Alkenyl, $(C_2\text{-}C_{10})$-Alkinyl, $(C_3\text{-}C_{10})$-Cycloalkyl, $(C_1\text{-}C_{10})$-Alkoxy, $(C_1\text{-}C_{10})$-Alkylthio, $(C_1\text{-}C_{10})$-Haloalkyl, $(C_2\text{-}C_{10})$-Haloalkenyl, $(C_2\text{-}C_{10})$-Haloalkinyl, $(C_1\text{-}C_{10})$-Haloalkoxy, $(C_1\text{-}C_{10})$-Haloalkylthio, $(C_3\text{-}C_{10})$-Cycloalkanyloxy, $(C_3\text{-}C_{10})$-Cycloalkanylthio, $(C_3\text{-}C_{10})$-Cycloalkenyl,

$(C_3-C_{10})$-Cycloalkenyloxy, $(C_3-C_{10})$-Cycloalkenylthio, Phenyl, Heteroaryl, Heterocyclyl, wobei in den vorgenannten Gruppen das Heteroaryl ein aromatischer fünf-, sechs-oder siebengliedriger Heterocyclus und das das Heterocyclyl ein gesättigter oder teilweise ungesättigter fünf-, sechs- oder siebengliedriger Heterocyclus ist, die jeweils ein, zwei, drei oder vier Heteroatome aus der Gruppe O, N und S enthalten, oder $NR^aR^b$, wobei zwei Reste $R^1$, die an dasselbe Kohlenstoffatom gebunden sind, gemeinsam mit dem Kohlenstoffatom, an das sie gebunden sind, auch einen $(C_3-C_6)$-Cycloalkylring bilden können; wobei jeder der Reste $R^a$, $R^b$, $R^c$, $R^d$, $R^e$, $R^f$ und $R^e$ inklusive Substituenten 1 bis 30 C-Atome aufweist, bedeutet, und jeder der letztgenannten drei Reste unabhängig voneinander unsubstituiert oder durch einen oder mehrere Reste aus der Gruppe Halogen, Hydroxy, $(C_1-C_4)$-Alkoxy und $(C_1-C_4)$-Alkylthio substituiert ist, bedeutet,

$R^5$ Wasserstoff, $(C_1-C_{10})$-Alkyl, $(C_2-C_{10})$-Alkenyl, $(C_2-C_{10})$-Alkinyl, $(C_3-C_{10})$-Cycloalkyl, $(C_1-C_{10})$-Haloalkyl, $(C_2-C_{10})$-Haloalkenyl, $(C_2-C_{10})$-Haloalkinyl, $(C_3-C_{10})$-Cycloalkenyl, wobei jeder der letztgenannten drei Reste unabhängig voneinander unsubstituiert oder durch einen oder mehrere Reste aus der Gruppe Halogen, CN, Hydroxy, $(C_1-C_4)$-Alkoxy und $(C_1-C_4)$-Alkylthio substituiert ist, bedeutet, oder einen Rest der Formel $SiR^aR^bR^c$, wobei in der letztgenannten Formel jeder der Reste $R^a$, $R^b$ und $R^c$ unabhängig voneinander Wasserstoff oder einen gegebenenfalls substituierten Kohlenwasserstoffrest bedeutet, der inklusive Substituenten 1 bis 30 C-Atome aufweist,

$R^6$ Wasserstoff, $(C_2-C_{10})$-Alkyl, $(C_2-C_{10})$-Alkenyl, $(C_2-C_{10})$-Alkinyl, $(C_3-C_{10})$-Cycloalkyl, $(C_1-C_{10})$-Haloalkyl, $(C_2-C_{10})$-Haloalkenyl, $(C_2-C_{10})$-Haloalkinyl, $(C_3-C_{10})$-Cycloalkanyloxy, $(C_3-C_{10})$-Cycloalkanylthio, $(C_3-C_{10})$-Cycloalkenyl, wobei jeder der letztgenannten drei Reste unabhängig voneinander unsubstituiert oder durch einen oder mehrere Reste aus der Gruppe Halogen, Hydroxy, $(C_1-C_4)$-Alkoxy und $(C_1-C_4)$-Alkylthio substituiert ist, bedeutet,

$R^7$ Wasserstoff, $(C_1-C_{10})$-Alkyl, $(C_2-C_{10})$-Alkenyl, $(C_2-C_{10})$-Alkinyl, $(C_3-C_{10})$-Cycloalkyl, $(C_1-C_{10})$-Haloalkyl, $(C_2-C_{10})$-Haloalkenyl, $(C_2-C_{10})$-Haloalkinyl, $(C_3-C_{10})$-Cycloalkenyl, $(C_3-C_{10})$-Cycloalkenyloxy, wobei jeder der letztgenannten drei Reste unabhängig voneinander unsubstituiert oder durch einen oder mehrere Reste aus der Gruppe Halogen, Hydroxy, $(C_1-C_4)$-Alkoxy und $(C_1-C_4)$-Alkylthio substituiert ist, bedeutet, oder einen Rest der Formel $SiR^aR^bR^c$, wobei in der letztgenannten Formel jeder der Reste $R^a$, $R^b$ und $R^c$ unabhängig voneinander Wasserstoff oder einen gegebenenfalls substituierten Kohlenwasserstoffrest bedeutet, der inklusive Substituenten 1 bis 30 C-Atome, aufweist,

$R^8$ für Wasserstoff, $(C_1-C_{10})$-Alkyl, $(C_3-C_{10})$-Cycloalkyl, Halogen, $(C_2-C_{10})$-Alkenyl, $(C_2-C_{10})$-Alkinyl, $(C_1-C_{10})$-Haloalkyl, $(C_1-C_{10})$-Cyanoalkyl, $(C_3-C_{10})$Cycloalkylalkyl, Aryl-$(C_1-C_{10})$-alkyl, Heteroaryl-$(C_1-C_{10})$-alkyl, $(C_1-C_{10})$-Alkylcarbonyl, $(C_1-C_{10})$-Alkoxycarbonyl, $(C_1-C_{10}$-Alkenyloxycarbonyl, $(C_2-C_{10})$-Alkenylalkyloxycarbonyl, Aryl-$(C_1-C_{10})$-alkyloxycarbonyl, $(C_3-C_{10})$-Cycloalkoxycarbonyl, $(C_3-C_{10})$-Cycloalkyloxycarbonyl, $(C_1-C_{10})$-Alkylsulfonyl, Arylsulfonyl, $(C_3-C_{10})$-Cycloalkylsulfonyl, $(C_1-C_{10})$-Alkylsulfinyl, Arylsulfinyl, $(C_3-C_{10})$-Cycloalkylsulfinyl, $(C_1-C_{10})$-Alkoxycarbonyl-$(C_1-C_{10})$-alkyl, Hydroxycarbonyl-$(C_1-C_{10})$-alkyl, Arylalkoxycarbonyl-$(C_1-C_{10})$-alkyl, $(C_3-C_{10})$Cycloalkylalkoxycarbonyl-$(C_1-C_{10})$-alkyl, Alkoxycarbonyl-$(C_3-C_{10})$-cycloalkyl, Hydroxycarbonyl-$(C_3-C_{10})$-cycloalkyl, $(C_1-C_{10})$-Alkoxycarbonyl-$(C_3-C_{10})$-cycloalkenyl, Hydroxycarbonyl-$(C_3-C_{10})$-cycloalkenyl, Bis-$(C_1$-C10)-Alkylamino-$(C_1-C_{10})$-alkyl steht, bedeutet,

$R^9$ für Wasserstoff, $(C_1-C_{10})$-Alkyl, $(C_3-C_{10})$-Cycloalkyl, Halogen, $(C_1-C_{10})$-Halogenalkyl, $(C_2-C_{10})$-Alkinyl, $(C_2-C_{10})$-Alkenyl, $(C_1-C_{10})$-Cyanoalkyl, Aryl-$(C_1-C_{10})$-alkyl, Heteroaryl-$(C_1-C_{10})$-alkyl, $(C_1-C_{10})$-Alkylcarbonyl, $(C_1-C_{10})$-Alkoxycarbonyl, $(C_1-C_{10})$-Alkylsulfonyl, Arylsulfonyl, $(C_3-C_{10})$-Cycloalkylsulfonyl, $(C_1-C_{10})$-Alkylsulfinyl, Arylsulfinyl, $(C_3-C_{10})$Cycloalkylsulfinyl, $(C_1-C_{10})$-Alkoxycarbonyl-$(C_1-C_{10})$-alkyl steht, oder

$R^8$ und $R^9$ zusammen Bestandteil einer gegebenenfalls substituierten Sulfilimin- oder Amidingruppe sind oder ein Iminophosphoran bilden.

**3.** Verwendung gemäß Anspruch 1, wobei in Formel (I) [X-Y] für die Gruppierung

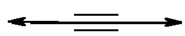

**[X-Y]¹**

steht,
Q für die Gruppierungen Q-1 steht

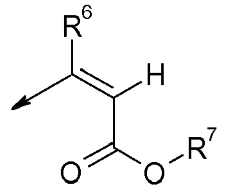

**Q-1**

$R^1$ $(C_1-C_6)$-Alkyl, $(C_2-C_6)$-Alkenyl, $(C_2-C_6)$-Alkinyl, $(C_3-C_6)$-Cycloalkyl, $(C_1-C_6)$-Alkoxy, $(C_1-C_6)$-Alkylthio, $(C_1-C_6)$-Haloalkyl, $(C_2-C_6)$-Haloalkenyl, $(C_2-C_6)$-Haloalkinyl, $(C_1-C_6)$-Haloalkoxy, $(C_1-C_6)$-Haloalkylthio, $(C_3-C_6)$-Cycloalkyl, $(C_3-C_6)$-Cycloalkanyloxy, $(C_3-C_6)$-Cycloalkanylthio, $(C_3-C_6)$-Cycloalkenyl, $(C_3-C_6)$-Cycloalkenyloxy, $(C_3-C_6)$-Cycloalkenylthio, Phenyl, Heteroaryl, Heterocyclyl, wobei in den vorgenannten Gruppen das Heteroaryl ein aromatischer fünf-, sechs- oder siebengliedriger Heterocyclus und das das Heterocyclyl ein gesättigter oder teilweise ungesättigter fünf-, sechs- oder siebengliedriger Heterocyclus ist, die jeweils ein, zwei, drei oder vier Heteroatome aus der Gruppe O, N und S enthalten, oder $NR^aR^b$, wobei zwei Reste $R^1$, die an dasselbe Kohlenstoffatom gebunden sind, gemeinsam mit dem Kohlenstoffatom, an das sie gebunden sind, auch einen $(C_3-C_6)$-Cycloalkylring bilden können; wobei $NR^aR^b$ wie unten definiert sind; wobei jeder der letztgenannten drei Reste unabhängig voneinander unsubstituiert oder durch einen oder mehrere Reste aus der Gruppe Halogen, Hydroxy, $(C_1-C_4)$-Alkoxy und $(C_1-C_4)$-Alkylthio substituiert ist, bedeutet,

$R^2$ Wasserstoff, $(C_1-C_6)$-Alkyl, $(C_2-C_6)$-Alkenyl, $(C_2-C_6)$-Alkinyl, $(C_3-C_6)$-Cycloalkyl, $(C_1-C_6)$-Alkoxy, $(C_1-C_6)$-Alkylthio, $(C_1-C_6)$-Haloalkyl, $(C_2-C_6)$-Haloalkenyl, $(C_2-C_6)$-Haloalkinyl, $(C_1-C_6)$-Haloalkoxy, $(C_1-C_6)$-Haloalkylthio, $(C_3-C_6)$-Cycloalkanyloxy, $(C_3-C_6)$-Cycloalkanylthio, $(C_3-C_6)$-Cycloalkenyl, $(C_3-C_6)$-Cycloalkenyloxy, $(C_3-C_6)$-Cycloalkenylthio, Phenyl, Heteroaryl, Heterocyclyl, wobei in den vorgenannten Gruppen das Heteroaryl ein aromatischer fünf-, sechs- oder siebengliedriger Heterocyclus und das das Heterocyclyl ein gesättigter oder teilweise ungesättigter fünf-, sechs- oder siebengliedriger Heterocyclus ist, die jeweils ein, zwei, drei oder vier Heteroatome aus der Gruppe O, N und S enthalten, oder $NR^aR^b$, wobei zwei Reste $R^1$, die an dasselbe Kohlenstoffatom gebunden sind, gemeinsam mit dem Kohlenstoffatom, an das sie gebunden sind, auch einen $(C_3-C_6)$-Cycloalkylring bilden können; wobei $NR^aR^b$ wie unten definiert sind; wobei jeder der letztgenannten drei Reste unabhängig voneinander unsubstituiert oder durch einen oder mehrere Reste aus der Gruppe Halogen, Hydroxy, $(C_1-C_4)$-Alkoxy und $(C_1-C_4)$-Alkylthio substituiert ist, bedeutet,

$R^3$ Wasserstoff, $(C_1-C_6)$-Alkyl, $(C_2-C_6)$-Alkenyl, $(C_2-C_6)$-Alkinyl, $(C_3-C_6)$-Cycloalkyl, $(C_1-C_6)$-Alkoxy, $(C_1-C_6)$-Alkylthio, $(C_1-C_6)$-Haloalkyl, $(C_2-C_6)$-Haloalkenyl, $(C_2-C_{10})$-Haloalkinyl, $(C_1-C_6)$-Haloalkoxy, $(C_1-C_6)$-Haloalkylthio, $(C_3-C_6)$-Cycloalkanyloxy, $(C_3-C_6)$-Cycloalkanylthio, $(C_3-C_6)$-Cycloalkenyl, $(C_3-C_6)$-Cycloalkenyloxy, $(C_3-C_6)$-Cycloalkenylthio, Phenyl, Heteroaryl, Heterocyclyl, wobei in den vorgenannten Gruppen das Heteroaryl ein aromatischer fünf-, sechs- oder siebengliedriger Heterocyclus und das das Heterocyclyl ein gesättigter oder teilweise ungesättigter fünf-, sechs- oder siebengliedriger Heterocyclus ist, die jeweils ein, zwei, drei oder vier Heteroatome aus der Gruppe O, N und S enthalten, oder $NR^aR^b$, wobei zwei Reste $R^1$, die an dasselbe Kohlenstoffatom gebunden sind, gemeinsam mit dem Kohlenstoffatom, an das sie gebunden sind, auch einen $(C_3-C_6)$-Cycloalkylring bilden können; wobei $NR^aR^b$ wie unten definiert sind; wobei jeder der letztgenannten drei Reste unabhängig voneinander unsubstituiert oder durch einen oder mehrere Reste aus der Gruppe Halogen, Hydroxy, $(C_1-C_4)$-Alkoxy und $(C_1-C_4)$-Alkylthio substituiert ist, bedeutet,

$R^4$ Wasserstoff, $(C_1-C_6)$-Alkyl, $(C_2-C_6)$-Alkenyl, $(C_2-C_6)$-Alkinyl, $(C_3-C_6)$-Cycloalkyl, $(C_1-C_6)$-Alkoxy, $(C_1-C_6)$-Alkylthio, $(C_1-C_6)$-Haloalkyl, $(C_1-C_6)$-Haloalkenyl, $(C_2-C_6)$-Haloalkinyl, $(C_1-C_6)$-Haloalkoxy, $(C_1-C_6)$-Haloalkylthio, $(C_3-C_6)$-Cycloalkanyloxy, $(C_3-C_6)$-Cycloalkanylthio, $(C_3-C_6)$-Cycloalkenyl, $(C_3-C_6)$-Cycloalkenyloxy, $(C_3-C_6)$-Cycloalkenylthio, Phenyl, Heteroaryl, Heterocyclyl, wobei in den vorgenannten Gruppen das Heteroaryl ein aromatischer fünf-, sechs- oder siebengliedriger Heterocyclus und das das Heterocyclyl ein gesättigter oder teilweise ungesättigter fünf-, sechs- oder siebengliedriger Heterocyclus ist, die jeweils ein, zwei, drei oder vier Heteroatome aus der Gruppe O, N und S enthalten, oder $NR^aR^b$, oder wobei zwei Reste $R^1$, die an dasselbe Kohlenstoffatom gebunden sind, gemeinsam mit dem Kohlenstoffatom, an das sie gebunden sind einen $(C_3-C_6)$-Cycloalkylring bilden; wobei jeder der Reste inklusive Substituenten 1 bis 30 C-Atome, aufweist, und jeder der letztgenannten drei Reste unabhängig voneinander unsubstituiert oder durch einen oder mehrere Reste aus der Gruppe Halogen, Hydroxy, $(C_1-C_4)$-Alkoxy und $(C_1-C_4)$-Alkylthio substituiert ist,

$R^5$ Wasserstoff, $(C_1-C_6)$-Alkyl, $(C_2-C_6)$-Alkenyl, $(C_2-C_6)$-Alkinyl, $(C_3-C_6)$-Cycloalkyl, $(C_1-C_6)$-Haloalkyl, $(C_2-C_6)$-Haloalkenyl, $(C_2-C_6)$-Haloalkinyl, $(C_3-C_6)$-Cycloalkenyl, wobei jeder der letztgenannten drei Reste unabhängig voneinander unsubstituiert oder durch einen oder mehrere Reste aus der Gruppe Halogen, CN, Hydroxy, $(C_1-C_4)$-Alkoxy und $(C_1-C_4)$-Alkylthio substituiert ist, bedeutet, oder einen Rest der Formel $SiR^aR^bR^c$,

wobei in der letztgenannten Formel jeder der Reste $R^a$, $R^b$ und $R^c$ unabhängig voneinander Wasserstoff oder einen gegebenenfalls substituierten Kohlenwasserstoffrest bedeutet, inklusive Substituenten 1 bis 30 C-Atome aufweist,

$R^6$ Wasserstoff, $(C_2$-$C_8)$-Alkyl, $(C_2$-$C_8)$-Alkenyl, $(C_2$-$C_8)$-Alkinyl, $(C_3$-$C_8)$-Cycloalkyl, $(C_1$-$C_8)$-Haloalkyl, $(C_2$-$C_8)$-Haloalkenyl, $(C_2$-$C_8)$-Haloalkinyl, $(C_3$-$C_8)$-Cycloalkanyloxy, $(C_3$-$C_8)$-Cycloalkanylthio, $(C_3$-$C_8)$-Cycloalkenyl, wobei jeder der letztgenannten drei Reste unabhängig voneinander unsubstituiert oder durch einen oder mehrere Reste aus der Gruppe Halogen, Hydroxy, $(C_1$-$C_4)$-Alkoxy und $(C_1$-$C_4)$-Alkylthio substituiert ist, bedeutet,

$R^7$ Wasserstoff, $(C_1$-$C_7)$-Alkyl, $(C_2$-$C_7)$-Alkenyl, $(C_2$-$C_7)$-Alkinyl, $(C_3$-$C_7)$-Cycloalkyl, $(C_1$-$C_7)$-Haloalkyl, $(C_2$-$C_7)$-Haloalkenyl, $(C_2$-$C_7)$-Haloalkinyl, $(C_3$-$C_7)$-Cycloalkenyl, $(C_3$-$C_7)$-Cycloalkenyloxy, wobei jeder der letztgenannten drei Reste unabhängig voneinander unsubstituiert oder durch einen oder mehrere Reste aus der Gruppe Halogen, Hydroxy, $(C_1$-$C_4)$-Alkoxy und $(C_1$-$C_4)$-Alkylthio substituiert ist, bedeutet, oder einen Rest der Formel $SiR^aR^bR^c$, wobei in der letztgenannten Formel jeder der Reste $R^a$, $R^b$ und $R^c$ unabhängig voneinander Wasserstoff oder einen gegebenenfalls substituierten Kohlenwasserstoffrest, der inklusive Substituenten 1 bis 30 C-Atome aufweist, bedeutet,

**4.** Behandlung von Pflanzen, umfassend die Applikation einer zur Steigerung der Widerstandsfähigkeit von Pflanzen gegenüber abiotischen Stressfaktoren wirksamen, nicht-toxischen Menge einer oder mehrere der Verbindungen der Formel (I), oder jeweils deren Salze gemäß einem der Ansprüche 1 bis 3.

**5.** Behandlung gemäß Anspruch 4, wobei die abiotischen Streßbedingungen einer oder mehrer Bedingungen ausgewählt aus der Gruppe von Dürre, Kälte- und Hitzebedingungen, Trockenstress, osmotischem Streß, Staunässe, erhöhtem Bodensalzgehalt, erhöhtem Ausgesetztsein an Mineralien, Ozonbedingungen, Starklichtbedingungen, beschränkter Verfügbarkeit von Stickstoffnährstoffen, beschränkter Verfügbarkeit von Phosphornährstoffen entsprechen.

**6.** Verwendung einer oder mehrere der Verbindungen der Formel (I), oder deren Salze gemäß einem der Ansprüche 1 bis 3 in der Sprühapplikation auf Pflanzen und Pflanzenteilen in Kombinationen mit einem oder mehrer Wirkstoffen ausgewählt aud der Gruppe der Insektizide, Lockstoffe, Akarizide, Fungizide, Nematizide, Herbizide, wachstumsregulatorische Stoffe, Safener, die Pflanzenreife beeinflussende Stoffe und Bakterizide.

**7.** Verwendung einer oder mehrere der Verbindungen der Formel (I), oder deren Salze gemäß einem der Ansprüche 1 bis 3 in der Sprühapplikation auf Pflanzen und Pflanzenteilen in Kombinationen mit Düngemitteln.

**8.** Verwendung einer oder mehrere der Verbindungen der Formel (I), oder deren Salze gemäß einem der Ansprüche 1 bis 3 zur Applikation auf gentechnisch veränderten Sorten, deren Saatgut, oder auf Anbauflächen auf denen diese Sorten wachsen.

**9.** Verwendung von Sprühlösungen, die eine oder mehrere der Verbindungen der Formel (I), oder jeweils deren Salze gemäß einem der Ansprüche 1 bis 3 enthalten, zur Steigerung der Widerstandsfähigkeit von Pflanzen gegenüber abiotischen Stressfaktoren.

**10.** Verfahren zur Erhöhung der Stresstoleranz bei Pflanzen ausgewählt aus der Gruppe der Nutzpflanzen, Zierpflanzen, Rasenarten, oder Bäumen, welches die Applikation einer ausreichenden, nicht-toxischen Menge einer oder mehrere der Verbindungen der Formel (I), oder deren Salze gemäß einem der Ansprüche 1 bis 3 auf die Fläche, wo die entsprechende Wirkung gewünscht wird, umfassend die Anwendung auf die Pflanzen, deren Saatgut oder auf die Fläche auf der die Pflanzen wachsen.

**11.** Verfahren gemäß Anspruch 10, wobei die Widerstandsfähigkeit der so behandelten Pflanzen gegenüber abiotischem Stress gegenüber nicht behandelten Pflanzen unter ansonsten gleichen physiologischen Bedingungen um mindestens 3% erhöht ist.

**12.** (2Z)-5(1-Hydroxycyclohexyl)pent-2-en-4-insäure-Derivate der allgemeinen Formel (I), oder deren Salze

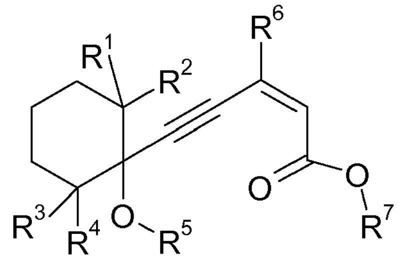

(I)

worin

$R^1$ $(C_1\text{-}C_6)$-Alkyl, $(C_2\text{-}C_6)$-Alkenyl, $(C_2\text{-}C_6)$-Alkinyl, $(C_3\text{-}C_6)$-Cycloalkyl, $(C_1\text{-}C_6)$-Alkoxy, $(C_1\text{-}C_6)$-Alkylthio, $(C_1\text{-}C_6)$-Haloalkyl, $(C_2\text{-}C_6)$-Haloalkenyl, $(C_2\text{-}C_6)$-Haloalkinyl, $(C_1\text{-}C_6)$-Haloalkoxy, $(C_1\text{-}C_6)$-Haloalkylthio, $(C_3\text{-}C_6)$-Cycloalkyl, $(C_3\text{-}C_6)$-Cycloalkanyloxy, $(C_3\text{-}C_6)$-Cycloalkanylthio, $(C_3\text{-}C_6)$-Cycloalkenyl, $(C_3\text{-}C_6)$-Cycloalkenyloxy, $(C_3\text{-}C_6)$-Cycloalkenylthio, Phenyl, Heteroaryl, Heterocyclyl, wobei in den vorgenannten Gruppen das Heteroaryl ein aromatischer fünf-, sechs- oder siebengliedriger Heterocyclus und das das Heterocyclyl ein gesättigter oder teilweise ungesättigter fünf-, sechs- oder siebengliedriger Heterocyclus ist, die jeweils ein, zwei, drei oder vier Heteroatome aus der Gruppe O, N und S enthalten, oder $NR^aR^b$, wobei zwei Reste $R^1$, die an dasselbe Kohlenstoffatom gebunden sind, gemeinsam mit dem Kohlenstoffatom, an das sie gebunden sind, auch einen $(C_3\text{-}C_6)$-Cycloalkylring bilden können; wobei $NR^aR^b$ wie unten definiert sind; wobei jeder der letztgenannten drei Reste unabhängig voneinander unsubstituiert oder durch einen oder mehrere Reste aus der Gruppe Halogen, Hydroxy, $(C_1\text{-}C_4)$-Alkoxy und $(C_1\text{-}C_4)$-Alkylthio substituiert ist, bedeutet,

$R^2$ Wasserstoff, $(C_1\text{-}C_6)$-Alkyl, $(C_2\text{-}C_6)$-Alkenyl, $(C_2\text{-}C_6)$-Alkinyl, $(C_3\text{-}C_6)$-Cycloalkyl, $(C_1\text{-}C_6)$-Alkoxy, $(C_1\text{-}C_6)$-Alkylthio, $(C_1\text{-}C_6)$-Haloalkyl, $(C_2\text{-}C_6)$-Haloalkenyl, $(C_2\text{-}C_6)$-Haloalkinyl, $(C_1\text{-}C_6)$-Haloalkoxy, $(C_1\text{-}C_6)$-Haloalkylthio, $(C_3\text{-}C_6)$-Cycloalkanyloxy, $(C_3\text{-}C_6)$-Cycloalkanylthio, $(C_3\text{-}C_6)$-Cycloalkenyl, $(C_3\text{-}C_6)$-Cycloalkenyloxy, $(C_3\text{-}C_6)$-Cycloalkenylthio, Phenyl, Heteroaryl, Heterocyclyl, wobei in den vorgenannten Gruppen das Heteroaryl ein aromatischer fünf-, sechs- oder siebengliedriger Heterocyclus und das das Heterocyclyl ein gesättigter oder teilweise ungesättigter fünf-, sechs- oder siebengliedriger Heterocyclus ist, die jeweils ein, zwei, drei oder vier Heteroatome aus der Gruppe O, N und S enthalten, oder $NR^aR^b$, wobei zwei Reste $R^1$, die an dasselbe Kohlenstoffatom gebunden sind, gemeinsam mit dem Kohlenstoffatom, an das sie gebunden sind, auch einen $(C_3\text{-}C_6)$-Cycloalkylring bilden können; wobei $NR^aR^b$ wie unten definiert sind; wobei jeder der letztgenannten drei Reste unabhängig voneinander unsubstituiert oder durch einen oder mehrere Reste aus der Gruppe Halogen, Hydroxy, $(C_1\text{-}C_4)$-Alkoxy und $(C_1\text{-}C_4)$-Alkylthio substituiert ist, bedeutet,

$R^3$ Wasserstoff, $(C_1\text{-}C_6)$-Alkyl, $(C_2\text{-}C_6)$-Alkenyl, $(C_2\text{-}C_6)$-Alkinyl, $(C_3\text{-}C_6)$-Cycloalkyl, $(C_1\text{-}C_6)$-Alkoxy, $(C_1\text{-}C_6)$-Alkylthio, $(C_1\text{-}C_6)$-Haloalkyl, $(C_1\text{-}C_6)$-Haloalkenyl, $(C_2\text{-}C_{10})$-Haloalkinyl, $(C_1\text{-}C_6)$-Haloalkoxy, $(C_1\text{-}C_6)$-Haloalkylthio, $(C_3\text{-}C_6)$-Cycloalkanyloxy, $(C_3\text{-}C_6)$-Cycloalkanylthio, $(C_3\text{-}C_6)$-Cycloalkenyl, $(C_3\text{-}C_6)$-Cycloalkenyloxy, $(C_3\text{-}C_6)$-Cycloalkenylthio, Phenyl, Heteroaryl, Heterocyclyl, wobei in den vorgenannten Gruppen das Heteroaryl ein aromatischer fünf-, sechs- oder siebengliedriger Heterocyclus und das das Heterocyclyl ein gesättigter oder teilweise ungesättigter fünf-, sechs- oder siebengliedriger Heterocyclus ist, die jeweils ein, zwei, drei oder vier Heteroatome aus der Gruppe O, N und S enthalten, oder $NR^aR^b$, wobei zwei Reste $R^1$, die an dasselbe Kohlenstoffatom gebunden sind, gemeinsam mit dem Kohlenstoffatom, an das sie gebunden sind, auch einen $(C_3\text{-}C_6)$-Cycloalkylring bilden können; wobei $NR^aR^b$ wie unten definiert sind; wobei jeder der letztgenannten drei Reste unabhängig voneinander unsubstituiert oder durch einen oder mehrere Reste aus der Gruppe Halogen, Hydroxy, $(C_1\text{-}C_4)$-Alkoxy und $(C_1\text{-}C_4)$-Alkylthio substituiert ist, bedeutet,

$R^4$ Wasserstoff, $(C_1\text{-}C_6)$-Alkyl, $(C_2\text{-}C_6)$-Alkenyl, $(C_2\text{-}C_6)$-Alkinyl, $(C_3\text{-}C_6)$-Cycloalkyl, $(C_1\text{-}C_6)$-Alkoxy, $(C_1\text{-}C_6)$-Alkylthio, $(C_1\text{-}C_6)$-Haloalkyl, $(C_1\text{-}C_6)$-Haloalkenyl, $(C_2\text{-}C_6)$-Haloalkinyl, $(C_1\text{-}C_6)$-Haloalkoxy, $(C_1\text{-}C_6)$-Haloalkylthio, $(C_3\text{-}C_6)$-Cycloalkanyloxy, $(C_3\text{-}C_6)$-Cycloalkanylthio, $(C_3\text{-}C_6)$-Cycloalkenyl, $(C_3\text{-}C_6)$-Cycloalkenyloxy, $(C_3\text{-}C_6)$-Cycloalkenylthio, Phenyl, Heteroaryl, Heterocyclyl, wobei in den vorgenannten Gruppen das Heteroaryl ein aromatischer fünf-, sechs- oder siebengliedriger Heterocyclus und das das Heterocyclyl ein gesättigter oder teilweise ungesättigter fünf-, sechs- oder siebengliedriger Heterocyclus ist, die jeweils ein, zwei, drei oder vier Heteroatome aus der Gruppe O, N und S enthalten, oder $NR^aR^b$, wobei zwei Reste $R^1$, die an dasselbe Kohlenstoffatom gebunden sind, gemeinsam mit dem Kohlenstoffatom, an das sie gebunden sind, auch einen $(C_3\text{-}C_6)$-Cycloalkylring bilden können; wobei jeder der Reste $R^a$, $R^b$ inklusive Substituenten 1 bis 30 C-Atome aufweist, bedeutet, und jeder der letztgenannten drei Reste unabhängig voneinander unsubstituiert oder durch einen oder mehrere Reste aus der Gruppe Halogen, Hydroxy, $(C_1\text{-}C_4)$-Alkoxy und $(C_1\text{-}C_4)$-Alkylthio substituiert ist,

$R^5$ Wasserstoff, $(C_1\text{-}C_6)$-Alkyl, $(C_2\text{-}C_6)$-Alkenyl, $(C_2\text{-}C_6)$-Alkinyl, $(C_3\text{-}C_6)$-Cycloalkyl, $(C_1\text{-}C_6)$-Haloalkyl,

$(C_2-C_6)$-Haloalkenyl, $(C_2-C_6)$-Haloalkinyl, $(C_3-C_6)$-Cycloalkenyl, wobei jeder der letztgenannten drei Reste unabhängig voneinander unsubstituiert oder durch einen oder mehrere Reste aus der Gruppe Halogen, CN, Hydroxy, $(C_1-C_4)$-Alkoxy und $(C_1-C_4)$-Alkylthio substituiert ist, bedeutet, oder einen Rest der Formel $SiR^aR^bR^c$, wobei in der letztgenannten Formel jeder der Reste $R^a$, $R^b$ und $R^c$ unabhängig voneinander Wasserstoff oder einen gegebenenfalls substituierten Kohlenwasserstoffrest bedeutet, inklusive Substituenten 1 bis 30 C-Atome, vorzugsweise 1 bis 24 C-Atome, insbesondere 1 bis 20 C-Atome aufweist, bedeutet,

$R^6$ Wasserstoff, $(C_2-C_8)$-Alkyl, $(C_2-C_8)$-Alkenyl, $(C_2-C_8)$-Alkinyl, $(C_3-C_8)$-Cycloalkyl, $(C_1-C_8)$-Haloalkyl, $(C_2-C_8)$-Haloalkenyl, $(C_2-C_8)$-Haloalkinyl, $(C_3-C_8)$-Cycloalkanyloxy, $(C_3-C_8)$-Cycloalkanylthio, $(C_3-C_8)$-Cycloalkenyl, wobei jeder der letztgenannten drei Reste unabhängig voneinander unsubstituiert oder durch einen oder mehrere Reste aus der Gruppe Halogen, Hydroxy, $(C_1-C_4)$-Alkoxy und $(C_1-C_4)$-Alkylthio substituiert ist, bedeutet,

$R^7$ Wasserstoff, $(C_1-C_7)$-Alkyl, $(C_2-C_7)$-Alkenyl, $(C_2-C_7)$-Alkinyl, $(C_3-C_7)$-Cycloalkyl, $(C_1-C_7)$-Haloalkyl, $(C_2-C_7)$-Haloalkenyl, $(C_2-C_7)$-Haloalkinyl, $(C_3-C_7)$-Cycloalkenyl, $(C_3-C_7)$-Cycloalkenyloxy, wobei jeder der letztgenannten drei Reste unabhängig voneinander unsubstituiert oder durch einen oder mehrere Reste aus der Gruppe Halogen, Hydroxy, $(C_1-C_4)$-Alkoxy und $(C_1-C_4)$-Alkylthio substituiert ist, bedeutet, oder einen Rest der Formel $SiR^aR^bR^c$, wobei in der letztgenannten Formel jeder der Reste $R^a$, $R^b$ und $R^c$ unabhängig voneinander Wasserstoff oder einen gegebenenfalls substituierten Kohlenwasserstoffrest bedeutet, inklusive Substituenten 1 bis 30 C-Atome, vorzugsweise 1 bis 24 C-Atome, insbesondere 1 bis 20 C-Atome aufweist, bedeutet,

**13.** Sprühlösung zur Behandlung von Pflanzen, enthaltend eine zur Steigerung der Widerstandsfähigkeit von Pflanzen gegenüber abiotischen Stressfaktoren wirksame Menge einer oder mehrerer der substituierten Isochinoline gemäß einem der Ansprüche 12.

Europäisches Patentamt
European Patent Office
Office européen des brevets

# EUROPÄISCHER RECHERCHENBERICHT

Nummer der Anmeldung
EP 12 19 5685

| Kategorie | EINSCHLÄGIGE DOKUMENTE<br>Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | QING F-L ET AL: "A stereoselective synthesis of ethyl 9-cis-7,8-didehydro-19-trifluoromethylretinoate", JOURNAL OF FLUORINE CHEMISTRY, ELSEVIER, NL, Bd. 101, Nr. 1, 1. Januar 2000 (2000-01-01), Seiten 31-33, XP004244491, ISSN: 0022-1139, DOI: 10.1016/S0022-1139(99)00186-4<br>* Paragraph 3.1 *<br>* Verbindung 4 *<br>----- | 12 | INV.<br>A01N37/36<br>A01P21/00 |
| X | QING F-L ET AL: "Ethyl 3-Iodo-4,4,4-Trifluoro-2(Z)-Butenoate: Regio- and Stereo-Specific Preparation and Palladium-Catalyzed Reaction with Terminal Alkynes", TETRAHEDRON LETTERS, ELSEVIER, AMSTERDAM, NL, Bd. 38, Nr. 38, 22. September 1997 (1997-09-22), Seiten 6729-6732, XP027546586, ISSN: 0040-4039, DOI: 10.1016/S0040-4039(97)01538-4<br>* Tabelle 1 *<br>* Beispiel 4h *<br>-----<br>-/-- | 12 | RECHERCHIERTE SACHGEBIETE (IPC)<br>A01N |

1

Der vorliegende Recherchenbericht wurde für alle Patentansprüche erstellt

| Recherchenort | Abschlußdatum der Recherche | Prüfer |
|---|---|---|
| München | 29. April 2013 | Habermann, Jörg |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

Europäisches Patentamt
European Patent Office
Office européen des brevets

# EUROPÄISCHER RECHERCHENBERICHT

Nummer der Anmeldung
EP 12 19 5685

| Kategorie | EINSCHLÄGIGE DOKUMENTE<br>Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | TAISHI UMEZAWA ET AL: "CYP707A3, a major ABA 8'-hydroxylase involved in dehydration and rehydration response in Arabidopsis thaliana",<br>THE PLANT JOURNAL,<br>Bd. 46, Nr. 2, 1. April 2006 (2006-04-01), Seiten 171-182, XP055061224,<br>ISSN: 0960-7412, DOI: 10.1111/j.1365-313X.2006.02683.x<br>* Seite 172, Spalte 1, Zeile 11 - Zeile 33 *<br>* Seite 174, Spalte 1, Zeile 28 - Zeile 36 *<br>* Seite 174, Spalte 1, Zeile 41 - Zeile 49 *<br>----- | 1-13 | |
| Y | KITAHATA N ET AL: "Chemical regulation of abscisic acid catabolism in plants by cytochrome P450 inhibitors",<br>BIOORGANIC & MEDICINAL CHEMISTRY, PERGAMON, GB,<br>Bd. 13, Nr. 14, 15. Juli 2005 (2005-07-15), Seiten 4491-4498, XP027638009,<br>ISSN: 0968-0896<br>[gefunden am 2005-07-15]<br>* Seite 4494, Spalte 2, Zeile 15 - Zeile 19 *<br>* Abbildungen 5, 7 *<br>-----<br>-/-- | 1-13 | RECHERCHIERTE SACHGEBIETE (IPC) |

Der vorliegende Recherchenbericht wurde für alle Patentansprüche erstellt

1

| Recherchenort | Abschlußdatum der Recherche | Prüfer |
|---|---|---|
| München | 29. April 2013 | Habermann, Jörg |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

Europäisches Patentamt
European Patent Office
Office européen des brevets

# EUROPÄISCHER RECHERCHENBERICHT

Nummer der Anmeldung
EP 12 19 5685

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | UENO ET AL: "Asymmetrical ligand binding by abscisic acid 8'-hydroxylase", BIOORGANIC & MEDICINAL CHEMISTRY, PERGAMON, GB, Bd. 15, Nr. 18, 25. Juli 2007 (2007-07-25), Seiten 6311-6322, XP022169177, ISSN: 0968-0896, DOI: 10.1016/J.BMC.2007.06.010<br>* Seite 6314, Spalte 1, Zeile 1 - Zeile 29 *<br>* Tabelle 1 *<br>----- | 1-13 | |
| X<br>Y | DE 19 53 152 A1 (FUJISAWA PHARMACEUTICAL CO) 21. Januar 1971 (1971-01-21)<br>* Seite 59, Zeile 1 - Zeile 3 *<br>* Seite 59, Zeile 10 - Zeile 15 *<br>* Seite 59, Zeile 17 - Zeile 25 *<br>* Seite 61, Zeile 15 - Zeile 19 *<br>* Ansprüche 1, 5 *<br>----- | 1-5,9,10<br>1-13 | |
| X | WO 94/15467 A1 (CANADA NAT RES COUNCIL [CA]) 21. Juli 1994 (1994-07-21)<br>* Seite 10, Zeile 30 - Zeile 31 *<br>* Seite 12, Zeile 6 - Zeile 11 *<br>* Verbindung 12 *<br>----- | 1,2,4,5,9,10 | RECHERCHIERTE SACHGEBIETE (IPC) |

Der vorliegende Recherchenbericht wurde für alle Patentansprüche erstellt

1

| Recherchenort | Abschlußdatum der Recherche | Prüfer |
|---|---|---|
| München | 29. April 2013 | Habermann, Jörg |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR.

EP 12 19 5685

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

29-04-2013

| Im Recherchenbericht angeführtes Patentdokument | Datum der Veröffentlichung | Mitglied(er) der Patentfamilie | Datum der Veröffentlichung |
|---|---|---|---|
| DE 1953152 A1 | 21-01-1971 | DE 1953152 A1<br>FR 2021619 A1<br>NL 6916195 A | 21-01-1971<br>24-07-1970<br>28-04-1970 |
| WO 9415467 A1 | 21-07-1994 | KEINE | |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- NL 6811769
- DE 1953152
- US 20100160166 A
- WO 2009005794 A
- WO 2009058216 A
- WO 200028055 A, Schading and Wei
- US 5518995 A, Abrams
- WO 2005108345 A, Abrams
- US 5201931 A, Abrams and Gusta
- WO 9723441 A, Abrams
- US 5481034 A, Kim
- US 5254694 A, Sasaki
- EP 78509 A, Bliesener
- DE 3534948, Draber
- DE 4103253, Bergmann
- DD 277832, Bergmann
- DD 277835, Bergmann
- WO 0004173 A
- WO 04090140 A
- US 6153786 A1
- DE 4128828 A
- DE 1905834 A
- DE 19631764 A
- WO 92005251 A
- WO 95009910 A
- WO 9827806 A
- WO 05002324 A
- WO 06021972 A
- US 6229072 B
- WO 8910396 A
- WO 91002069 A
- WO 0166704 A
- EP 0837944 A
- WO 00066746 A
- WO 00066747 A
- WO 02026995 A
- US 5776760 A
- US 5463175 A
- WO 02036782 A
- WO 03092360 A
- WO 05012515 A
- WO 07024782 A
- WO 01024615 A
- WO 03013226 A
- US 5561236 A
- US 5648477 A
- US 5646024 A
- US 5273894 A
- US 5637489 A
- US 5276268 A
- US 5739082 A
- US 5908810 A
- US 7112665 B
- WO 96038567 A
- WO 99024585 A
- WO 99024586 A
- WO 99034008 A
- WO 0236787 A
- WO 04024928 A
- US 5605011 A
- US 5378824 A
- US 5141870 A
- US 5013659 A
- US 5767361 A
- US 5731180 A
- US 5304732 A
- US 4761373 A
- US 5331107 A
- US 5928937 A
- WO 96033270 A
- WO 04040012 A
- WO 04106529 A
- WO 05020673 A
- WO 05093093 A
- WO 06007373 A
- WO 06015376 A
- WO 06024351 A
- WO 06060634 A
- US 5084082 A
- WO 9741218 A
- US 5773702 A
- WO 99057965 A
- US 5198599 A
- WO 01065922 A
- WO 07027777 A
- WO 9421795 A
- WO 00004173 A
- EP 04077984 A
- EP 06009836 A
- EP 04077624 A
- WO 06133827 A
- EP 07002433 W
- EP 0571427 A
- WO 95004826 A
- EP 0719338 A
- WO 9615248 A
- WO 9619581 A
- WO 9627674 A

- WO 9711188 A
- WO 9726362 A
- WO 9732985 A
- WO 9742328 A
- WO 9744472 A
- WO 9745545 A
- WO 9827212 A
- WO 9840503 A
- WO 9958688 A
- WO 9958690 A
- WO 9958654 A
- WO 00008184 A
- WO 00008185 A
- WO 0028052 A
- WO 0077229 A
- WO 0112782 A
- WO 0112826 A
- WO 02101059 A
- WO 03071860 A
- WO 04056999 A
- WO 05030942 A
- WO 05030941 A
- WO 05095632 A
- WO 05095617 A
- WO 05095619 A
- WO 05095618 A
- WO 05123927 A
- WO 06018319 A
- WO 06103107 A
- WO 06108702 A
- WO 07009823 A
- WO 0022140 A
- WO 06063862 A
- WO 06072603 A
- WO 02034923 A
- EP 06090134 A
- EP 06090228 A
- EP 06090227 A
- EP 07090007 A
- EP 07090009 A
- WO 0114569 A
- WO 0279410 A
- WO 0333540 A
- WO 04078983 A
- WO 0119975 A
- WO 9526407 A
- WO 9634968 A
- WO 9820145 A
- WO 9912950 A
- WO 9966050 A
- WO 9953072 A
- US 6734341 B
- WO 0011192 A
- WO 9822604 A
- WO 9832326 A
- WO 0198509 A
- WO 05002359 A
- US 5824790 A
- US 6013861 A
- WO 94004693 A
- WO 94009144 A
- WO 9411520 A
- WO 9535026 A
- WO 9720936 A
- EP 0663956 A
- WO 96001904 A
- WO 96021023 A
- WO 98039460 A
- WO 99024593 A
- WO 95031553 A
- US 2002031826 A
- US 6284479 B
- US 5712107 A
- WO 97047806 A
- WO 97047807 A
- WO 97047808 A
- WO 0014249 A
- WO 200073422 A
- WO 00047727 A
- EP 06077301 A
- US 5908975 A
- EP 0728213 A
- WO 06032538 A
- WO 07039314 A
- WO 07039315 A
- WO 07039316 A
- JP 2006304779 A
- WO 05012529 A
- WO 98000549 A
- WO 04053219 A
- WO 01017333 A
- WO 0245485 A
- WO 05017157 A
- WO 06136351 A
- US 5969169 A
- US 5840946 A
- US 6323392 B
- US 6063 A
- US 947 A
- US 6270828 B
- US 6169190 B
- US 5965755 A
- US 5434283 A
- WO 2004067528 A
- WO 9107874 A
- EP 333131 A
- EP 269806 A
- EP 268554 A
- EP 174562 A
- EP 346620 A
- WO 9108202 A
- WO 9507897 A
- EP 86750 A
- EP 94349 A
- EP 191736 A
- EP 0492366 A

- WO 200234048 A
- EP 0582198 A
- WO 9745016 A
- WO 9916744 A
- EP 365484 A
- WO 2004084631 A
- WO 2005015994 A
- WO 2005016001 A
- WO 2005112630 A
- WO 199838856 A
- WO 9827049 A
- WO 1999000020 A
- WO 2007023719 A
- WO 2007023764 A
- WO 199813361 A
- JP 60087254 A
- WO 2008131861 A
- WO 2008131860 A
- DE 3335514
- EP 30287 A
- DE 2906507
- US 5123951 A


**In der Beschreibung aufgeführte Nicht-Patentliteratur**


- *Current Med. Chem.,* 2010, vol. 17, 3230
- *Bioorg. Med. Chem.,* 2007, vol. 15, 2736
- *Org. Lett.,* 2005, vol. 7, 1363
- *Can. J. Chem.,* 1973, vol. 51, 3620
- *J. Am. Chem. Soc.,* 1954, vol. 76, 5380
- *Synlett,* 2005, 2919
- Pflanzenbiochemie. Spektrum Akademischer Verlag, 1996, 393-462
- Molecular Biology of Plants. American Society of Plant Physiologists, 2000, 1102-1203
- **JAGLO-OTTOSEN et al.** *Science,* 1998, vol. 280, 104-106
- **HASEGAWA et al.** *Annu Rev Plant Physiol Plant Mol Biol,* 2000, vol. 51, 463-499
- **INGRAM ; BARTELS.** *Annu Rev Plant Physiol Plant Mol Biol,* 1996, vol. 47, 277-403
- **CLOSE.** *Physiol Plant,* 1997, vol. 100, 291-296
- **BRAY.** *Plant Physiol,* 1993, vol. 103, 1035-1040
- **KIRCH et al.** *Plant Mol Biol,* 2005, vol. 57, 315-332
- **YU et al.** *Mol Cells,* 2005, vol. 19, 328-333
- Biochemistry and Molecular Biology of Plants. American Society of Plant Physiologists, 2000, 850-929
- **SEMBDNER ; PARTHIER.** *Ann. Rev. Plant Physiol. Plant Mol. Biol.,* 1993, vol. 44, 569-589
- **MELCHER et al.** *Nature Structural & Molecular Biology advanced online publication,* 2010
- **ASAMI ; YOSHIDA.** *RIKEN Review,* 1999, vol. 21, 17-19
- **UENO et al.** *Bioorganic & Medicinal Chemistry,* 2005, vol. 13, 3359-3370
- **TODOROKI et al.** *Current Medicinal Chemistry,* 2010, vol. 17, 3230-3244
- **CUTLER et al.** *Annu. Rev. Plant Biol.,* 2010, vol. 61, 651-679
- **TODOROKI et al.** *Bioorganic & Medicinal Chemistry,* 2004, vol. 12, 363-370
- *BIOORGANIC & MEDICINAL CHEMISTRY,* vol. 52, 8081-8098
- **TODOROKI et al.** *Tetrahedron,* 2000, vol. 56, 8095-8100
- **TODOROKI et al.** *Tetrahedron,* 1996, vol. 52, 8081-8098
- **CHURCHILL et al.** *Plant Growth Regul,* 1998, vol. 25, 35-45
- **SCHUBERT et al.** *Plant Growth Regul,* 1991, vol. 10, 27-32
- **CHEN ; MACTAGGERT.** *Agricultural and Biological Chemistry,* 1986, vol. 50, 1097-1100
- **MORRISON ; ANDREWS.** *J Plant Growth Regul,* 1992, vol. 11, 113-117
- **PARK et al.** *Science,* 2009, vol. 324, 1068-1071
- **CHOLEWA et al.** *Can. J. Botany,* 1997, vol. 75, 375-382
- **BARTLETT et al.** *Pest Manag Sci,* vol. 60, 309
- **CEDERGREEN.** *Env. Pollution,* 2008, vol. 156, 1099
- **CHEN et al.** *Plant Cell Environ,* 2000, vol. 23, 609-618
- **BLOCK et al.** *The Plant Journal,* 2004, vol. 41, 95
- **LEVINE et al.** *FEBS Lett.,* 1998, vol. 440, 1
- *Curr. Org. Chem.,* 2003, vol. 7, 967-993
- *Journal of Organic Chemistry,* 1964, vol. 29, 1872-1876
- *Journal of American Chemical Society,* 1992, vol. 114, 2544-2559
- *Journal of American Chemical Society,* 1987, vol. 109, 4717
- *Chemical Communications,* 1985, 958
- *Org. Biomol. Chem.,* 2006, vol. 4, 4186-4192
- *Journal of Fluorine Chemistry,* 2000, vol. 101, 31-33
- *Tetrahedron Letters,* 1997, vol. 38 (38), 6729-6732
- *Journal of the American Chemical Society,* 1997, vol. 119 (4), 698-708
- *Tetrahedron Letters,* 1996, vol. 37 (23), 3971-3974
- *Tetrahedron Letters,* 1985, vol. 26 (24), 2881-2884

- **R. D. LARSEN.** Organometallics in Process Chemistry. Springer Verlag, 2004
- **I. TSUJI.** Palladium Reagents and Catalysts. Wiley, 2004
- **M. BELLER ; C. BOLM.** Transition Metals for Organic Synthesis. VCH-Wiley, 2004
- **E.-I NEGISHI ; C. XU.** Handbook of Organopalladium Chemistry for Organic Synthesis. 2002, vol. 1, 531-549
- *Chem. Rev.,* 2003, vol. 103, 1979-2017
- *Pure Appl. Chem.,* 2004, vol. 76, 565-576
- *Tetrahedron,* 2009, vol. 65, 8418-8427
- *Journal of Combinatorial Chemistry,* 2009, vol. 11, 900-906
- *Journal of Fluorine Chemistry,* 1981, vol. 17, 249
- *Organic Letters,* 2000, vol. 2, 3407-3410
- *Tetrahedron letters,* 2008, vol. 49 (5), 794-798
- *Tetrahedron,* 1987, vol. 43, 4107
- *Tetrahedron,* 1983, vol. 39, 2315
- *Journal of Organic Synthesis,* 1983, vol. 48, 4436
- *Journal of the American Chemical Society,* 1984, vol. 106, 2735
- *Helvetica Chimica Acta,* 1978, vol. 61, 2616-2627
- *Bioorg. Med. Chem.,* 2004, vol. 12, 363-370
- *Tetrahedron,* 2003, vol. 59, 9091-9100
- *Org. Biomol. Chem.,* 2006, vol. 4, 1400-1412
- *Synthesis,* 1977, 561
- *Tetrahedron Letters,* 1992, vol. 33, 3477
- *Tetrahedron Letters,* 1974, 1593
- *Organic Letters,* 2004, vol. 6, 1785
- *Helvetica Chimica Acta,* 1986, vol. 69, 368
- *Journal of the American Chemical Society,* 2002, vol. 124, 7622
- *Journal of the American Chemical Society,* 2005, vol. 127, 17645
- **N. LEO BENOITON.** Chemistry of Peptide Synthesis. CRC Press, 2006
- *Organic Letters,* 2002, vol. 4, 703
- *Angewandte Chemie Int. Ed.,* 2006, vol. 45, 2916
- *Organic Letters,* 2010, vol. 12, 1056
- *Organic Letters,* 2005, vol. 7, 5191
- *Biooganic Medicinal Chemistry,* 2007, vol. 15 (18), 6311-6322
- Ullmann's Encyclopedia of Industrial Chemistry. Verlagsgesellschaft, 1987, vol. A 10, 323-431
- Ullmann's Encyclopedia of Industrial Chemistry. 1987, vol. A 10, 363-401
- **COMAI et al.** *Science,* 1983, vol. 221, 370-371
- **BARRY et al.** *Curr. Topics Plant Physiol.,* 1992, vol. 7, 139-145
- **SHAH et al.** *Science,* 1986, vol. 233, 478-481
- **GASSER et al.** *J. Biol. Chem.,* 1988, vol. 263, 4280-4289
- **TRANEL ; WRIGHT.** *Weed Science,* 2002, vol. 50, 700-712
- **VON CRICKMORE et al.** *Microbiology and Molecular Biology Reviews,* 1998, vol. 62, 807-813
- **CRICKMORE et al.** *Bacillus thuringiensis-Toxinnomenklatur aktualisiert,* 2005, http://www.lifesci.sussex.ac.uk/Home/Neil-Crickmore/Bt
- **MOELLENBECK et al.** *Nat. Biotechnol.,* 2001, vol. 19, 668-72
- **SCHNEPF et al.** *Applied Environm. Microb.,* 2006, vol. 71, 1765-1774
- **R. WEGLER.** Chemie der Pflanzenschutz- und Schädlingsbekämpfungsmittel. Springer Verlag, 1970, vol. 2, 401-412
- *Biotechn. Adv.,* 2006, vol. 24, 357-367
- *Bot. Bull. Acad. Sin.,* 1990, (40), 1-7
- *Plant Growth Reg.,* 1993, vol. 13, 41-46
- *Weed Research,* 1986, vol. 26, 441-445
- The Pesticide Manual. The British Crop Protection Council and the Royal Soc. of Chemistry, 1999